# EUROPÄISCHE PATENTSCHRIFT

(11) **EP 1 844 150 B1**
(45) Veröffentlichungstag und Bekanntmachung des Hinweises auf die Patenterteilung: **22.02.2012**
(21) Anmeldenummer: 06705826.3
(22) Anmeldetag: 20.01.2006
(51) Int. Cl.: C12N 15/70

(54) **REKOMBINANTE EXPRESSION VON PROTEINEN IN EINER DISULFIDVERBRÜCKTEN, ZWEIKETTIGEN FORM**
RECOMBINANT EXPRESSION OF PROTEINS IN A DISULFIDE-BRIDGED, TWO-CHAIN FORM
EXPRESSION RECOMBINEE DE PROTEINES A FORME BICATENAIRE A PONT DISULFURE

(30) Priorität: 21.01.2005 DE 102005002978
(43) Veröffentlichungstag der Anmeldung: 17.10.2007
(73) Patentinhaber: Merz Pharma GmbH & Co. KGaA, 60318 Frankfurt am Main (DE)
(72) Erfinder: SPECHT, Volker, 12207 Berlin (DE)
(74) Vertreter: Dehmel, Albrecht
(86) Internationale Anmeldenummer: PCT/DE2006/000088
(87) Internationale Veröffentlichungsnummer: WO 2006/076902

(56) Entgegenhaltungen:
- WO-A-01/14570
- WO-A-02/44199
- WO-A-2004/024909
- WO-A-2004/099254
- US-A1- 2004 018 589
- SUTTON J M ET AL: "Preparation of specifically activatable endopeptidase derivatives of Clostridium botulinum toxins type A, B, and C and their applications" PROTEIN EXPRESSION AND PURIFICATION, ACADEMIC PRESS, SAN DIEGO, CA, US, Bd. 40, Nr. 1, 8. Januar 2005 (2005-01-08), Seiten 31-41, XP004753559 ISSN: 1046-5928
- CHIRON M F ET AL: "Pseudomonas exotoxin exhibits increased sensitivity to furin when sequences at the cleavage site are mutated to resemble the arginine-rich loop of diphtheria toxin." MOLECULAR MICROBIOLOGY. NOV 1996, Bd. 22, Nr. 4, November 1996 (1996-11), Seiten 769-778, XP002391666 ISSN: 0950-382X

### SEQUENCE LISTING

<110> BioteCon Therapeutics GmbH
<120> Rekombinante Expression von Proteinen in einer disulfidverbrückten, zweikettigen Form
<130> BIO-008 PCT
<140> unknown
   <141> 2006-01-20
<150> 10 2005 002978.7
   <151> 2005-01-21
<160> 22
<170> PatentIn version 3.3
<210> 1
   <211> 3915
   <212> DNA
   <213> Clostridium botulinum
<400> 1
   atggcatttg ttaataaaca atttaattat aaagatcctg taaatggtgt tgatattgct 60
   tatataaaaa ttccaaatgc aggacaaatg caaccagtaa aagcttttaa aattcataat 120
   aaaatatggg ttattccaga aagagataca tttacaaatc ctgaagaagg agatttaaat 180
   ccaccaccag aagcaaaaca agttccagtt tcatattatg attcaacata tttaagtaca 240
   gataatgaaa aagataatta tttaaaggga gttacaaaat tatttgagag aatttattca 300
   actgatcttg gaagaatgtt gttaacatca atagtaaggg gaataccatt ttggggtgga 360
   agtacaatag atacagaatt aaaagttatt gatactaatt gtattaatgt gatacaacca 420
   gatggtagtt atagatcaga agaacttaat ctagtaataa taggaccctc agctgatatt 480
   atacagtttg aatgtaaaag ctttggacat gaagttttga atcttacgcg aaatggttat 540
   ggctctactc aatacattag atttagccca gattttacat ttggttttga ggagtcactt 600
   gaagttgata caaatcctct tttaggtgca ggcaaatttg ctacagatcc agcagtaaca 660
   ttagcacatg aacttataca tgctggacat agattatatg gaatagcaat taatccaaat 720
   agggttttta aagtaaatac taatgcctat tatgaaatga gtgggttaga agtaagcttt 780
   gaggaactta gaacatttgg gggacatgat gcaaagttta tagatagttt acaggaaaac 840
   gaatttcgtc tatattatta taataagttt aaagatatag caagtacact taataaagct 900
   aaatcaatag taggtactac tgcttcatta cagtatatga aaaatgtttt taaagagaaa 960
   tatctcctat ctgaagatac atctggaaaa ttttcggtag ataaattaaa atttgataag 1020
   ttatacaaaa tgttaacaga gatttacaca gaggataatt ttgttaagtt ttttaaagta 1080
   cttaacagaa aaacatattt gaattttgat aaagccgtat ttaagataaa tatagtacct 1140
   aaggtaaatt acacaatata tgatggattt aatttaagaa atacaaattt agcagcaaac 1200
   tttaatggtc aaaatacaga aattaataat atgaatttta ctaaactaaa aaattttact 1260
   ggattgtttg aattttataa gttgctatgt gtaagaggga taataacttc taaaactaaa 1320
   tcattagttc cgcgtggatc caaggcctta aatgatttat gtatcaaagt taataattgg 1380
   gacttgtttt ttagtccttc agaagataat tttactaatg atctaaataa aggagaagaa 1440
   attacatctg atactaatat agaagcagca gaagaaaata ttagtttaga tttaatacaa 1500
   caatattatt taacctttaa ttttgataat gaacctgaaa atatttcaat agaaaatctt 1560
   tcaagtgaca ttataggcca attagaactt atgcctaata tagaaagatt tcctaatgga 1620
   aaaaagtatg agttagataa atatactatg ttccattatc ttcgtgctca agaatttgaa 1680
   catggtaaat ctaggattgc tttaacaaat tctgttaacg aagcattatt aaatcctagt 1740
   cgtgtttata catttttttc ttcagactat gtaaagaaag ttaataaagc tacggaggca 1800
   gctatgtttt taggctgggt agaacaatta gtatatgatt ttaccgatga aactagcgaa 1860
   gtaagtacta cggataaaat tgcggatata actataatta ttccatatat aggacctgct 1920
   ttaaatatag gtaatatgtt atataaagat gattttgtag gtgctttaat attttcagga 1980
   gctgttattc tgttagaatt tataccagag attgcaatac ctgtattagg tacttttgca 2040
   cttgtatcat atattgcgaa taaggttcta accgttcaaa caatagataa tgctttaagt 2100
   aaaagaaatg aaaaatggga tgaggtctat aaatatatag taacaaattg gttagcaaag 2160
   gttaatacac agattgatct aataagaaaa aaaatgaaag aagctttaga aaatcaagca 2220
   gaagcaacaa aggctataat aaactatcag tataatcaat atactgagga agagaaaaat 2280
   aatattaatt ttaatattga tgatttaagt tcgaaactta atgagtctat aaataaagct 2340
   atgattaata taaataaatt tttgaatcaa tgctctgttt catatttaat gaattctatg   2400
   atcccttatg gtgttaaacg gttagaagat tttgatgcta gtcttaaaga tgcattatta   2460
   aagtatatat atgataatag aggaacttta attggtcaag tagatagatt aaaagataaa   2520
   gttaataata cacttagtac agatatacct tttcagcttt ccaaatacgt agataatcaa   2580
   agattattat ctacatttac tgaatatatt aagaatatta ttaatacttc tatattgaat   2640
   ttaagatatg aaagtaatca tttaatagac ttatctaggt atgcatcaaa aataaatatt   2700
   ggtagtaaag taaattttga tccaatagat aaaaatcaaa ttcaattatt taatttagaa   2760
   agtagtaaaa ttgaggtaat tttaaaaaat gctattgtat ataatagtat gtatgaaaat   2820
   tttagtacta gcttttggat aagaattcct aagtatttta acagtataag tctaaataat   2880
   gaatatacaa taataaattg tatggaaaat aattcaggat ggaaagtatc acttaattat   2940
   ggtgaaataa tctggacttt acaggatact caggaaataa aacaaagagt agtttttaaa   3000
   tacagtcaaa tgattaatat atcagattat ataaacagat ggatttttgt aactatcact   3060
   aataatagat taaataactc taaaatttat ataaatggaa gattaataga tcaaaaacca   3120
   atttcaaatt taggtaatat tcatgctagt aataatataa tgtttaaatt agatggttgt   3180
   agagatacac atagatatat ttggataaaa tattttaatc tttttgataa ggaattaaat   3240
   gaaaaagaaa tcaaagattt atatgataat caatcaaatt caggtatttt aaaagacttt   3300
   tggggtgatt atttacaata tgataaacca tactatatgt taaatttata tgatccaaat   3360
   aaatatgtcg atgtaaataa tgtaggtatt agaggttata tgtatcttaa agggcctaga   3420
   ggtagcgtaa tgactacaaa catttattta aattcaagtt tgtatagggg gacaaaattt   3480
   attataaaaa aatatgcttc tggaaataaa gataatattg ttagaaataa tgatcgtgta   3540
   tatattaatg tagtagttaa aaataaagaa tataggttag ctactaatgc atcacaggca   3600
   ggcgtagaaa aaatactaag tgcattagaa atacctgatg taggaaatct aagtcaagta   3660
   gtagtaatga agtcaaaaaa tgatcaagga ataacaaata aatgcaaaat gaatttacaa   3720
   gataataatg ggaatgatat aggctttata ggatttcatc agtttaataa tatagctaaa   3780
   ctagtagcaa gtaattggta taatagacaa atagaaagat ctagtaggac tttgggttgc   3840
   tcatgggaat ttattcctgt agatgatgga tggggagaaa ggccactgct cgagcaccac   3900
   caccaccacc actaa                                                    3915
<210> 2
   <211> 1304
   <212> PRT
   <213> Clostridium botulinum
<400> 2
   Met Ala Phe Val Asn Lys Gln Phe Asn Tyr Lys Asp Pro Val Asn Gly
   1 5 10 15
   Val Asp Ile Ala Tyr Ile Lys Ile Pro Asn Val Gly Gln Met Gln Pro
   20 25 30
   Val Lys Ala Phe Lys Ile His Asn Lys Ile Trp Val Ile Pro Glu Arg
   35 40 45
   Asp Thr Phe Thr Asn Pro Glu Glu Gly Asp Leu Asn Pro Pro Pro Glu
   50 55 60
   Ala Lys Gln Val Pro Val Ser Tyr Tyr Asp Ser Thr Tyr Leu Ser Thr
   65 70 75 80
   Asp Asn Glu Lys Asp Asn Tyr Leu Lys Gly Val Thr Lys Leu Phe Glu
   85 90 95
   Arg Ile Tyr Ser Thr Asp Leu Gly Arg Met Leu Leu Thr Ser Ile Val
   100 105 110
   Arg Gly Ile Pro Phe Trp Gly Gly Ser Thr Ile Asp Thr Glu Leu Lys
   115 120 125
   Val Ile Asp Thr Asn Cys Ile Asn Val Ile Gln Pro Asp Gly Ser Tyr
   130 135 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile His Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305                 310                 315                 320
   Tyr Leu Leu Ser Glu Asp Thr Ser Gly Lys Phe Ser Val Asp Lys Leu
                   325                 330                 335
   Lys Phe Asp Lys Leu Tyr Lys Met Leu Thr Glu Ile Tyr Thr Glu Asp
               340                 345                 350
   Asn Phe Val Lys Phe Phe Lys Val Leu Asn Arg Lys Thr Tyr Leu Asn
           355                 360                 365
   Phe Asp Lys Ala Val Phe Lys Ile Asn Ile Val Pro Lys Val Asn Tyr
       370                 375                 380
   Thr Ile Tyr Asp Gly Phe Asn Leu Arg Asn Thr Asn Leu Ala Ala Asn
   385                 390                 395                 400
   Phe Asn Gly Gln Asn Thr Glu Ile Asn Asn Met Asn Phe Thr Lys Leu
                   405                 410                 415
   Lys Asn Phe Thr Gly Leu Phe Glu Phe Tyr Lys Leu Leu Cys Val Arg
               420                 425                 430
   Gly Ile Ile Thr Ser Lys Thr Lys Ser Leu Val Pro Arg Gly Ser Lys
           435                 440                 445
   Ala Leu Asn Asp Leu Cys Ile Lys Val Asn Asn Trp Asp Leu Phe Phe
       450                 455                 460
   Ser Pro Ser Glu Asp Asn Phe Thr Asn Asp Leu Asn Lys Gly Glu Glu
   465                 470                 475                 480
   Ile Thr Ser Asp Thr Asn Ile Glu Ala Ala Glu Glu Asn Ile Ser Leu
                   485                 490                 495
   Asp Leu Ile Gln Gln Tyr Tyr Leu Thr Phe Asn Phe Asp Asn Glu Pro
               500                 505                 510
   Glu Asn Ile Ser Ile Glu Asn Leu Ser Ser Asp Ile Ile Gly Gln Leu
           515                 520                 525
   Glu Leu Met Pro Asn Ile Glu Arg Phe Pro Asn Gly Lys Lys Tyr Glu
       530                 535                 540
   Leu Asp Lys Tyr Thr Met Phe His Tyr Leu Arg Ala Gln Glu Phe Glu
   545                 550                 555                 560
   His Gly Lys Ser Arg Ile Ala Leu Thr Asn Ser Val Asn Glu Ala Leu
                   565                 570                 575
   Leu Asn Pro Ser Arg Val Tyr Thr Phe Phe Ser Ser Asp Tyr Val Lys
               580                 585                 590
   Lys Val Asn Lys Ala Thr Glu Ala Ala Met Phe Leu Gly Trp Val Glu
           595                 600                 605
   Gln Leu Val Tyr Asp Phe Thr Asp Glu Thr Ser Glu Val Ser Thr Thr
       610                 615                 620
   Asp Lys Ile Ala Asp Ile Thr Ile Ile Ile Pro Tyr Ile Gly Pro Ala
   625                 630                 635                 640
   Leu Asn Ile Gly Asn Met Leu Tyr Lys Asp Asp Phe Val Gly Ala Leu
                   645                 650                 655
   Ile Phe Ser Gly Ala Val Ile Leu Leu Glu Phe Ile Pro Glu Ile Ala
               660                 665                 670
   Ile Pro Val Leu Gly Thr Phe Ala Leu Val Ser Tyr Ile Ala Asn Lys
           675                 680                 685
   Val Leu Thr Val Gln Thr Ile Asp Asn Ala Leu Ser Lys Arg Asn Glu
       690                 695                 700
   Lys Trp Asp Glu Val Tyr Lys Tyr Ile Val Thr Asn Trp Leu Ala Lys
   705                 710                 715                 720
   Val Asn Thr Gln Ile Asp Leu Ile Arg Lys Lys Met Lys Glu Ala Leu
                   725                 730                 735
   Glu Asn Gln Ala Glu Ala Thr Lys Ala Ile Ile Asn Tyr Gln Tyr Asn
               740                 745                 750
   Gln Tyr Thr Glu Glu Glu Lys Asn Asn Ile Asn Phe Asn Ile Asp Asp
           755                 760                 765
   Leu Ser Ser Lys Leu Asn Glu Ser Ile Asn Lys Ala Met Ile Asn Ile
       770                 775                 780
   Asn Lys Phe Leu Asn Gln Cys Ser Val Ser Tyr Leu Met Asn Ser Met
   785                 790                 795                 800
   Ile Pro Tyr Gly Val Lys Arg Leu Glu Asp Phe Asp Ala Ser Leu Lys
                   805                 810                 815
   Asp Ala Leu Leu Lys Tyr Ile Tyr Asp Asn Arg Gly Thr Leu Ile Gly
               820                 825                 830
   Gln Val Asp Arg Leu Lys Asp Lys Val Asn Asn Thr Leu Ser Thr Asp
           835                 840                 845
   Ile Pro Phe Gln Leu Ser Lys Tyr Val Asp Asn Gln Arg Leu Leu Ser
       850                 855                 860
   Thr Phe Thr Glu Tyr Ile Lys Asn Ile Ile Asn Thr Ser Ile Leu Asn
   865                 870                 875                 880
   Leu Arg Tyr Glu Ser Asn His Leu Ile Asp Leu Ser Arg Tyr Ala Ser
                   885                 890                 895
   Lys Ile Asn Ile Gly Ser Lys Val Asn Phe Asp Pro Ile Asp Lys Asn
               900                 905                 910
   Gln Ile Gln Leu Phe Asn Leu Glu Ser Ser Lys Ile Glu Val Ile Leu
           915                 920                 925
   Lys Asn Ala Ile Val Tyr Asn Ser Met Tyr Glu Asn Phe Ser Thr Ser
       930                 935                 940
   Phe Trp Ile Arg Ile Pro Lys Tyr Phe Asn Ser Ile Ser Leu Asn Asn
   945                 950                 955                 960
   Glu Tyr Thr Ile Ile Asn Cys Met Glu Asn Asn Ser Gly Trp Lys Val
                   965                 970                 975
   Ser Leu Asn Tyr Gly Glu Ile Ile Trp Thr Leu Gln Asp Thr Gln Glu
               980                 985                 990
   Ile Lys Gln Arg Val Val Phe Lys  Tyr Ser Gln Met Ile  Asn Ile Ser
           995                 1000                1005
   Asp Tyr  Ile Asn Arg Trp Ile  Phe Val Thr Ile Thr  Asn Asn Arg
       1010                1015                1020
   Leu Asn  Asn Ser Lys Ile Tyr  Ile Asn Gly Arg Leu  Ile Asp Gln
       1025                1030                1035
   Lys Pro  Ile Ser Asn Leu Gly  Asn Ile His Ala Ser  Asn Asn Ile
       1040                1045                1050
   Met Phe  Lys Leu Asp Gly Cys  Arg Asp Thr His Arg  Tyr Ile Trp
       1055                1060                1065
   Ile Lys  Tyr Phe Asn Leu Phe  Asp Lys Glu Leu Asn  Glu Lys Glu
       1070                1075                1080
   Ile Lys  Asp Leu Tyr Asp Asn  Gln Ser Asn Ser Gly  Ile Leu Lys
       1085                1090                1095
   Asp Phe  Trp Gly Asp Tyr Leu  Gln Tyr Asp Lys Pro  Tyr Tyr Met
       1100                1105                1110
   Leu Asn  Leu Tyr Asp Pro Asn  Lys Tyr Val Asp Val  Asn Asn Val
       1115                1120                1125
   Gly Ile  Arg Gly Tyr Met Tyr  Leu Lys Gly Pro Arg  Gly Ser Val
       1130                1135                1140
   Met Thr Thr Asn Ile Tyr Leu Asn Ser Ser Leu Tyr Arg Gly Thr
       1145                1150                1155
   Lys Phe Ile Ile Lys Lys Tyr Ala Ser Gly Asn Lys Asp Asn Ile
       1160                1165                1170
   Val Arg Asn Asn Asp Arg Val Tyr Ile Asn Val Val Val Lys Asn
       1175                1180                1185
   Lys Glu Tyr Arg Leu Ala Thr Asn Ala Ser Gln Ala Gly Val Glu
       1190                1195                1200
   Lys Ile Leu Ser Ala Leu Glu Ile Pro Asp Val Gly Asn Leu Ser
       1205                1210                1215
   Gln Val Val Val Met Lys Ser Lys Asn Asp Gln Gly Ile Thr Asn
       1220                1225                1230
   Lys Cys Lys Met Asn Leu Gln Asp Asn Asn Gly Asn Asp Ile Gly
       1235                1240                1245
   Phe Ile Gly Phe His Gln Phe Asn Asn Ile Ala Lys Leu Val Ala
       1250                1255                1260
   Ser Asn Trp Tyr Asn Arg Gln Ile Glu Arg Ser Ser Arg Thr Leu
       1265                1270                1275
   Gly Cys Ser Trp Glu Phe Ile Pro Val Asp Asp Gly Trp Gly Glu
       1280                1285                1290
   Arg Pro Leu Leu Glu His His His His His His
       1295                1300
<210> 3
   <211> 2646
   <212> DNA
   <213> Clostridium botulinum
<400> 3
   atggcatttg ttaataaaca atttaattat aaagatcctg taaatggtgt tgatattgct      60
   tatataaaaa ttccaaatgc aggacaaatg caaccagtaa aagcttttaa aattcataat     120
   aaaatatggg ttattccaga aagagataca tttacaaatc ctgaagaagg agatttaaat     180
   ccaccaccag aagcaaaaca agttccagtt tcatattatg attcaacata tttaagtaca     240
   gataatgaaa aagataatta tttaaaggga gttacaaaat tatttgagag aatttattca     300
   actgatcttg gaagaatgtt gttaacatca atagtaaggg gaataccatt ttggggtgga     360
   agtacaatag atacagaatt aaaagttatt gatactaatt gtattaatgt gatacaacca     420
   gatggtagtt atagatcaga agaacttaat ctagtaataa taggaccctc agctgatatt     480
   atacagtttg aatgtaaaag ctttggacat gaagttttga atcttacgcg aaatggttat     540
   ggctctactc aatacattag atttagccca gattttacat ttggttttga ggagtcactt     600
   gaagttgata caaatcctct tttaggtgca ggcaaatttg ctacagatcc agcagtaaca     660
   ttagcacatg aacttataca tgctggacat agattatatg gaatagcaat taatccaaat     720
   agggttttta aagtaaatac taatgcctat tatgaaatga gtgggttaga agtaagcttt     780
   gaggaactta gaacatttgg gggacatgat gcaaagttta tagatagttt acaggaaaac     840
   gaatttcgtc tatattatta taataagttt aaagatatag caagtacact taataaagct     900
   aaatcaatag taggtactac tgcttcatta cagtatatga aaaatgtttt taaagagaaa     960
   tatctcctat ctgaagatac atctggaaaa ttttcggtag ataaattaaa atttgataag    1020
   ttatacaaaa tgttaacaga gatttacaca gaggataatt ttgttaagtt ttttaaagta    1080
   cttaacagaa aaacatattt gaattttgat aaagccgtat ttaagataaa tatagtacct    1140
   aaggtaaatt acacaatata tgatggattt aatttaagaa atacaaattt agcagcaaac    1200
   tttaatggtc aaaatacaga aattaataat atgaatttta ctaaactaaa aaattttact    1260
   ggattgtttg aattttataa gttgctatgt gtaagaggga taataacttc taaaactaaa    1320
   tcattagttc cgcgtggatc caaggcctta aatgatttat gtatcaaagt taataattgg    1380
   gacttgtttt ttagtccttc agaagataat tttactaatg atctaaataa aggagaagaa    1440
   attacatctg atactaatat agaagcagca gaagaaaata ttagtttaga tttaatacaa    1500
   caatattatt taacctttaa ttttgataat gaacctgaaa atatttcaat agaaaatctt    1560
   tcaagtgaca ttataggcca attagaactt atgcctaata tagaaagatt tcctaatgga    1620
   aaaaagtatg agttagataa atatactatg ttccattatc ttcgtgctca agaatttgaa    1680
   catggtaaat ctaggattgc tttaacaaat tctgttaacg aagcattatt aaatcctagt    1740
   cgtgtttata catttttttc ttcagactat gtaaagaaag ttaataaagc tacggaggca    1800
   gctatgtttt taggctgggt agaacaatta gtatatgatt ttaccgatga aactagcgaa    1860
   gtaagtacta cggataaaat tgcggatata actataatta ttccatatat aggacctgct    1920
   ttaaatatag gtaatatgtt atataaagat gattttgtag gtgctttaat attttcagga    1980
   gctgttattc tgttagaatt tataccagag attgcaatac ctgtattagg tacttttgca    2040
   cttgtatcat atattgcgaa taaggttcta accgttcaaa caatagataa tgctttaagt    2100
   aaaagaaatg aaaaatggga tgaggtctat aaatatatag taacaaattg gttagcaaag    2160
   gttaatacac agattgatct aataagaaaa aaaatgaaag aagctttaga aaatcaagca    2220
   gaagcaacaa aggctataat aaactatcag tataatcaat atactgagga agagaaaaat    2280
   aatattaatt ttaatattga tgatttaagt tcgaaactta atgagtctat aaataaagct    2340
   atgattaata taaataaatt tttgaatcaa tgctctgttt catatttaat gaattctatg    2400
   atcccttatg gtgttaaacg gttagaagat tttgatgcta gtcttaaaga tgcattatta    2460
   aagtatatat atgataatag aggaacttta attggtcaag tagatagatt aaaagataaa    2520
   gttaataata cacttagtac agatatacct tttcagcttt ccaaatacgt agataatcaa    2580
   agattattat ctacatttac tgaatatatt aagaatattc tcgagcacca ccaccaccac    2640
   cactaa                                                               2646
<210> 4
   <211> 881
   <212> PRT
   <213> Clostridium botulinum
<400> 4
   Met Ala Phe Val Asn Lys Gln Phe Asn Tyr Lys Asp Pro Val Asn Gly
   1               5                   10                  15
   Val Asp Ile Ala Tyr Ile Lys Ile Pro Asn Val Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
   165 170 175
   Arg Asn Gly Tyr Gly Ser Thr Gln Tyr Ile Arg Phe Ser Pro Asp Phe
   180 185 190
   Thr Phe Gly Phe Glu Glu Ser Leu Glu Val Asp Thr Asn Pro Leu Leu
   195 200 205
   Gly Ala Gly Lys Phe Ala Thr Asp Pro Ala Val Thr Leu Ala His Glu
   210 215 220
   Leu Ile His Ala Gly His Arg Leu Tyr Gly Ile Ala Ile Asn Pro Asn
   225 230 235 240
   Arg Val Phe Lys Val Asn Thr Asn Ala Tyr Tyr Glu Met Ser Gly Leu
   245 250 255
   Glu Val Ser Phe Glu Glu Leu Arg Thr Phe Gly Gly His Asp Ala Lys
   260 265 270
   Phe Ile Asp Ser Leu Gln Glu Asn Glu Phe Arg Leu Tyr Tyr Tyr Asn
   275 280 285
   Lys Phe Lys Asp Ile Ala Ser Thr Leu Asn Lys Ala Lys Ser Ile Val
   290 295 300
   Gly Thr Thr Ala Ser Leu Gln Tyr Met Lys Asn Val Phe Lys Glu Lys
   305 310 315 320
   Tyr Leu Leu Ser Glu Asp Thr Ser Gly Lys Phe Ser Val Asp Lys Leu
   325 330 335
   Lys Phe Asp Lys Leu Tyr Lys Met Leu Thr Glu Ile Tyr Thr Glu Asp
   340 345 350
   Asn Phe Val Lys Phe Phe Lys Val Leu Asn Arg Lys Thr Tyr Leu Asn
   355 360 365
   Phe Asp Lys Ala Val Phe Lys Ile Asn Ile Val Pro Lys Val Asn Tyr
   370 375 380
   Thr Ile Tyr Asp Gly Phe Asn Leu Arg Asn Thr Asn Leu Ala Ala Asn
   385 390 395 400
   Phe Asn Gly Gln Asn Thr Glu Ile Asn Asn Met Asn Phe Thr Lys Leu
   405 410 415
   Lys Asn Phe Thr Gly Leu Phe Glu Phe Tyr Lys Leu Leu Cys Val Arg
   420 425 430
   Gly Ile Ile Thr Ser Lys Thr Lys Ser Leu Val Pro Arg Gly Ser Lys
   435 440 445
   Ala Leu Asn Asp Leu Cys Ile Lys Val Asn Asn Trp Asp Leu Phe Phe
   450 455 460
   Ser Pro Ser Glu Asp Asn Phe Thr Asn Asp Leu Asn Lys Gly Glu Glu
   465 470 475 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Leu Glu His His His His His
   865 870 875 880
   His
<210> 5
   <211> 3312
   <212> DNA
   <213> Clostridium botulinum
<400> 5
   atggcatttg ttaataaaca atttaattat aaagatcctg taaatggtgt tgatattgct      60
   tatataaaaa ttccaaatgc aggacaaatg caaccagtaa aagcttttaa aattcataat     120
   aaaatatggg ttattccaga aagagataca tttacaaatc ctgaagaagg agatttaaat     180
   ccaccaccag aagcaaaaca agttccagtt tcatattatg attcaacata tttaagtaca     240
   gataatgaaa aagataatta tttaaaggga gttacaaaat tatttgagag aatttattca     300
   actgatcttg gaagaatgtt gttaacatca atagtaaggg gaataccatt ttggggtgga     360
   agtacaatag atacagaatt aaaagttatt gatactaatt gtattaatgt gatacaacca     420
   gatggtagtt atagatcaga agaacttaat ctagtaataa taggaccctc agctgatatt     480
   atacagtttg aatgtaaaag ctttggacat gaagttttga atcttacgcg aaatggttat     540
   ggctctactc aatacattag atttagccca gattttacat ttggttttga ggagtcactt     600
   gaagttgata caaatcctct tttaggtgca ggcaaatttg ctacagatcc agcagtaaca     660
   ttagcacatg aacttataca tgctggacat agattatatg gaatagcaat taatccaaat     720
   agggttttta aagtaaatac taatgcctat tatgaaatga gtgggttaga agtaagcttt     780
   gaggaactta gaacatttgg gggacatgat gcaaagttta tagatagttt acaggaaaac     840
   gaatttcgtc tatattatta taataagttt aaagatatag caagtacact taataaagct     900
   aaatcaatag taggtactac tgcttcatta cagtatatga aaaatgtttt taaagagaaa     960
   tatctcctat ctgaagatac atctggaaaa ttttcggtag ataaattaaa atttgataag    1020
   ttatacaaaa tgttaacaga gatttacaca gaggataatt ttgttaagtt ttttaaagta    1080
   cttaacagaa aaacatattt gaattttgat aaagccgtat ttaagataaa tatagtacct    1140
   aaggtaaatt acacaatata tgatggattt aatttaagaa atacaaattt agcagcaaac    1200
   tttaatggtc aaaatacaga aattaataat atgaatttta ctaaactaaa aaattttact    1260
   ggattgtttg aattttataa gttgctatgt gtaagaggga taataacttc taaaactaaa    1320
   tcattagttc cgcgtggatc caaggcctta aatgatttat gtatcaaagt taataattgg    1380
   gacttgtttt ttagtccttc agaagataat tttactaatg atctaaataa aggagaagaa    1440
   attacatctg atactaatat agaagcagca gaagaaaata ttagtttaga tttaatacaa    1500
   caatattatt taacctttaa ttttgataat gaacctgaaa atatttcaat agaaaatctt    1560
   tcaagtgaca ttataggcca attagaactt atgcctaata tagaaagatt tcctaatgga    1620
   aaaaagtatg agttagataa atatactatg ttccattatc ttcgtgctca agaatttgaa    1680
   catggtaaat ctaggattgc tttaacaaat tctgttaacg aagcattatt aaatcctagt    1740
   cgtgtttata catttttttc ttcagactat gtaaagaaag ttaataaagc tacggaggca    1800
   gctatgtttt taggctgggt agaacaatta gtatatgatt ttaccgatga aactagcgaa    1860
   gtaagtacta cggataaaat tgcggatata actataatta ttccatatat aggacctgct    1920
   ttaaatatag gtaatatgtt atataaagat gattttgtag gtgctttaat attttcagga    1980
   gctgttattc tgttagaatt tataccagag attgcaatac ctgtattagg tacttttgca    2040
   cttgtatcat atattgcgaa taaggttcta accgttcaaa caatagataa tgctttaagt    2100
   aaaagaaatg aaaaatggga tgaggtctat aaatatatag taacaaattg gttagcaaag    2160
   gttaatacac agattgatct aataagaaaa aaaatgaaag aagctttaga aaatcaagca    2220
   gaagcaacaa aggctataat aaactatcag tataatcaat atactgagga agagaaaaat    2280
   aatattaatt ttaatattga tgatttaagt tcgaaactta atgagtctat aaataaagct    2340
   atgattaata taaataaatt tttgaatcaa tgctctgttt catatttaat gaattctatg    2400
   atcccttatg gtgttaaacg gttagaagat tttgatgcta gtcttaaaga tgcattatta    2460
   aagtatatat atgataatag aggaacttta attggtcaag tagatagatt aaaagataaa    2520
   gttaataata cacttagtac agatatacct tttcagcttt ccaaatacgt agataatcaa    2580
   agattattat ctacatttac tgaatatatt aagaatatta ttaatacttc tatattgaat    2640
   ttaagatatg aaagtaatca tttaatagac ttatctaggt atgcatcaaa aataaatatt    2700
   ggtagtaaag taaattttga tccaatagat aaaaatcaaa ttcaattatt taatttagaa    2760
   agtagtaaaa ttgaggtaat tttaaaaaat gctattgtat ataatagtat gtatgaaaat    2820
   tttagtacta gcttttggat aagaattcct aagtatttta acagtataag tctaaataat    2880
   gaatatacaa taataaattg tatggaaaat aattcaggat ggaaagtatc acttaattat    2940
   ggtgaaataa tctggacttt acaggatact caggaaataa aacaaagagt agtttttaaa    3000
   tacagtcaaa tgattaatat atcagattat ataaacagat ggatttttgt aactatcact    3060
   aataatagat taaataactc taaaatttat ataaatggaa gattaataga tcaaaaacca    3120
   atttcaaatt taggtaatat tcatgctagt aataatataa tgtttaaatt agatggttgt    3180
   agagatacac atagatatat ttggataaaa tattttaatc tttttgataa ggaattaaat    3240
   gaaaaagaaa tcaaagattt atatgataat caatcaaatt caggtctcga gcaccaccac    3300
   caccaccact aa                                                        3312
<210> 6
   <211> 1103
   <212> PRT
   <213> Clostridium botulinum
<400> 6
   Met Ala Phe Val Asn Lys Gln Phe Asn Tyr Lys Asp Pro Val Asn Gly
   1               5                   10                  15
   Val Asp Ile Ala Tyr Ile Lys Ile Pro Asn Val Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile His Ala Gly His Arg Leu Tyr Gly Ile Ala Ile Asn Pro Asn
   225 230 235 240
   Arg Val Phe Lys Val Asn Thr Asn Ala Tyr Tyr Glu Met Ser Gly Leu
   245 250 255
   Glu Val Ser Phe Glu Glu Leu Arg Thr Phe Gly Gly His Asp Ala Lys
   260 265 270
   Phe Ile Asp Ser Leu Gln Glu Asn Glu Phe Arg Leu Tyr Tyr Tyr Asn
   275 280 285
   Lys Phe Lys Asp Ile Ala Ser Thr Leu Asn Lys Ala Lys Ser Ile Val
   290 295 300
   Gly Thr Thr Ala Ser Leu Gln Tyr Met Lys Asn Val Phe Lys Glu Lys
   305 310 315 320
   Tyr Leu Leu Ser Glu Asp Thr Ser Gly Lys Phe Ser Val Asp Lys Leu
   325 330 335
   Lys Phe Asp Lys Leu Tyr Lys Met Leu Thr Glu Ile Tyr Thr Glu Asp
   340 345 350
   Asn Phe Val Lys Phe Phe Lys Val Leu Asn Arg Lys Thr Tyr Leu Asn
   355 360 365
   Phe Asp Lys Ala Val Phe Lys Ile Asn Ile Val Pro Lys Val Asn Tyr
   370 375 380
   Thr Ile Tyr Asp Gly Phe Asn Leu Arg Asn Thr Asn Leu Ala Ala Asn
   385 390 395 400
   Phe Asn Gly Gln Asn Thr Glu Ile Asn Asn Met Asn Phe Thr Lys Leu
   405 410 415
   Lys Asn Phe Thr Gly Leu Phe Glu Phe Tyr Lys Leu Leu Cys Val Arg
   420 425 430
   Gly Ile Ile Thr Ser Lys Thr Lys Ser Leu Val Pro Arg Gly Ser Lys
   435 440 445
   Ala Leu Asn Asp Leu Cys Ile Lys Val Asn Asn Trp Asp Leu Phe Phe
   450 455 460
   Ser Pro Ser Glu Asp Asn Phe Thr Asn Asp Leu Asn Lys Gly Glu Glu
   465 470 475 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Ile Asn Thr Ser Ile Leu Asn
   865 870 875 880
   Leu Arg Tyr Glu Ser Asn His Leu Ile Asp Leu Ser Arg Tyr Ala Ser
   885 890 895
   Lys Ile Asn Ile Gly Ser Lys Val Asn Phe Asp Pro Ile Asp Lys Asn
               900                     905                     910
   Gln Ile Gln Leu Phe Asn Leu Glu Ser Ser Lys Ile Glu Val Ile Leu
           915                     920                     925
   Lys Asn Ala Ile Val Tyr Asn Ser Met Tyr Glu Asn Phe Ser Thr Ser
       930                     935                     940
   Phe Trp Ile Arg Ile Pro Lys Tyr Phe Asn Ser Ile Ser Leu Asn Asn
   945                     950                     955                     960
   Glu Tyr Thr Ile Ile Asn Cys Met Glu Asn Asn Ser Gly Trp Lys Val
                   965                     970                     975
   Ser Leu Asn Tyr Gly Glu Ile Ile Trp Thr Leu Gln Asp Thr Gln Glu
               980                     985                     990
   Ile Lys Gln Arg Val Val Phe Lys  Tyr Ser Gln Met Ile  Asn Ile Ser
           995                     1000                    1005
   Asp Tyr  Ile Asn Arg Trp Ile  Phe Val Thr Ile Thr  Asn Asn Arg
       1010                     1015                    1020
   Leu Asn  Asn Ser Lys Ile Tyr  Ile Asn Gly Arg Leu  Ile Asp Gln
       1025                     1030                    1035
   Lys Pro  Ile Ser Asn Leu Gly  Asn Ile His Ala Ser  Asn Asn Ile
       1040                     1045                    1050
   Met Phe  Lys Leu Asp Gly Cys  Arg Asp Thr His Arg  Tyr Ile Trp
       1055                     1060                    1065
   Ile Lys  Tyr Phe Asn Leu Phe  Asp Lys Glu Leu Asn  Glu Lys Glu
       1070                     1075                    1080
   Ile Lys  Asp Leu Tyr Asp Asn  Gln Ser Asn Ser Gly  Leu Glu His
       1085                     1090                    1095
   His His  His His His
       1100
<210> 7
   <211> 3942
   <212> DNA
   <213> Clostridium botulinum
<400> 7
   atggcagtta caataaataa ttttaactat aatgatccta ttgataataa taatattatt    60
   atgatggagc ctccatttgc gagaggtacg gggagatatt ataaagcttt taaaatcaca   120
   gatcgtattt ggataatacc ggaaagatat acttttggat ataaacctga ggattttaat   180
   aaaagttccg gtatttttaa tagagatgtt tgtgaatatt atgatccaga ttacttaaat   240
   actaatgata aaaagaatat atttttacaa acaatgatca agttatttaa tagaatcaaa   300
   tcaaaaccat tgggtgaaaa gttattagag atgattataa atggtatacc ttatcttgga   360
   gatagacgtg ttccactcga agagtttaac acaaacattg ctagtgtaac tgttaataaa   420
   ttaatcagta atccaggaga agtggagcga aaaaaaggta ttttcgcaaa tttaataata   480
   tttggacctg ggccagtttt aaatgaaaat gagactatag atataggtat acaaaatcat   540
   tttgcatcaa gggaaggctt cgggggtata atgcaaatga agttttgccc agaatatgta   600
   agcgtattta ataatgttca agaaaacaaa ggcgcaagta tatttaatag acgtggatat   660
   ttttcagatc cagccttgat attaatgcat gaacttatac atgttttaca tggattatat   720
   ggcattaaag tagatgattt accaattgta ccaaatgaaa aaaaattttt tatgcaatct   780
   acagatgcta tacaggcaga agaactatat acatttggag gacaagatcc cagcatcata   840
   actccttcta cggataaaag tatctatgat aaagttttgc aaaattttag agggatagtt   900
   gatagactta acaaggtttt agtttgcata tcagatccta acattaatat taatatatat   960
   aaaaataaat ttaaagataa atataaattc gttgaagatt ctgagggaaa atatagtata  1020
   gatgtagaaa gttttgataa attatataaa agcttaatgt ttggttttac agaaactaat  1080
   atagcagaaa attataaaat aaaaactaga gcttcttatt ttagtgattc cttaccacca  1140
   gtaaaaataa aaaatttatt agataatgaa atctatacta tagaggaagg gtttaatata  1200
   tctgataaag atatggaaaa agaatataga ggtcagaata aagctataaa taaacaagct  1260
   tatgaagaaa ttagcaagga gcatttggct gtatataaga tacaaatgtg taaaagtggg  1320
   ataataactt ctaaaactaa atcattggtt ccacgtggat ccaaggcctc aggaatatgt  1380
   attgatgttg ataatgaaga tttgttcttt atagctgata aaaatagttt ttcagatgat  1440
   ttatctaaaa acgaaagaat agaatataat acacagagta attatataga aaatgacttc  1500
   cctataaatg aattaatttt agatactgat ttaataagta aaatagaatt accaagtgaa  1560
   aatacagaat cacttactga ttttaatgta gatgttccag tatatgaaaa acaacccgct  1620
   ataaaaaaaa tttttacaga tgaaaatacc atctttcaat atttatactc tcagacattt  1680
   cctctagata taagagatat aagtttaaca tcttcatttg atgatgcatt attattttct  1740
   aacaaagttt attcattttt ttctatggat tatattaaaa ctgctaataa agtggtagaa  1800
   gcaggattat ttgcaggttg ggtgaaacag atagtaaatg attttgtaat cgaagctaat  1860
   aaaagcaata ctatggataa aattgcagat atatctctaa ttgttcctta tataggatta  1920
   gctttaaatg taggaaatga aacagctaaa ggaaattttg aaaatgcttt tgagattgca  1980
   ggagccagta ttctactaga atttatacca gaacttttaa tacctgtagt tggagccttt  2040
   ttattagaat catatattga caataaaaat aaaattatta aaacaataga taatgcttta  2100
   actaaaagaa atgaaaaatg gagtgatatg tacggattaa tagtagcgca atggctctca  2160
   acagttaata ctcaatttta tacaataaaa gagggaatgt ataaggcttt aaattatcaa  2220
   gcacaagcat tggaagaaat aataaaatac agatataata tatattctga aaaagaaaag  2280
   tcaaatatta acatcgattt taatgatata aattctaaac ttaatgaggg tattaaccaa  2340
   gctatagata atataaataa ttttataaat ggatgttctg tatcatattt aatgaaaaaa  2400
   atgattccat tagctgtaga aaaattacta gactttgata atactctcaa aaaaaatttg  2460
   ttaaattata tagatgaaaa taaattatat ttgattggaa gtgcagaata tgaaaaatca  2520
   aaagtaaata aatacttgaa aaccattatg ccgtttgatc tttcaatata taccaatgat  2580
   acaatactaa tagaaatgtt taataaatat aatagcgaaa ttttaaataa tattatctta  2640
   aatttaagat ataaggataa taatttaata gatttatcag gatatggggc aaaggtagag  2700
   gtatatgatg gagtcgagct taatgataaa aatcaattta aattaactag ttcagcaaat  2760
   agtaagatta gagtgactca aaatcagaat atcatattta atagtgtgtt ccttgatttt  2820
   agcgttagct tttggataag aatacctaaa tataagaatg atggtataca aaattatatt  2880
   cataatgaat atacaataat taattgtatg aaaaataatt cgggctggaa aatatctatt  2940
   aggggtaata ggataatatg gactttaatt gatataaatg gaaaaaccaa atcggtattt  3000
   tttgaatata acataagaga agatatatca gagtatataa atagatggtt ttttgtaact  3060
   attactaata atttgaataa cgctaaaatt tatattaatg gtaagctaga atcaaataca  3120
   gatattaaag atataagaga agttattgct aatggtgaaa taatatttaa attagatggt  3180
   gatatagata gaacacaatt tatttggatg aaatatttca gtatttttaa tacggaatta  3240
   agtcaatcaa atattgaaga aagatataaa attcaatcat atagcgaata tttaaaagat  3300
   ttttggggaa atcctttaat gtacaataaa gaatattata tgtttaatgc ggggaataaa  3360
   aattcatata ttaaactaaa gaaagattca cctgtaggtg aaattttaac acgtagcaaa  3420
   tataatcaaa attctaaata tataaattat agagatttat atattggaga aaaatttatt  3480
   ataagaagaa agtcaaattc tcaatctata aatgatgata tagttagaaa agaagattat  3540
   atatatctag atttttttaa tttaaatcaa gagtggagag tatataccta taaatatttt  3600
   aagaaagagg aagaaaaatt gtttttagct cctataagtg attctgatga gttttacaat  3660
   actatacaaa taaaagaata tgatgaacag ccaacatata gttgtcagtt gctttttaaa  3720
   aaagatgaag aaagtactga tgagatagga ttgattggta ttcatcgttt ctacgaatct  3780
   ggaattgtat ttgaagagta taaagattat ttttgtataa gtaaatggta cttaaaagag  3840
   gtaaaaagga aaccatataa tttaaaattg ggatgtaatt ggcagtttat tcctaaagat  3900
   gaagggtgga ctgaactcga gcaccaccac caccaccact aa                      3942
<210> 8
   <211> 1313
   <212> PRT
   <213> Clostridium botulinum
<400> 8
   Met Ala Val Thr Ile Asn Asn Phe Asn Tyr Asn Asp Pro Ile Asp Asn
   1               5                   10                  15
   Asn Asn Ile Ile Met Met Glu Pro Pro Phe Ala Arg Gly Thr Gly Arg
               20                  25                  30
   Tyr Tyr Lys Ala Phe Lys Ile Thr Asp Arg Ile Trp Ile Ile Pro Glu
           35                  40                  45
   Arg Tyr Thr Phe Gly Tyr Lys Pro Glu Asp Phe Asn Lys Ser Ser Gly
       50                  55                  60
   Ile Phe Asn Arg Asp Val Cys Glu Tyr Tyr Asp Pro Asp Tyr Leu Asn
   65                  70                  75                  80
   Thr Asn Asp Lys Lys Asn Ile Phe Leu Gln Thr Met Ile Lys Leu Phe
                   85                  90                  95
   Asn Arg Ile Lys Ser Lys Pro Leu Gly Glu Lys Leu Leu Glu Met Ile
               100                 105                 110
   Ile Asn Gly Ile Pro Tyr Leu Gly Asp Arg Arg Val Pro Leu Glu Glu
           115                 120                 125
   Phe Asn Thr Asn Ile Ala Ser Val Thr Val Asn Lys Leu Ile Ser Asn
       130                 135                 140
   Pro Gly Glu Val Glu Arg Lys Lys Gly Ile Phe Ala Asn Leu Ile Ile
   145                 150                 155                 160
   Phe Gly Pro Gly Pro Val Leu Asn Glu Asn Glu Thr Ile Asp Ile Gly
                   165                 170                 175
   Ile Gln Asn His Phe Ala Ser Arg Glu Gly Phe Gly Gly Ile Met Gln
               180                 185                 190
   Met Lys Phe Cys Pro Glu Tyr Val Ser Val Phe Asn Asn Val Gln Glu
           195                 200                 205
   Asn Lys Gly Ala Ser Ile Phe Asn Arg Arg Gly Tyr Phe Ser Asp Pro
       210                 215                 220
   Ala Leu Ile Leu Met His Glu Leu Ile His Val Leu His Gly Leu Tyr
   225                 230                 235                 240
   Gly Ile Lys Val Asp Asp Leu Pro Ile Val Pro Asn Glu Lys Lys Phe
                   245                 250                 255
   Phe Met Gln Ser Thr Asp Ala Ile Gln Ala Glu Glu Leu Tyr Thr Phe
               260                 265                 270
   Gly Gly Gln Asp Pro Ser Ile Ile Thr Pro Ser Thr Asp Lys Ser Ile
           275                 280                 285
   Tyr Asp Lys Val Leu Gln Asn Phe Arg Gly Ile Val Asp Arg Leu Asn
       290                 295                 300
   Lys Val Leu Val Cys Ile Ser Asp Pro Asn Ile Asn Ile Asn Ile Tyr
   305                 310                 315                 320
   Lys Asn Lys Phe Lys Asp Lys Tyr Lys Phe Val Glu Asp Ser Glu Gly
                   325                 330                 335
   Lys Tyr Ser Ile Asp Val Glu Ser Phe Asp Lys Leu Tyr Lys Ser Leu
   340 345 350
   Met Phe Gly Phe Thr Glu Thr Asn Ile Ala Glu Asn Tyr Lys Ile Lys
   355 360 365
   Thr Arg Ala Ser Tyr Phe Ser Asp Ser Leu Pro Pro Val Lys Ile Lys
   370 375 380
   Asn Leu Leu Asp Asn Glu Ile Tyr Thr Ile Glu Glu Gly Phe Asn Ile
   385 390 395 400
   Ser Asp Lys Asp Met Glu Lys Glu Tyr Arg Gly Gln Asn Lys Ala Ile
   405 410 415
   Asn Lys Gln Ala Tyr Glu Glu Ile Ser Lys Glu His Leu Ala Val Tyr
   420 425 430
   Lys Ile Gln Met Cys Lys Ser Gly Ile Ile Thr Ser Lys Thr Lys Ser
   435 440 445
   Leu Val Pro Arg Gly Ser Lys Ala Ser Gly Ile Cys Ile Asp Val Asp
   450 455 460
   Asn Glu Asp Leu Phe Phe Ile Ala Asp Lys Asn Ser Phe Ser Asp Asp
   465 470 475 480
   Leu Ser Lys Asn Glu Arg Ile Glu Tyr Asn Thr Gln Ser Asn Tyr Ile
   485 490 495
   Glu Asn Asp Phe Pro Ile Asn Glu Leu Ile Leu Asp Thr Asp Leu Ile
   500 505 510
   Ser Lys Ile Glu Leu Pro Ser Glu Asn Thr Glu Ser Leu Thr Asp Phe
   515 520 525
   Asn Val Asp Val Pro Val Tyr Glu Lys Gln Pro Ala Ile Lys Lys Ile
   530 535 540
   Phe Thr Asp Glu Asn Thr Ile Phe Gln Tyr Leu Tyr Ser Gln Thr Phe
   545 550 555 560
   Pro Leu Asp Ile Arg Asp Ile Ser Leu Thr Ser Ser Phe Asp Asp Ala
   565 570 575
   Leu Leu Phe Ser Asn Lys Val Tyr Ser Phe Phe Ser Met Asp Tyr Ile
   580 585 590
   Lys Thr Ala Asn Lys Val Val Glu Ala Gly Leu Phe Ala Gly Trp Val
   595 600 605
   Lys Gln Ile Val Asn Asp Phe Val Ile Glu Ala Asn Lys Ser Asn Thr
   610 615 620
   Met Asp Lys Ile Ala Asp Ile Ser Leu Ile Val Pro Tyr Ile Gly Leu
   625 630 635 640
   Ala Leu Asn Val Gly Asn Glu Thr Ala Lys Gly Asn Phe Glu Asn Ala
   645 650 655
   Phe Glu Ile Ala Gly Ala Ser Ile Leu Leu Glu Phe Ile Pro Glu Leu
   660 665 670
   Leu Ile Pro Val Val Gly Ala Phe Leu Leu Glu Ser Tyr Ile Asp Asn
   675 680 685
   Lys Asn Lys Ile Ile Lys Thr Ile Asp Asn Ala Leu Thr Lys Arg Asn
   690 695 700
   Glu Lys Trp Ser Asp Met Tyr Gly Leu Ile Val Ala Gln Trp Leu Ser
   705 710 715 720
   Thr Val Asn Thr Gln Phe Tyr Thr Ile Lys Glu Gly Met Tyr Lys Ala
   725 730 735
   Leu Asn Tyr Gln Ala Gln Ala Leu Glu Glu Ile Ile Lys Tyr Arg Tyr
   740 745 750
   Asn Ile Tyr Ser Glu Lys Glu Lys Ser Asn Ile Asn Ile Asp Phe Asn
   755 760 765
   Asp Ile Asn Ser Lys Leu Asn Glu Gly Ile Asn Gln Ala Ile Asp Asn
   770 775 780
   Ile Asn Asn Phe Ile Asn Gly Cys Ser Val Ser Tyr Leu Met Lys Lys
   785 790 795 800
   Met Ile Pro Leu Ala Val Glu Lys Leu Leu Asp Phe Asp Asn Thr Leu
   805 810 815
   Lys Lys Asn Leu Leu Asn Tyr Ile Asp Glu Asn Lys Leu Tyr Leu Ile
   820 825 830
   Gly Ser Ala Glu Tyr Glu Lys Ser Lys Val Asn Lys Tyr Leu Lys Thr
   835 840 845
   Ile Met Pro Phe Asp Leu Ser Ile Tyr Thr Asn Asp Thr Ile Leu Ile
   850 855 860
   Glu Met Phe Asn Lys Tyr Asn Ser Glu Ile Leu Asn Asn Ile Ile Leu
   865 870 875 880
   Asn Leu Arg Tyr Lys Asp Asn Asn Leu Ile Asp Leu Ser Gly Tyr Gly
   885 890 895
   Ala Lys Val Glu Val Tyr Asp Gly Val Glu Leu Asn Asp Lys Asn Gln
   900 905 910
   Phe Lys Leu Thr Ser Ser Ala Asn Ser Lys Ile Arg Val Thr Gln Asn
   915 920 925
   Gln Asn Ile Ile Phe Asn Ser Val Phe Leu Asp Phe Ser Val Ser Phe
   930 935 940
   Trp Ile Arg Ile Pro Lys Tyr Lys Asn Asp Gly Ile Gln Asn Tyr Ile
   945 950 955 960
   His Asn Glu Tyr Thr Ile Ile Asn Cys Met Lys Asn Asn Ser Gly Trp
   965 970 975
   Lys Ile Ser Ile Arg Gly Asn Arg Ile Ile Trp Thr Leu Ile Asp Ile
   980 985 990
   Asn Gly Lys Thr Lys Ser Val Phe Phe Glu Tyr Asn Ile Arg Glu Asp
   995 1000 1005
   Ile Ser Glu Tyr Ile Asn Arg Trp Phe Phe Val Thr Ile Thr Asn
   1010 1015 1020
   Asn Leu Asn Asn Ala Lys Ile Tyr Ile Asn Gly Lys Leu Glu Ser
   1025 1030 1035
   Asn Thr Asp Ile Lys Asp Ile Arg Glu Val Ile Ala Asn Gly Glu
   1040 1045 1050
   Ile Ile Phe Lys Leu Asp Gly Asp Ile Asp Arg Thr Gln Phe Ile
   1055 1060 1065
   Trp Met Lys Tyr Phe Ser Ile Phe Asn Thr Glu Leu Ser Gln Ser
   1070 1075 1080
   Asn Ile Glu Glu Arg Tyr Lys Ile Gln Ser Tyr Ser Glu Tyr Leu
   1085 1090 1095
   Lys Asp Phe Trp Gly Asn Pro Leu Met Tyr Asn Lys Glu Tyr Tyr
   1100 1105 1110
   Met Phe Asn Ala Gly Asn Lys Asn Ser Tyr Ile Lys Leu Lys Lys
   1115 1120 1125
   Asp Ser Pro Val Gly Glu Ile Leu Thr Arg Ser Lys Tyr Asn Gln
   1130 1135 1140
   Asn Ser Lys Tyr Ile Asn Tyr Arg Asp Leu Tyr Ile Gly Glu Lys
   1145 1150 1155
   Phe Ile Ile Arg Arg Lys Ser Asn Ser Gln Ser Ile Asn Asp Asp
   1160 1165 1170
   Ile Val Arg Lys Glu Asp Tyr Ile Tyr Leu Asp Phe Phe Asn Leu
   1175 1180 1185
   Asn Gln Glu Trp Arg Val Tyr Thr Tyr Lys Tyr Phe Lys Lys Glu
   1190 1195 1200
   Glu Glu Lys Leu Phe Leu Ala Pro Ile Ser Asp Ser Asp Glu Phe
   1205 1210 1215
   Tyr Asn Thr Ile Gln Ile Lys Glu Tyr Asp Glu Gln Pro Thr Tyr
   1220 1225 1230
   Ser Cys Gln Leu Leu Phe Lys Lys Asp Glu Glu Ser Thr Asp Glu
   1235 1240 1245
   Ile Gly Leu Ile Gly Ile His Arg Phe Tyr Glu Ser Gly Ile Val
   1250 1255 1260
   Phe Glu Glu Tyr Lys Asp Tyr Phe Cys Ile Ser Lys Trp Tyr Leu
   1265 1270 1275
   Lys Glu Val Lys Arg Lys Pro Tyr Asn Leu Lys Leu Gly Cys Asn
   1280 1285 1290
   Trp Gln Phe Ile Pro Lys Asp Glu Gly Trp Thr Glu Leu Glu His
   1295 1300 1305
   His His His His His
   1310
<210> 9
   <211> 2649
   <212> DNA
   <213> Clostridium botulinum
<400> 9
   atggcagtta caataaataa ttttaattat aatgatccta ttgataataa taatattatt      60
   atgatggagc ctccatttgc gagaggtacg gggagatatt ataaagcttt taaaatcaca     120
   gatcgtattt ggataatacc ggaaagatat acttttggat ataaacctga ggattttaat     180
   aaaagttccg gtatttttaa tagagatgtt tgtgaatatt atgatccaga ttacttaaat     240
   actaatgata aaaagaatat atttttacaa acaatgatca agttatttaa tagaatcaaa     300
   tcaaaaccat tgggtgaaaa gttattagag atgattataa atggtatacc ttatcttgga     360
   gatagacgtg ttccactcga agagtttaac acaaacattg ctagtgtaac tgttaataaa     420
   ttaatcagta atccaggaga agtggagcga aaaaaaggta ttttcgcaaa tttaataata     480
   tttggacctg ggccagtttt aaatgaaaat gagactatag atataggtat acaaaatcat     540
   tttgcatcaa gggaaggctt cgggggtata atgcaaatga agttttgccc agaatatgta     600
   agcgtattta ataatgttca agaaaacaaa ggcgcaagta tatttaatag acgtggatat     660
   ttttcagatc cagccttgat attaatgcat gaacttatac atgttttaca tggattatat     720
   ggcattaaag tagatgattt accaattgta ccaaatgaaa aaaaattttt tatgcaatct     780
   acagatgcta tacaggcaga agaactatat acatttggag gacaagatcc cagcatcata     840
   actccttcta cggataaaag tatctatgat aaagttttgc aaaattttag agggatagtt     900
   gatagactta acaaggtttt agtttgcata tcagatccta acattaatat taatatatat     960
   aaaaataaat ttaaagataa atataaattc gttgaagatt ctgagggaaa atatagtata    1020
   gatgtagaaa gttttgataa attatataaa agcttaatgt ttggttttac agaaactaat    1080
   atagcagaaa attataaaat aaaaactaga gcttcttatt ttagtgattc cttaccacca    1140
   gtaaaaataa aaaatttatt agataatgaa atctatacta tagaggaagg gtttaatata    1200
   tctgataaag atatggaaaa agaatataga ggtcagaata aagctataaa taaacaagct    1260
   tatgaagaaa ttagcaagga gcatttggct gtatataaga tacaaatgtg taaaagtggg    1320
   ataataactt ctaaaactaa atcattggtt ccacgtggat ccaaggcctc aggaatatgt    1380
   attgatgttg ataatgaaga tttgttcttt atagctgata aaaatagttt ttcagatgat    1440
   ttatctaaaa acgaaagaat agaatataat acacagagta attatataga aaatgacttc    1500
   cctataaatg aattaatttt agatactgat ttaataagta aaatagaatt accaagtgaa    1560
   aatacagaat cacttactga ttttaatgta gatgttccag tatatgaaaa acaacccgct    1620
   ataaaaaaaa tttttacaga tgaaaatacc atctttcaat atttatactc tcagacattt    1680
   cctctagata taagagatat aagtttaaca tcttcatttg atgatgcatt attattttct    1740
   aacaaagttt attcattttt ttctatggat tatattaaaa ctgctaataa agtggtagaa    1800
   gcaggattat ttgcaggttg ggtgaaacag atagtaaatg attttgtaat cgaagctaat    1860
   aaaagcaata ctatggataa aattgcagat atatctctaa ttgttcctta tataggatta    1920
   gctttaaatg taggaaatga aacagctaaa ggaaattttg aaaatgcttt tgagattgca    1980
   ggagccagta ttctactaga atttatacca gaacttttaa tacctgtagt tggagccttt    2040
   ttattagaat catatattga caataaaaat aaaattatta aaacaataga taatgcttta    2100
   actaaaagaa atgaaaaatg gagtgatatg tacggattaa tagtagcgca atggctctca    2160
   acagttaata ctcaatttta tacaataaaa gagggaatgt ataaggcttt aaattatcaa    2220
   gcacaagcat tggaagaaat aataaaatac agatataata tatattctga aaaagaaaag    2280
   tcaaatatta acatcgattt taatgatata aattctaaac ttaatgaggg tattaaccaa    2340
   gctatagata atataaataa ttttataaat ggatgttctg tatcatattt aatgaaaaaa    2400
   atgattccat tagctgtaga aaaattacta gactttgata atactctcaa aaaaaatttg    2460
   ttaaattata tagatgaaaa taaattatat ttgattggaa gtgcagaata tgaaaaatca    2520
   aaagtaaata aatacttgaa aaccattatg ccgtttgatc tttcaatata taccaatgat    2580
   acaatactaa tagaaatgtt taataaatat aatagcgaaa ttctcgagca ccaccaccac    2640
   caccactaa                                                             2649
<210> 10
   <211> 882
   <212> PRT
   <213> Clostridium botulinum
<400> 10
   Met Ala Val Thr Ile Asn Asn Phe Asn Tyr Asn Asp Pro Ile Asp Asn
   1               5                   10                  15
   Asn Asn Ile Ile Met Met Glu Pro Pro Phe Ala Arg Gly Thr Gly Arg
               20                  25                  30
   Tyr Tyr Lys Ala Phe Lys Ile Thr Asp Arg Ile Trp Ile Ile Pro Glu
           35                  40                  45
   Arg Tyr Thr Phe Gly Tyr Lys Pro Glu Asp Phe Asn Lys Ser Ser Gly
       50                  55                  60
   Ile Phe Asn Arg Asp Val Cys Glu Tyr Tyr Asp Pro Asp Tyr Leu Asn
   65                  70                  75                  80
   Thr Asn Asp Lys Lys Asn Ile Phe Leu Gln Thr Met Ile Lys Leu Phe
                   85                  90                  95
   Asn Arg Ile Lys Ser Lys Pro Leu Gly Glu Lys Leu Leu Glu Met Ile
               100                 105                 110
   Ile Asn Gly Ile Pro Tyr Leu Gly Asp Arg Arg Val Pro Leu Glu Glu
           115                 120                 125
   Phe Asn Thr Asn Ile Ala Ser Val Thr Val Asn Lys Leu Ile Ser Asn
       130                 135                 140
   Pro Gly Glu Val Glu Arg Lys Lys Gly Ile Phe Ala Asn Leu Ile Ile
   145                 150                 155                 160
   Phe Gly Pro Gly Pro Val Leu Asn Glu Asn Glu Thr Ile Asp Ile Gly
                   165                 170                 175
   Ile Gln Asn His Phe Ala Ser Arg Glu Gly Phe Gly Gly Ile Met Gln
               180                 185                 190
   Met Lys Phe Cys Pro Glu Tyr Val Ser Val Phe Asn Asn Val Gln Glu
           195                 200                 205
   Asn Lys Gly Ala Ser Ile Phe Asn Arg Arg Gly Tyr Phe Ser Asp Pro
       210                 215                 220
   Ala Leu Ile Leu Met His Glu Leu Ile His Val Leu His Gly Leu Tyr
   225                 230                 235                 240
   Gly Ile Lys Val Asp Asp Leu Pro Ile Val Pro Asn Glu Lys Lys Phe
                   245                 250                 255
   Phe Met Gln Ser Thr Asp Ala Ile Gln Ala Glu Glu Leu Tyr Thr Phe
               260                 265                 270
   Gly Gly Gln Asp Pro Ser Ile Ile Thr Pro Ser Thr Asp Lys Ser Ile
           275                 280                 285
   Tyr Asp Lys Val Leu Gln Asn Phe Arg Gly Ile Val Asp Arg Leu Asn
       290                 295                 300
   Lys Val Leu Val Cys Ile Ser Asp Pro Asn Ile Asn Ile Asn Ile Tyr
   305                 310                 315                 320
   Lys Asn Lys Phe Lys Asp Lys Tyr Lys Phe Val Glu Asp Ser Glu Gly
                   325                 330                 335
   Lys Tyr Ser Ile Asp Val Glu Ser Phe Asp Lys Leu Tyr Lys Ser Leu
               340                 345                 350
   Met Phe Gly Phe Thr Glu Thr Asn Ile Ala Glu Asn Tyr Lys Ile Lys
           355                 360                 365
   Thr Arg Ala Ser Tyr Phe Ser Asp Ser Leu Pro Pro Val Lys Ile Lys
       370                 375                 380
   Asn Leu Leu Asp Asn Glu Ile Tyr Thr Ile Glu Glu Gly Phe Asn Ile
   385                 390                 395                 400
   Ser Asp Lys Asp Met Glu Lys Glu Tyr Arg Gly Gln Asn Lys Ala Ile
                   405                 410                 415
   Asn Lys Gln Ala Tyr Glu Glu Ile Ser Lys Glu His Leu Ala Val Tyr
               420                 425                 430
   Lys Ile Gln Met Cys Lys Ser Gly Ile Ile Thr Ser Lys Thr Lys Ser
           435                 440                 445
   Leu Val Pro Arg Gly Ser Lys Ala Ser Gly Ile Cys Ile Asp Val Asp
       450                 455                 460
   Asn Glu Asp Leu Phe Phe Ile Ala Asp Lys Asn Ser Phe Ser Asp Asp
   465                 470                 475                 480
   Leu Ser Lys Asn Glu Arg Ile Glu Tyr Asn Thr Gln Ser Asn Tyr Ile
                   485                 490                 495
   Glu Asn Asp Phe Pro Ile Asn Glu Leu Ile Leu Asp Thr Asp Leu Ile
               500                 505                 510
   Ser Lys Ile Glu Leu Pro Ser Glu Asn Thr Glu Ser Leu Thr Asp Phe
           515                 520                 525
   Asn Val Asp Val Pro Val Tyr Glu Lys Gln Pro Ala Ile Lys Lys Ile
       530                 535                 540
   Phe Thr Asp Glu Asn Thr Ile Phe Gln Tyr Leu Tyr Ser Gln Thr Phe
   545                 550                 555                 560
   Pro Leu Asp Ile Arg Asp Ile Ser Leu Thr Ser Ser Phe Asp Asp Ala
                   565                 570                 575
   Leu Leu Phe Ser Asn Lys Val Tyr Ser Phe Phe Ser Met Asp Tyr Ile
               580                 585                 590
   Lys Thr Ala Asn Lys Val Val Glu Ala Gly Leu Phe Ala Gly Trp Val
           595                 600                 605
   Lys Gln Ile Val Asn Asp Phe Val Ile Glu Ala Asn Lys Ser Asn Thr
       610                 615                 620
   Met Asp Lys Ile Ala Asp Ile Ser Leu Ile Val Pro Tyr Ile Gly Leu
   625                 630                 635                 640
   Ala Leu Asn Val Gly Asn Glu Thr Ala Lys Gly Asn Phe Glu Asn Ala
                   645                 650                 655
   Phe Glu Ile Ala Gly Ala Ser Ile Leu Leu Glu Phe Ile Pro Glu Leu
               660                 665                 670
   Leu Ile Pro Val Val Gly Ala Phe Leu Leu Glu Ser Tyr Ile Asp Asn
           675                 680                 685
   Lys Asn Lys Ile Ile Lys Thr Ile Asp Asn Ala Leu Thr Lys Arg Asn
       690                 695                 700
   Glu Lys Trp Ser Asp Met Tyr Gly Leu Ile Val Ala Gln Trp Leu Ser
   705                 710                 715                 720
   Thr Val Asn Thr Gln Phe Tyr Thr Ile Lys Glu Gly Met Tyr Lys Ala
                   725                 730                 735
   Leu Asn Tyr Gln Ala Gln Ala Leu Glu Glu Ile Ile Lys Tyr Arg Tyr
               740                 745                 750
   Asn Ile Tyr Ser Glu Lys Glu Lys Ser Asn Ile Asn Ile Asp Phe Asn
           755                 760                 765
   Asp Ile Asn Ser Lys Leu Asn Glu Gly Ile Asn Gln Ala Ile Asp Asn
       770                 775                 780
   Ile Asn Asn Phe Ile Asn Gly Cys Ser Val Ser Tyr Leu Met Lys Lys
   785                 790                 795                 800
   Met Ile Pro Leu Ala Val Glu Lys Leu Leu Asp Phe Asp Asn Thr Leu
                   805                 810                 815
   Lys Lys Asn Leu Leu Asn Tyr Ile Asp Glu Asn Lys Leu Tyr Leu Ile
               820                 825                 830
   Gly Ser Ala Glu Tyr Glu Lys Ser Lys Val Asn Lys Tyr Leu Lys Thr
           835                 840                 845
   Ile Met Pro Phe Asp Leu Ser Ile Tyr Thr Asn Asp Thr Ile Leu Ile
       850                 855                 860
   Glu Met Phe Asn Lys Tyr Asn Ser Glu Ile Leu Glu His His His His
   865                 870                 875                 880
   His His
<210> 11
   <211> 3885
   <212> DNA
   <213> Clostridium botulinum
<400> 11
   atggcaataa caattaacaa ctttaattat tcagatcctg ttgataataa aaatatttta     60
   tatttagata ctcatttaaa tacattagct aatgagcctg aaaaagcctt tcgcattata    120
   gggaatatat gggtaatacc cgatagattt tcaagagatt ctaatccaaa tttaaataaa    180
   cctccacgag ttacaagccc taaaagtggt tattatgatc ctaattattt gagtactgat    240
   tctgaaaaag atacattttt aaaagaaatt ataaagttat ttaaaagaat taactctaga    300
   gaaataggag aagaattaat atatagactt gcaacagaca taccctttcc tgggaataac    360
   aatactccaa ttaatacttt tgattttgat gtagatttta acagtgttga tgttaaaact    420
   agacaaggta acaactgggt taaaactggt agtataaatc ctagtgttat aataactgga    480
   cctagagaaa acattataga cccagaaact tctacgttta aattaactaa caatactttt    540
   gcggcacaag aaggatttgg tgctttatca ataatttcaa tatcacctag atttatgcta    600
   acatatagta atgcaactaa taatgtagga gagggtagat tttctaagtc tgaattttgc     660
   atggatccaa tactaatttt aatgcatgaa cttaatcatg caatgcataa tttatatgga    720
   atagctatac caaatgatca aagaatttca tctgtaacta gtaatatttt ttattctcaa    780
   tataatgtga aattagagta tgcagaaata tatgcatttg gaggtccaac tatagacctt    840
   attcctaaaa gtgcaaggaa atattttgag gaaaaggcat tggattatta tagatccata    900
   gctaaaagac ttaatagtat aactactgca aatccttcaa gctttaataa atatatagga   960
   gaatataaac agaaacttat tagaaagtat agattcgtag tagaatcttc aggtgaagtt  1020
   gcagtagatc gtaataagtt tgctgagtta tataaagaac ttacacaaat atttacagaa  1080
   tttaactacg ctaaaatata taatgtacaa aataggaaaa tatatctttc aaatgtatat  1140
   actccggtta cggcaaatat attagacgat aatgtttatg atatacaaaa tggatttaac  1200
   atacctaaaa gtaatttaaa tgtactattt atgggtcaaa atttatctcg aaatccagca  1260
   ttaagaaaag tcaatcctga aaatatgctt tatttattta caaaattttg tcatagaggg   1320
   ataataactt ctaaaactaa atcattagtt ccgcgtggat ccaaggcctt agattgtaga  1380
   gagcttttag ttaaaaatac tgacttaccc tttataggtg atattagtga tatcaaaact  1440
   gatatatttt taagcaaaga tattaatgaa gaaactgaag ttatagacta tccggacaat  1500
   gtttcagtgg atcaagttat tctcagtaag aatacctcag aacatggaca actagattta  1560
   ttatacccta ttattgaagg tgagagtcaa gtattaccgg gagagaatca agtcttttat   1620
   gataatagaa ctcaaaatgt tgattatttg aattcttatt attacctaga atctcaaaaa  1680
   ctaagtgata atgttgaaga ttttactttt acgacatcaa ttgaggaagc tttggataat  1740
   agtggaaaag tatatactta ctttcctaaa ctagctgata aagtaaatac gggtgttcaa  1800
   ggtggtttat ttttaatgtg ggcaaatgat gtagttgaag attttactac aaatattcta  1860
   agaaaagata cattagataa aatatcagat gtatcagcta ttattcccta tataggacct  1920
   gcattaaata taagtaattc tgtaagaagg ggaaatttta ctgaagcatt tgcagttacc  1980
   ggtgtaacta ttttattaga agcgtttcaa gaatttacaa tacctgcact tggtgcattt  2040
   gtgatttata gtaaggttca agaaagaaac gagattatta aaactataga taattgttta  2100
   gaacaaagga ttaaaagatg gaaagattca tatgaatgga tgataggaac gtggttatcc  2160
   aggattacta ctcaatttaa taatataagt tatcaaatgt atgattcttt aaattatcag  2220
   gcagatgcaa tcaaagataa aatagattta gaatataaaa aatactcagg aagtgataaa  2280
   gaaaatataa aaagtcaagt tgaaaattta aaaaatagtt tagatataaa aatctcggaa  2340
   gcaatgaata atataaataa atttatacga gaatgttctg taacatactt atttaaaaat  2400
   atgctcccta aagtaattga tgaattaaat aagtttgatt taaaaactaa aacagaatta  2460
   attaatctta tagatagtca taatattatt ctagttggtg aagtagatag attaaaagca  2520
   aaaataaatg agagttttga aaatacaata ccctttaata ttttttcata tactaataat  2580
   tctttattaa aagatatact cgaggaatat ttcaatagta ttaatgattc aaaaattttg  2640
   agcttacaaa acaaaaaaaa tgctttagtg gatacatcag gatataatgc agaagtgagg  2700
   ctagaaggtg atgttcaagt taatacgata tatacaaatg attttaaatt aagtagttca  2760
   ggagataaaa ttatagtaaa tttaaataat aatattttat atagcgctat ttatgagaac  2820
   tctagtgtta gtttttggat taagatatct aaagatttaa ctaattctca taatgaatat  2880
   actataatta atagtataaa acaaaattct gggtggaaat tatgtattag aaatggcaat  2940
   atagaatgga ttttacaaga tattaataga aagtataaaa gtttaatttt tgattatagt  3000
   gaatcattaa gtcatacagg atatacaaat aaatggtttt ttgttactat aactaataat  3060
   ataatggggt atatgaaact ttatataaat ggagaattaa agcagagtga aagaattgaa  3120
   gatttagatg aggttaagtt agataaaacc atagtatttg gaatagatga gaatatagat  3180
   gagaatcaga tgctttggat tagagatttt aatatttttt ctaaagaatt aagcaatgaa  3240
   gatattaata ttgtatatga gggacaaata ttaagaaatg ttattaaaga ttattgggga  3300
   aatcctttga agtttgatac agaatattat atgattaatt ataattatat agataggtat  3360
   atagcaccta aaaataatat acttgtactt gttcagtatt cagatatatc taaattatat  3420
   actaaaaatc ctattactat taaatcagca gctaataaga atccttatag tagaatttta  3480
   aatggagatg atataatgtt tcatatgtta tatgatagta gggaatatat gataataaga  3540
   gatactgata caatatatgc aacacaagga ggacagtgtt caaaaaattg tgtatatgca  3600
   ttaaaattac agagtaattt aggtaattat ggtataggta tatttagtat aaaaaatatt  3660
   gtatctcaaa ataaatattg tagtcaaatt ttctctagtt ttatgaaaaa tacaatgctt  3720
   ctagcagata tatataaacc ttggagattt tcttttgaaa atgcatacac gccagttgca  3780
   gtaactaatt atgagacaaa actattatca acttcatctt tttggaaatt tatttctagg  3840
   gatccaggat gggtagagct cgagcaccac caccaccacc actaa                   3885
<210> 12
   <211> 1294
   <212> PRT
   <213> Clostridium botulinum
<400> 12
   Met Ala Ile Thr Ile Asn Asn Phe Asn Tyr Ser Asp Pro Val Asp Asn
   1               5                   10                  15
   Lys Asn Ile Leu Tyr Leu Asp Thr His Leu Asn Thr Leu Ala Asn Glu
   20 25 30
   Pro Glu Lys Ala Phe Arg Ile Ile Gly Asn Ile Trp Val Ile Pro Asp
   35 40 45
   Arg Phe Ser Arg Asp Ser Asn Pro Asn Leu Asn Lys Pro Pro Arg Val
   50 55 60
   Thr Ser Pro Lys Ser Gly Tyr Tyr Asp Pro Asn Tyr Leu Ser Thr Asp
   65 70 75 80
   Ser Glu Lys Asp Thr Phe Leu Lys Glu Ile Ile Lys Leu Phe Lys Arg
   85 90 95
   Ile Asn Ser Arg Glu Ile Gly Glu Glu Leu Ile Tyr Arg Leu Ala Thr
   100 105 110
   Asp Ile Pro Phe Pro Gly Asn Asn Asn Thr Pro Ile Asn Thr Phe Asp
   115 120 125
   Phe Asp Val Asp Phe Asn Ser Val Asp Val Lys Thr Arg Gln Gly Asn
   130 135 140
   Asn Trp Val Lys Thr Gly Ser Ile Asn Pro Ser Val Ile Ile Thr Gly
   145 150 155 160
   Pro Arg Glu Asn Ile Ile Asp Pro Glu Thr Ser Thr Phe Lys Leu Thr
   165 170 175
   Asn Asn Thr Phe Ala Ala Gln Glu Gly Phe Gly Ala Leu Ser Ile Ile
   180 185 190
   Ser Ile Ser Pro Arg Phe Met Leu Thr Tyr Ser Asn Ala Thr Asn Asn
   195 200 205
   Val Gly Glu Gly Arg Phe Ser Lys Ser Glu Phe Cys Met Asp Pro Ile
   210 215 220
   Leu Ile Leu Met His Glu Leu Asn His Ala Met His Asn Leu Tyr Gly
   225 230 235 240
   Ile Ala Ile Pro Asn Asp Gln Arg Ile Ser Ser Val Thr Ser Asn Ile
   245 250 255
   Phe Tyr Ser Gln Tyr Asn Val Lys Leu Glu Tyr Ala Glu Ile Tyr Ala
   260 265 270
   Phe Gly Gly Pro Thr Ile Asp Leu Ile Pro Lys Ser Ala Arg Lys Tyr
   275 280 285
   Phe Glu Glu Lys Ala Leu Asp Tyr Tyr Arg Ser Ile Ala Lys Arg Leu
   290 295 300
   Asn Ser Ile Thr Thr Ala Asn Pro Ser Ser Phe Asn Lys Tyr Ile Gly
   305 310 315 320
   Glu Tyr Lys Gln Lys Leu Ile Arg Lys Tyr Arg Phe Val Val Glu Ser
   325 330 335
   Ser Gly Glu Val Ala Val Asp Arg Asn Lys Phe Ala Glu Leu Tyr Lys
   340 345 350
   Glu Leu Thr Gln Ile Phe Thr Glu Phe Asn Tyr Ala Lys Ile Tyr Asn
   355 360 365
   Val Gln Asn Arg Lys Ile Tyr Leu Ser Asn Val Tyr Thr Pro Val Thr
   370 375 380
   Ala Asn Ile Leu Asp Asp Asn Val Tyr Asp Ile Gln Asn Gly Phe Asn
   385 390 395 400
   Ile Pro Lys Ser Asn Leu Asn Val Leu Phe Met Gly Gln Asn Leu Ser
   405 410 415
   Arg Asn Pro Ala Leu Arg Lys Val Asn Pro Glu Asn Met Leu Tyr Leu
   420 425 430
   Phe Thr Lys Phe Cys His Arg Gly Ile Ile Thr Ser Lys Thr Lys Ser
   435 440 445
   Leu Val Pro Arg Gly Ser Lys Ala Leu Asp Cys Arg Glu Leu Leu Val
   450 455 460
   Lys Asn Thr Asp Leu Pro Phe Ile Gly Asp Ile Ser Asp Ile Lys Thr
   465 470 475 480
   Asp Ile Phe Leu Ser Lys Asp Ile Asn Glu Glu Thr Glu Val Ile Asp
   485 490 495
   Tyr Pro Asp Asn Val Ser Val Asp Gln Val Ile Leu Ser Lys Asn Thr
   500 505 510
   Ser Glu His Gly Gln Leu Asp Leu Leu Tyr Pro Ile Ile Glu Gly Glu
   515 520 525
   Ser Gln Val Leu Pro Gly Glu Asn Gln Val Phe Tyr Asp Asn Arg Thr
   530 535 540
   Gln Asn Val Asp Tyr Leu Asn Ser Tyr Tyr Tyr Leu Glu Ser Gln Lys
   545 550 555 560
   Leu Ser Asp Asn Val Glu Asp Phe Thr Phe Thr Thr Ser Ile Glu Glu
   565 570 575
   Ala Leu Asp Asn Ser Gly Lys Val Tyr Thr Tyr Phe Pro Lys Leu Ala
   580 585 590
   Asp Lys Val Asn Thr Gly Val Gln Gly Gly Leu Phe Leu Met Trp Ala
   595 600 605
   Asn Asp Val Val Glu Asp Phe Thr Thr Asn Ile Leu Arg Lys Asp Thr
   610 615 620
   Leu Asp Lys Ile Ser Asp Val Ser Ala Ile Ile Pro Tyr Ile Gly Pro
   625 630 635 640
   Ala Leu Asn Ile Ser Asn Ser Val Arg Arg Gly Asn Phe Thr Glu Ala
   645 650 655
   Phe Ala Val Thr Gly Val Thr Ile Leu Leu Glu Ala Phe Gln Glu Phe
   660 665 670
   Thr Ile Pro Ala Leu Gly Ala Phe Val Ile Tyr Ser Lys Val Gln Glu
   675 680 685
   Arg Asn Glu Ile Ile Lys Thr Ile Asp Asn Cys Leu Glu Gln Arg Ile
   690 695 700
   Lys Arg Trp Lys Asp Ser Tyr Glu Trp Met Ile Gly Thr Trp Leu Ser
   705 710 715 720
   Arg Ile Thr Thr Gln Phe Asn Asn Ile Ser Tyr Gln Met Tyr Asp Ser
   725 730 735
   Leu Asn Tyr Gln Ala Asp Ala Ile Lys Asp Lys Ile Asp Leu Glu Tyr
   740 745 750
   Lys Lys Tyr Ser Gly Ser Asp Lys Glu Asn Ile Lys Ser Gln Val Glu
   755 760 765
   Asn Leu Lys Asn Ser Leu Asp Ile Lys Ile Ser Glu Ala Met Asn Asn
   770 775 780
   Ile Asn Lys Phe Ile Arg Glu Cys Ser Val Thr Tyr Leu Phe Lys Asn
   785 790 795 800
   Met Leu Pro Lys Val Ile Asp Glu Leu Asn Lys Phe Asp Leu Lys Thr
   805 810 815
   Lys Thr Glu Leu Ile Asn Leu Ile Asp Ser His Asn Ile Ile Leu Val
   820 825 830
   Gly Glu Val Asp Arg Leu Lys Ala Lys Ile Asn Glu Ser Phe Glu Asn
   835 840 845
   Thr Ile Pro Phe Asn Ile Phe Ser Tyr Thr Asn Asn Ser Leu Leu Lys
   850 855 860
   Asp Ile Leu Glu Glu Tyr Phe Asn Ser Ile Asn Asp Ser Lys Ile Leu
   865 870 875 880
   Ser Leu Gln Asn Lys Lys Asn Ala Leu Val Asp Thr Ser Gly Tyr Asn
   885 890 895
   Ala Glu Val Arg Leu Glu Gly Asp Val Gln Val Asn Thr Ile Tyr Thr
   900 905 910
   Asn Asp Phe Lys Leu Ser Ser Ser Gly Asp Lys Ile Ile Val Asn Leu
   915 920 925
   Asn Asn Asn Ile Leu Tyr Ser Ala Ile Tyr Glu Asn Ser Ser Val Ser
   930 935 940
   Phe Trp Ile Lys Ile Ser Lys Asp Leu Thr Asn Ser His Asn Glu Tyr
   945 950 955 960
   Thr Ile Ile Asn Ser Ile Lys Gln Asn Ser Gly Trp Lys Leu Cys Ile
   965 970 975
   Arg Asn Gly Asn Ile Glu Trp Ile Leu Gln Asp Ile Asn Arg Lys Tyr
   980 985 990
   Lys Ser Leu Ile Phe Asp Tyr Ser Glu Ser Leu Ser His Thr Gly Tyr
   995 1000 1005
   Thr Asn Lys Trp Phe Phe Val Thr Ile Thr Asn Asn Ile Met Gly
   1010 1015 1020
   Tyr Met Lys Leu Tyr Ile Asn Gly Glu Leu Lys Gln Ser Glu Arg
   1025 1030 1035
   Ile Glu Asp Leu Asp Glu Val Lys Leu Asp Lys Thr Ile Val Phe
   1040 1045 1050
   Gly Ile Asp Glu Asn Ile Asp Glu Asn Gln Met Leu Trp Ile Arg
   1055 1060 1065
   Asp Phe Asn Ile Phe Ser Lys Glu Leu Ser Asn Glu Asp Ile Asn
   1070 1075 1080
   Ile Val Tyr Glu Gly Gln Ile Leu Arg Asn Val Ile Lys Asp Tyr
   1085 1090 1095
   Trp Gly Asn Pro Leu Lys Phe Asp Thr Glu Tyr Tyr Met Ile Asn
   1100 1105 1110
   Tyr Asn Tyr Ile Asp Arg Tyr Ile Ala Pro Lys Asn Asn Ile Leu
   1115 1120 1125
   Val Leu Val Gln Tyr Ser Asp Ile Ser Lys Leu Tyr Thr Lys Asn
   1130 1135 1140
   Pro Ile Thr Ile Lys Ser Ala Ala Asn Lys Asn Pro Tyr Ser Arg
   1145 1150 1155
   Ile Leu Asn Gly Asp Asp Ile Met Phe His Met Leu Tyr Asp Ser
   1160 1165 1170
   Arg Glu Tyr Met Ile Ile Arg Asp Thr Asp Thr Ile Tyr Ala Thr
   1175 1180 1185
   Gln Gly Gly Gln Cys Ser Lys Asn Cys Val Tyr Ala Leu Lys Leu
   1190 1195 1200
   Gln Ser Asn Leu Gly Asn Tyr Gly Ile Gly Ile Phe Ser Ile Lys
   1205 1210 1215
   Asn Ile Val Ser Gln Asn Lys Tyr Cys Ser Gln Ile Phe Ser Ser
   1220 1225 1230
   Phe Met Lys Asn Thr Met Leu Leu Ala Asp Ile Tyr Lys Pro Trp
   1235 1240 1245
   Arg Phe Ser Phe Glu Asn Ala Tyr Thr Pro Val Ala Val Thr Asn
   1250 1255 1260
   Tyr Glu Thr Lys Leu Leu Ser Thr Ser Ser Phe Trp Lys Phe Ile
   1265 1270 1275
   Ser Arg Asp Pro Gly Trp Val Glu Leu Glu His His His His His
   1280 1285 1290
   His
<210> 13
   <211> 2625
   <212> DNA
   <213> Clostridium botulinum
<400> 13
<210> 14
   <211> 874
   <212> PRT
   <213> Clostridium botulinum
<400> 14
   Met Ala Ile Thr Ile Asn Asn Phe Asn Tyr Ser Asp Pro Val Asp Asn
   1               5                   10                  15
   Lys Asn Ile Leu Tyr Leu Asp Thr His Leu Asn Thr Leu Ala Asn Glu
               20                  25                  30
   Pro Glu Lys Ala Phe Arg Ile Ile Gly Asn Ile Trp Val Ile Pro Asp
           35                  40                  45
   Arg Phe Ser Arg Asp Ser Asn Pro Asn Leu Asn Lys Pro Pro Arg Val
       50                  55                  60
   Thr Ser Pro Lys Ser Gly Tyr Tyr Asp Pro Asn Tyr Leu Ser Thr Asp
   65                  70                  75                  80
   Ser Glu Lys Asp Thr Phe Leu Lys Glu Ile Ile Lys Leu Phe Lys Arg
                   85                  90                  95
   Ile Asn Ser Arg Glu Ile Gly Glu Glu Leu Ile Tyr Arg Leu Ala Thr
               100                 105                 110
   Asp Ile Pro Phe Pro Gly Asn Asn Asn Thr Pro Ile Asn Thr Phe Asp
           115                 120                 125
   Phe Asp Val Asp Phe Asn Ser Val Asp Val Lys Thr Arg Gln Gly Asn
       130                 135                 140
   Asn Trp Val Lys Thr Gly Ser Ile Asn Pro Ser Val Ile Ile Thr Gly
   145                 150                 155                 160
   Pro Arg Glu Asn Ile Ile Asp Pro Glu Thr Ser Thr Phe Lys Leu Thr
                   165                 170                 175
   Asn Asn Thr Phe Ala Ala Gln Glu Gly Phe Gly Ala Leu Ser Ile Ile
               180                 185                 190
   Ser Ile Ser Pro Arg Phe Met Leu Thr Tyr Ser Asn Ala Thr Asn Asn
           195                 200                 205
   Val Gly Glu Gly Arg Phe Ser Lys Ser Glu Phe Cys Met Asp Pro Ile
       210                 215                 220
   Leu Ile Leu Met His Glu Leu Asn His Ala Met His Asn Leu Tyr Gly
   225                 230                 235                 240
   Ile Ala Ile Pro Asn Asp Gln Arg Ile Ser Ser Val Thr Ser Asn Ile
                   245                 250                 255
   Phe Tyr Ser Gln Tyr Asn Val Lys Leu Glu Tyr Ala Glu Ile Tyr Ala
               260                 265                 270
   Phe Gly Gly Pro Thr Ile Asp Leu Ile Pro Lys Ser Ala Arg Lys Tyr
           275                 280                 285
   Phe Glu Glu Lys Ala Leu Asp Tyr Tyr Arg Ser Ile Ala Lys Arg Leu
       290                 295                 300
   Asn Ser Ile Thr Thr Ala Asn Pro Ser Ser Phe Asn Lys Tyr Ile Gly
   305                 310                 315                 320
   Glu Tyr Lys Gln Lys Leu Ile Arg Lys Tyr Arg Phe Val Val Glu Ser
                   325                 330                 335
   Ser Gly Glu Val Ala Val Asp Arg Asn Lys Phe Ala Glu Leu Tyr Lys
               340                 345                 350
   Glu Leu Thr Gln Ile Phe Thr Glu Phe Asn Tyr Ala Lys Ile Tyr Asn
           355                 360                 365
   Val Gln Asn Arg Lys Ile Tyr Leu Ser Asn Val Tyr Thr Pro Val Thr
       370                 375                 380
   Ala Asn Ile Leu Asp Asp Asn Val Tyr Asp Ile Gln Asn Gly Phe Asn
   385 390 395 400
   Ile Pro Lys Ser Asn Leu Asn Val Leu Phe Met Gly Gln Asn Leu Ser
   405 410 415
   Arg Asn Pro Ala Leu Arg Lys Val Asn Pro Glu Asn Met Leu Tyr Leu
   420 425 430
   Phe Thr Lys Phe Cys His Arg Gly Ile Ile Thr Ser Lys Thr Lys Ser
   435 440 445
   Leu Val Pro Arg Gly Ser Lys Ala Leu Asp Cys Arg Glu Leu Leu Val
   450 455 460
   Lys Asn Thr Asp Leu Pro Phe Ile Gly Asp Ile Ser Asp Ile Lys Thr
   465 470 475 480
   Asp Ile Phe Leu Ser Lys Asp Ile Asn Glu Glu Thr Glu Val Ile Asp
   485 490 495
   Tyr Pro Asp Asn Val Ser Val Asp Gln Val Ile Leu Ser Lys Asn Thr
   500 505 510
   Ser Glu His Gly Gln Leu Asp Leu Leu Tyr Pro Ile Ile Glu Gly Glu
   515 520 525
   Ser Gln Val Leu Pro Gly Glu Asn Gln Val Phe Tyr Asp Asn Arg Thr
   530 535 540
   Gln Asn Val Asp Tyr Leu Asn Ser Tyr Tyr Tyr Leu Glu Ser Gln Lys
   545 550 555 560
   Leu Ser Asp Asn Val Glu Asp Phe Thr Phe Thr Thr Ser Ile Glu Glu
   565 570 575
   Ala Leu Asp Asn Ser Gly Lys Val Tyr Thr Tyr Phe Pro Lys Leu Ala
   580 585 590
   Asp Lys Val Asn Thr Gly Val Gln Gly Gly Leu Phe Leu Met Trp Ala
   595 600 605
   Asn Asp Val Val Glu Asp Phe Thr Thr Asn Ile Leu Arg Lys Asp Thr
   610 615 620
   Leu Asp Lys Ile Ser Asp Val Ser Ala Ile Ile Pro Tyr Ile Gly Pro
   625 630 635 640
   Ala Leu Asn Ile Ser Asn Ser Val Arg Arg Gly Asn Phe Thr Glu Ala
   645 650 655
   Phe Ala Val Thr Gly Val Thr Ile Leu Leu Glu Ala Phe Gln Glu Phe
   660 665 670
   Thr Ile Pro Ala Leu Gly Ala Phe Val Ile Tyr Ser Lys Val Gln Glu
   675 680 685
   Arg Asn Glu Ile Ile Lys Thr Ile Asp Asn Cys Leu Glu Gln Arg Ile
   690 695 700
   Lys Arg Trp Lys Asp Ser Tyr Glu Trp Met Ile Gly Thr Trp Leu Ser
   705 710 715 720
   Arg Ile Thr Thr Gln Phe Asn Asn Ile Ser Tyr Gln Met Tyr Asp Ser
                   725                 730                 735
   Leu Asn Tyr Gln Ala Asp Ala Ile Lys Asp Lys Ile Asp Leu Glu Tyr
               740                 745                 750
   Lys Lys Tyr Ser Gly Ser Asp Lys Glu Asn Ile Lys Ser Gln Val Glu
           755                 760                 765
   Asn Leu Lys Asn Ser Leu Asp Ile Lys Ile Ser Glu Ala Met Asn Asn
       770                 775                 780
   Ile Asn Lys Phe Ile Arg Glu Cys Ser Val Thr Tyr Leu Phe Lys Asn
   785                 790                 795                 800
   Met Leu Pro Lys Val Ile Asp Glu Leu Asn Lys Phe Asp Leu Lys Thr
                   805                 810                 815
   Lys Thr Glu Leu Ile Asn Leu Ile Asp Ser His Asn Ile Ile Leu Val
               820                 825                 830
   Gly Glu Val Asp Arg Leu Lys Ala Lys Ile Asn Glu Ser Phe Glu Asn
           835                 840                 845
   Thr Ile Pro Phe Asn Ile Phe Ser Tyr Thr Asn Asn Ser Leu Leu Lys
       850                 855                 860
   Asp Ile Leu Glu His His His His His His
   865                 870
<210> 15
   <211> 3810
   <212> DNA
   <213> Clostridium botulinum
<400> 15
   atggcaacaa ttaatagttt taattataat gatcctgtta ataatagaac aattttatat 60
   attaaaccag gcggttgtca acaattttat aaatcattta atattatgaa aaatatttgg 120
   ataattccag agagaaatgt aattggtaca attccccaag attttcttcc gcctacttca 180
   ttgaaaaatg gagatagtag ttattatgac cctaattatt tacaaagtga tcaagaaaag 240
   gataaatttt taaaaatagt cacaaaaata tttaatagaa taaatgataa tctttcagga 300
   aggattttat tagaagaact gtcaaaagct aatccatatt taggaaatga taatactcca 360
   gatggtgact tcattattaa tgatgcatca gcagttccaa ttcaattctc aaatggtagc 420
   caaagcatac tattacctaa tgttattata atgggagcag agcctgattt atttgaaact 480
   aacagttcca atatttctct aagaaataat tatatgccaa gcaatcacgg ttttggatca 540
   atagctatag taacattctc acctgaatat tcttttagat ttaaagataa tagtatgaat 600
   gaatttattc aagatcctgc tcttacatta atgcatgaat taatacattc attacatgga 660
   ctatatgggg ctaaagggat tactacaaag tatactataa cacaaaaaca aaatccccta 720
   ataacaaata taagaggtac aaatattgaa gaattcttaa cttttggagg tactgattta 780
   aacattatta ctagtgctca gtccaatgat atctatacta atcttctagc tgattataaa 840
   aaaatagcgt ctaaacttag caaagtacaa gtatctaatc cactacttaa tccttataaa 900
   gatgtttttg aagcaaagta tggattagat aaagatgcta gcggaattta ttcggtaaat 960
   ataaacaaat ttaatgatat ttttaaaaaa ttatacagct ttacggaatt tgatttagca 1020
   actaaatttc aagttaaatg taggcaaact tatattggac agtataaata cttcaaactt 1080
   tcaaacttgt taaatgattc tatttataat atatcagaag gctataatat aaataattta 1140
   aaggtaaatt ttagaggaca gaatgcaaat ttaaatccta gaattattac accaattaca 1200
   ggtagaggac tagtaaaaaa aatcattaga ttttgtaaaa gtgggataat aacttctaaa 1260
   actaaatcat tggttccacg tggatccaag gcctcaatat gtatcgaaat aaataatggt 1320
   gagttatttt ttgtggcttc cgagaatagt tataatgatg ataatataaa tactcctaaa 1380
   gaaattgacg atacagtaac ttcaaataat aattatgaaa atgatttaga tcaggttatt 1440
   ttaaatttta atagtgaatc agcacctgga ctttcagatg aaaaattaaa tttaactatc 1500
   caaaatgatg cttatatacc aaaatatgat tctaatggaa caagtgatat agaacaacat   1560
   gatgttaatg aacttaatgt atttttctat ttagatgcac agaaagtgcc cgaaggtgaa   1620
   aataatgtca atctcacctc ttcaattgat acagcattat tagaacaacc taaaatatat   1680
   acattttttt catcagaatt tattaataat gtcaataaac ctgtgcaagc agcattattt   1740
   gtaggctgga tacaacaagt attagtagat tttactactg aagctaacca aaaaagtact   1800
   gttgataaaa ttgcagatat ttctatagtt gttccatata taggtcttgc tttaaatata   1860
   ggaaatgaag cacaaaaagg aaattttaaa gatgcacttg aattattagg agcaggtatt   1920
   ttattagaat ttgaacccga gcttttaatt cctacaattt tagtattcac gataaaatct   1980
   tttttaggtt catctgataa taaaaataaa gttattaaag caataaataa tgcattgaaa   2040
   gaaagagatg aaaaatggaa agaagtatat agttttatag tatcgaattg gatgactaaa   2100
   attaatacac aatttaataa aagaaaagaa caaatgtatc aagctttaca aaatcaagta   2160
   aatgcactta aagcaataat agaatctaag tataatagtt atactttaga agaaaaaaat   2220
   gagcttacaa ataaatatga tattgagcaa atagaaaatg aacttaatca aaaggtttct   2280
   atagcaatga ataatataga caggttctta actgaaagtt ctatatctta tttaatgaaa   2340
   ttaataaatg aagtaaaaat taataaatta agagaatatg atgaaaatgt taaaacgtat   2400
   ttattagatt atattataaa acatggatca atcttgggag agagtcagca agaactaaat   2460
   tctatggtaa ttgataccct aaataatagt attcctttta agctttcttc ttatacagat   2520
   gataaaattt taatttcata ttttaataag ttctttaaga gaattaaaag tagttctgtt   2580
   ttaaatatga gatataaaaa tgataaatac gtagatactt caggatatga ttcaaatata   2640
   aatattaatg gagatgtata taaatatcca actaataaaa atcaatttgg aatatataat   2700
   gataaactta gtgaagttaa tatatctcaa aatgattaca ttatatatga taataaatat   2760
   aaaaatttta gtattagttt ttgggtaaga attcctaact atgataataa gatagtaaat   2820
   gttaataatg aatacactat aataaattgt atgagggata ataattcagg atggaaagta   2880
   tctcttaatc ataatgaaat aatttggaca ttgcaagata attcaggaat taatcaaaaa   2940
   ttagcattta actatggtaa cgcaaatggt atttctgatt atataaataa gtggattttt   3000
   gtaactataa ctaatgatag attaggagat tctaaacttt atattaatgg aaatttaata   3060
   gataaaaaat caattttaaa tttaggtaat attcatgtta gtgacaatat attatttaaa   3120
   atagttaatt gtagttatac aagatatatt ggtattagat attttaatat ttttgataaa    3180
   gaattagatg aaacagaaat tcaaacttta tataacaatg agcctaatgc aaatatttta   3240
   aaggattttt ggggaaatta tttgctttat gacaaagaat attatttatt aaatgtgtta   3300
   aaaccaaata actttattaa taggagaaca gattctactt taagcattaa taatataaga   3360
   agcactattc ttttagctaa tagattatat agtggaataa aagttaaaat acaaagagtt   3420
   aataatagta gtactaacga taatcttgtt agaaagaatg atcaggtata tattaatttt   3480
   gtagccagca aaactcactt acttccatta tatgctgata cagctaccac aaataaagag   3540
   aaaacaataa aaatatcatc atctggcaat agatttaatc aagtagtagt tatgaattca   3600
   gtaggaaata attgtacaat gaattttaaa aataataatg gaaataatat tgggttgtta   3660
   ggtttcaagg cagatactgt agttgctagt acttggtatt atacacatat gagagataat   3720
   acaaacagca atggattttt ttggaacttt atttctgaag aacatggatg gcaagaaaaa   3780
   acgcgttctc accaccacca ccaccactaa                                    3810
<210> 16
   <211> 1269
   <212> PRT
   <213> Clostridium botulinum
<400> 16
   Met Ala Thr Ile Asn Ser Phe Asn Tyr Asn Asp Pro Val Asn Asn Arg
   1               5                   10                  15
   Thr Ile Leu Tyr Ile Lys Pro Gly Gly Cys Gln Gln Phe Tyr Lys Ser
               20                  25                  30
   Phe Asn Ile Met Lys Asn Ile Trp Ile Ile Pro Glu Arg Asn Val Ile
           35                  40                  45
   Gly Thr Ile Pro Gln Asp Phe Leu Pro Pro Thr Ser Leu Lys Asn Gly
       50                  55                  60
   Asp Ser Ser Tyr Tyr Asp Pro Asn Tyr Leu Gln Ser Asp Gln Glu Lys
   65                  70                  75                  80
   Asp Lys Phe Leu Lys Ile Val Thr Lys Ile Phe Asn Arg Ile Asn Asp
                   85                  90                  95
   Asn Leu Ser Gly Arg Ile Leu Leu Glu Glu Leu Ser Lys Ala Asn Pro
               100                 105                 110
   Tyr Leu Gly Asn Asp Asn Thr Pro Asp Gly Asp Phe Ile Ile Asn Asp
           115                 120                 125
   Ala Ser Ala Val Pro Ile Gln Phe Ser Asn Gly Ser Gln Ser Ile Leu
       130                 135                 140
   Leu Pro Asn Val Ile Ile Met Gly Ala Glu Pro Asp Leu Phe Glu Thr
   145                 150                 155                 160
   Asn Ser Ser Asn Ile Ser Leu Arg Asn Asn Tyr Met Pro Ser Asn His
                   165                 170                 175
   Gly Phe Gly Ser Ile Ala Ile Val Thr Phe Ser Pro Glu Tyr Ser Phe
               180                 185                 190
   Arg Phe Lys Asp Asn Ser Met Asn Glu Phe Ile Gln Asp Pro Ala Leu
           195                 200                 205
   Thr Leu Met His Glu Leu Ile His Ser Leu His Gly Leu Tyr Gly Ala
       210                 215                 220
   Lys Gly Ile Thr Thr Lys Tyr Thr Ile Thr Gln Lys Gln Asn Pro Leu
   225                 230                 235                 240
   Ile Thr Asn Ile Arg Gly Thr Asn Ile Glu Glu Phe Leu Thr Phe Gly
                   245                 250                 255
   Gly Thr Asp Leu Asn Ile Ile Thr Ser Ala Gln Ser Asn Asp Ile Tyr
               260                 265                 270
   Thr Asn Leu Leu Ala Asp Tyr Lys Lys Ile Ala Ser Lys Leu Ser Lys
           275                 280                 285
   Val Gln Val Ser Asn Pro Leu Leu Asn Pro Tyr Lys Asp Val Phe Glu
       290                 295                 300
   Ala Lys Tyr Gly Leu Asp Lys Asp Ala Ser Gly Ile Tyr Ser Val Asn
   305                 310                 315                 320
   Ile Asn Lys Phe Asn Asp Ile Phe Lys Lys Leu Tyr Ser Phe Thr Glu
                   325                 330                 335
   Phe Asp Leu Ala Thr Lys Phe Gln Val Lys Cys Arg Gln Thr Tyr Ile
               340                 345                 350
   Gly Gln Tyr Lys Tyr Phe Lys Leu Ser Asn Leu Leu Asn Asp Ser Ile
           355                 360                 365
   Tyr Asn Ile Ser Glu Gly Tyr Asn Ile Asn Asn Leu Lys Val Asn Phe
       370                 375                 380
   Arg Gly Gln Asn Ala Asn Leu Asn Pro Arg Ile Ile Thr Pro Ile Thr
   385                 390                 395                 400
   Gly Arg Gly Leu Val Lys Lys Ile Ile Arg Phe Cys Lys Ser Gly Ile
                   405                 410                 415
   Ile Thr Ser Lys Thr Lys Ser Leu Val Pro Arg Gly Ser Lys Ala Ser
               420                 425                 430
   Ile Cys Ile Glu Ile Asn Asn Gly Glu Leu Phe Phe Val Ala Ser Glu
           435                 440                 445
   Asn Ser Tyr Asn Asp Asp Asn Ile Asn Thr Pro Lys Glu Ile Asp Asp
       450                 455                 460
   Thr Val Thr Ser Asn Asn Asn Tyr Glu Asn Asp Leu Asp Gln Val Ile
   465                 470                 475                 480
   Leu Asn Phe Asn Ser Glu Ser Ala Pro Gly Leu Ser Asp Glu Lys Leu
                   485                 490                 495
   Asn Leu Thr Ile Gln Asn Asp Ala Tyr Ile Pro Lys Tyr Asp Ser Asn
               500                 505                 510
   Gly Thr Ser Asp Ile Glu Gln His Asp Val Asn Glu Leu Asn Val Phe
           515                 520                 525
   Phe Tyr Leu Asp Ala Gln Lys Val Pro Glu Gly Glu Asn Asn Val Asn
       530                 535                 540
   Leu Thr Ser Ser Ile Asp Thr Ala Leu Leu Glu Gln Pro Lys Ile Tyr
   545                 550                 555                 560
   Thr Phe Phe Ser Ser Glu Phe Ile Asn Asn Val Asn Lys Pro Val Gln
                   565                 570                 575
   Ala Ala Leu Phe Val Gly Trp Ile Gln Gln Val Leu Val Asp Phe Thr
               580                 585                 590
   Thr Glu Ala Asn Gln Lys Ser Thr Val Asp Lys Ile Ala Asp Ile Ser
           595                 600                 605
   Ile Val Val Pro Tyr Ile Gly Leu Ala Leu Asn Ile Gly Asn Glu Ala
       610                 615                 620
   Gln Lys Gly Asn Phe Lys Asp Ala Leu Glu Leu Leu Gly Ala Gly Ile
   625                 630                 635                 640
   Leu Leu Glu Phe Glu Pro Glu Leu Leu Ile Pro Thr Ile Leu Val Phe
                   645                 650                 655
   Thr Ile Lys Ser Phe Leu Gly Ser Ser Asp Asn Lys Asn Lys Val Ile
               660                 665                 670
   Lys Ala Ile Asn Asn Ala Leu Lys Glu Arg Asp Glu Lys Trp Lys Glu
           675                 680                 685
   Val Tyr Ser Phe Ile Val Ser Asn Trp Met Thr Lys Ile Asn Thr Gln
       690                 695                 700
   Phe Asn Lys Arg Lys Glu Gln Met Tyr Gln Ala Leu Gln Asn Gln Val
   705                 710                 715                 720
   Asn Ala Leu Lys Ala Ile Ile Glu Ser Lys Tyr Asn Ser Tyr Thr Leu
                   725                 730                 735
   Glu Glu Lys Asn Glu Leu Thr Asn Lys Tyr Asp Ile Glu Gln Ile Glu
               740                 745                 750
   Asn Glu Leu Asn Gln Lys Val Ser Ile Ala Met Asn Asn Ile Asp Arg
   755 760 765
   Phe Leu Thr Glu Ser Ser Ile Ser Tyr Leu Met Lys Leu Ile Asn Glu
   770 775 780
   Val Lys Ile Asn Lys Leu Arg Glu Tyr Asp Glu Asn Val Lys Thr Tyr
   785 790 795 800
   Leu Leu Asp Tyr Ile Ile Lys His Gly Ser Ile Leu Gly Glu Ser Gln
   805 810 815
   Gln Glu Leu Asn Ser Met Val Ile Asp Thr Leu Asn Asn Ser Ile Pro
   820 825 830
   Phe Lys Leu Ser Ser Tyr Thr Asp Asp Lys Ile Leu Ile Ser Tyr Phe
   835 840 845
   Asn Lys Phe Phe Lys Arg Ile Lys Ser Ser Ser Val Leu Asn Met Arg
   850 855 860
   Tyr Lys Asn Asp Lys Tyr Val Asp Thr Ser Gly Tyr Asp Ser Asn Ile
   865 870 875 880
   Asn Ile Asn Gly Asp Val Tyr Lys Tyr Pro Thr Asn Lys Asn Gln Phe
   885 890 895
   Gly Ile Tyr Asn Asp Lys Leu Ser Glu Val Asn Ile Ser Gln Asn Asp
   900 905 910
   Tyr Ile Ile Tyr Asp Asn Lys Tyr Lys Asn Phe Ser Ile Ser Phe Trp
   915 920 925
   Val Arg Ile Pro Asn Tyr Asp Asn Lys Ile Val Asn Val Asn Asn Glu
   930 935 940
   Tyr Thr Ile Ile Asn Cys Met Arg Asp Asn Asn Ser Gly Trp Lys Val
   945 950 955 960
   Ser Leu Asn His Asn Glu Ile Ile Trp Thr Leu Gln Asp Asn Ser Gly
   965 970 975
   Ile Asn Gln Lys Leu Ala Phe Asn Tyr Gly Asn Ala Asn Gly Ile Ser
   980 985 990
   Asp Tyr Ile Asn Lys Trp Ile Phe Val Thr Ile Thr Asn Asp Arg Leu
   995 1000 1005
   Gly Asp Ser Lys Leu Tyr Ile Asn Gly Asn Leu Ile Asp Lys Lys
   1010 1015 1020
   Ser Ile Leu Asn Leu Gly Asn Ile His Val Ser Asp Asn Ile Leu
   1025 1030 1035
   Phe Lys Ile Val Asn Cys Ser Tyr Thr Arg Tyr Ile Gly Ile Arg
   1040 1045 1050
   Tyr Phe Asn Ile Phe Asp Lys Glu Leu Asp Glu Thr Glu Ile Gln
   1055 1060 1065
   Thr Leu Tyr Asn Asn Glu Pro Asn Ala Asn Ile Leu Lys Asp Phe
   1070 1075 1080
   Trp Gly  Asn Tyr Leu Leu Tyr  Asp Lys Glu Tyr Tyr  Leu Leu Asn
       1085                 1090                 1095
   Val Leu  Lys Pro Asn Asn Phe  Ile Asn Arg Arg Thr  Asp Ser Thr
       1100                 1105                 1110
   Leu Ser  Ile Asn Asn Ile Arg  Ser Thr Ile Leu Leu  Ala Asn Arg
       1115                 1120                 1125
   Leu Tyr  Ser Gly Ile Lys Val  Lys Ile Gln Arg Val  Asn Asn Ser
       1130                 1135                 1140
   Ser Thr  Asn Asp Asn Leu Val  Arg Lys Asn Asp Gln  Val Tyr Ile
       1145                 1150                 1155
   Asn Phe  Val Ala Ser Lys Thr  His Leu Leu Pro Leu  Tyr Ala Asp
       1160                 1165                 1170
   Thr Ala  Thr Thr Asn Lys Glu  Lys Thr Ile Lys Ile  Ser Ser Ser
       1175                 1180                 1185
   Gly Asn  Arg Phe Asn Gln Val  Val Val Met Asn Ser  Val Gly Asn
       1190                 1195                 1200
   Asn Cys  Thr Met Asn Phe Lys  Asn Asn Asn Gly Asn  Asn Ile Gly
       1205                 1210                 1215
   Leu Leu  Gly Phe Lys Ala Asp  Thr Val Val Ala Ser  Thr Trp Tyr
       1220                 1225                 1230
   Tyr Thr  His Met Arg Asp Asn  Thr Asn Ser Asn Gly  Phe Phe Trp
       1235                 1240                 1245
   Asn Phe  Ile Ser Glu Glu His  Gly Trp Gln Glu Lys  Thr Arg Ser
       1250                 1255                 1260
   His His  His His His His
       1265
<210> 17
   <211> 1116
   <212> DNA
   <213> Pseudomonas aeruginosa
<400> 17
210> 18
   <211> 371
   <212> PRT
   <213> Pseudomonas aeruginosa
<400> 18
<210> 19
   <211> 1215
   <212> DNA
   <213> Corynebacterium diphtheriae
<400> 19
   atggctgatg atgttgttga ttcttctaaa tcttttgtga tggaaaactt ttcttcgtac     60
   cacgggacta aacctggtta tgtagattcc attcaaaaag gtatacaaaa gccaaaatct    120
   ggtacacaag gaaattatga cgatgattgg aaagggtttt atagtaccga caataaatac    180
   gacgctgcgg gatactctgt agataacgaa aacccgctct ctggaaaagc tggaggcgtg    240
   gtcaaagtga cgtatccagg actgacgaag gtcctcgcac taaaagtgga taatgccgaa    300
   actattaaga aagagttagg tttaagtctc actgaaccgt tgatggagca agtcggaaca    360
   gaagagttta tcaaaaggtt cggggatggt gcttcgcgtg tagtactcag ccttcccttt    420
   gccgagggga gttctagcgt cgaatatatt aataactggg aacaggcgaa agcgttaagc    480
   gtagaacttg agattaattt tgaaacccgt ggaaaacgtg gccaagatgc gatgtatgag    540
   tatatggctc aagcctgtgc aagagggata ataacttcta aaactaaatc attggttcca    600
   cgtggatcca aggccttgtc atgcataaat cttgattggg atgtcataag ggataaaact    660
   aagacaaaga tagagtcttt gaaagagcat ggccctatca aaaataaaat gagcgaaagt    720
   cccaataaaa cagtatctga ggaaaaagct aaacaatacc tagaagaatt tcatcaaacc    780
   gcattagagc atcctgaatt gtcagaactt aaaaccgtta ctgggaccaa tcctcttttc    840
   gctggggcta actatgcggc gtgggcagta aacgttgcgc aagttatcga tagcgaaaca    900
   gctgataatt tggaaaagac aactgctgct ctttcgatac ttcctggtat cggtagcgta    960
   atgggcattg cagacggtgc cgttcaccac aatacagaag agatagtggc acaatcaata   1020
   gctttatcgt ctttaatggt tgctcaagct attccattgg taggagagct agttgatatt   1080
   ggtttcgctg catataattt tgtagagagt attatcaatt tgtttcaagt agttcataat   1140
   tcgtataatc gtcccgcgta ttctccgggg cataaaacgc aaccatttct cgagcaccac   1200
   caccaccacc actaa                                                    1215
<210> 20
   <211> 404
   <212> PRT
   <213> Corynebacterium diphtheriae
<400> 20
   Met Ala Asp Asp Val Val Asp Ser Ser Lys Ser Phe Val Met Glu Asn
   1               5                   10                  15
   Phe Ser Ser Tyr His Gly Thr Lys Pro Gly Tyr Val Asp Ser Ile Gln
   20 25 30
   Lys Gly Ile Gln Lys Pro Lys Ser Gly Thr Gln Gly Asn Tyr Asp Asp
   35 40 45
   Asp Trp Lys Gly Phe Tyr Ser Thr Asp Asn Lys Tyr Asp Ala Ala Gly
   50 55 60
   Tyr Ser Val Asp Asn Glu Asn Pro Leu Ser Gly Lys Ala Gly Gly Val
   65 70 75 80
   Val Lys Val Thr Tyr Pro Gly Leu Thr Lys Val Leu Ala Leu Lys Val
   85 90 95
   Asp Asn Ala Glu Thr Ile Lys Lys Glu Leu Gly Leu Ser Leu Thr Glu
   100 105 110
   Pro Leu Met Glu Gln Val Gly Thr Glu Glu Phe Ile Lys Arg Phe Gly
   115 120 125
   Asp Gly Ala Ser Arg Val Val Leu Ser Leu Pro Phe Ala Glu Gly Ser
   130 135 140
   Ser Ser Val Glu Tyr Ile Asn Asn Trp Glu Gln Ala Lys Ala Leu Ser
   145 150 155 160
   Val Glu Leu Glu Ile Asn Phe Glu Thr Arg Gly Lys Arg Gly Gln Asp
   165 170 175
   Ala Met Tyr Glu Tyr Met Ala Gln Ala Cys Ala Arg Gly Ile Ile Thr
   180 185 190
   Ser Lys Thr Lys Ser Leu Val Pro Arg Gly Ser Lys Ala Leu Ser Cys
   195 200 205
   Ile Asn Leu Asp Trp Asp Val Ile Arg Asp Lys Thr Lys Thr Lys Ile
   210 215 220
   Glu Ser Leu Lys Glu His Gly Pro Ile Lys Asn Lys Met Ser Glu Ser
   225 230 235 240
   Pro Asn Lys Thr Val Ser Glu Glu Lys Ala Lys Gln Tyr Leu Glu Glu
   245 250 255
   Phe His Gln Thr Ala Leu Glu His Pro Glu Leu Ser Glu Leu Lys Thr
   260 265 270
   Val Thr Gly Thr Asn Pro Val Phe Ala Gly Ala Asn Tyr Ala Ala Trp
   275 280 285
   Ala Val Asn Val Ala Gln Val Ile Asp Ser Glu Thr Ala Asp Asn Leu
   290 295 300
   Glu Lys Thr Thr Ala Ala Leu Ser Ile Leu Pro Gly Ile Gly Ser Val
   305 310 315 320
   Met Gly Ile Ala Asp Gly Ala Val His His Asn Thr Glu Glu Ile Val
   325 330 335
   Ala Gln Ser Ile Ala Leu Ser Ser Leu Met Val Ala Gln Ala Ile Pro
   340 345 350
   Leu Val Gly Glu Leu Val Asp Ile Gly Phe Ala Ala Tyr Asn Phe Val
   355 360 365
   Glu Ser Ile Ile Asn Leu Phe Gln Val Val His Asn Ser Tyr Asn Arg
   370 375 380
   Pro Ala Tyr Ser Pro Gly His Lys Thr Gln Pro Phe Leu Glu His His
   385 390 395 400
   His His His His
<210> 21
   <211> 1650
   <212> DNA
   <213> Ricinus communis
<400> 21
   atggtcccca aacaataccc aattataaac tttaccacag cgggtgccac tgtgcaaagc     60
   tacacaaact ttatcagagc tgttcgcggt cgtttaacaa ctggagctga tgtgagacat    120
   gatataccag tgttgccaaa cagagttggt ttgcctataa accaacggtt tattttagtt    180
   gaactctcaa atcatgcaga gctttctgtt acattagccc tggatgtcac caatgcatat    240
   gtggtcggct accgtgctgg aaatagcgca tatttctttc atcctgacaa tcaggaagat    300
   gcagaagcaa tcactcatct tttcactgat gttcaaaatc gatatacatt cgcctttggt    360
   ggtaattatg atagacttga acaacttgct ggtaatctga gagaaaatat cgagttggga    420
   aatggtccac tagaggaggc tatctcagcg ctttattatt acagtactgg tggcactcag    480
   cttccaactc tggctcgttc ctttataatt tgcatccaaa tgatttcaga agcagcaaga    540
   ttccaatata ttgagggaga aatgcgcacg agaattaggt acaaccggag atctgcacca    600
   gatcctagcg taattacact tgagaatagt tgggggagac tttccactgc aattcaagag    660
   tctaaccaag gagcctttgc tagtccaatt caactgcaaa gacgtaatgg ttccaaattc    720
   agtgtgtacg atgtgagtat attaatccct atcatagctc tcatggtgta tagatgcgca    780
   cctcctaggg ggataataac ttctaaaact aaatcattgg ttccacgtgg atccaaggct    840
   gatgtttgta tggatcctga gcccatagtg cgtatcgtag gtcgaaatgg tctatgtgtt    900
   gatgttaggg atggaagatt ccacaacgga aacgcaatac agttgtggcc atgcaagtct    960
   aatacagatg caaatcagct ctggactttg aaaagagaca atactattcg atctaatgga   1020
   aagtgtttaa ctacttacgg gtacagtccg ggagtctatg tgatgatcta tgattgcaat   1080
   actgctgcaa ctgatgccac ccgctggcaa atatgggata atggaaccat cataaatccc   1140
   agatctagtc tagttttagc agcgacatca gggaacagtg gtaccacact tacggtgcaa   1200
   accaacattt atgccgttag tcaaggttgg cttcctacta ataatacaca accttttgtt   1260
   acaaccattg ttgggctata tggtctgtgc ttgcaagcaa atagtggaca agtatggata   1320
   gaggactgta gcagtgaaaa ggctgaacaa cagtgggctc tttatgcaga tggttcaata   1380
   cgtcctcagc aaaaccgaga taattgcctt acaagtgatt ctaatatacg ggaaacagtt   1440
   gttaagatcc tctcttgtgg ccctgcatcc tctggccaac gatggatgtt caagaatgat   1500
   ggaaccattt taaatttgta tagtggattg gtgttagatg tgaggcgatc ggatccgagc   1560
   cttaaacaaa tcattcttta ccctctccat ggtgacccaa accaaatatg gttaccatta   1620
   tttctcgagc accaccacca ccaccactaa                                     1650
<210> 22
   <211> 549
   <212> PRT
   <213> Ricinus communis
<400> 22
   Met Val Pro Lys Gln Tyr Pro Ile Ile Asn Phe Thr Thr Ala Gly Ala
   1 5 10 15
   Thr Val Gln Ser Tyr Thr Asn Phe Ile Arg Ala Val Arg Gly Arg Leu
   20 25 30
   Thr Thr Gly Ala Asp Val Arg His Asp Ile Pro Val Leu Pro Asn Arg
           35                  40                  45
   Val Gly Leu Pro Ile Asn Gln Arg Phe Ile Leu Val Glu Leu Ser Asn
       50                  55                  60
   His Ala Glu Leu Ser Val Thr Leu Ala Leu Asp Val Thr Asn Ala Tyr
   65                  70                  75                  80
   Val Val Gly Tyr Arg Ala Gly Asn Ser Ala Tyr Phe Phe His Pro Asp
                   85                  90                  95
   Asn Gln Glu Asp Ala Glu Ala Ile Thr His Leu Phe Thr Asp Val Gln
               100                 105                 110
   Asn Arg Tyr Thr Phe Ala Phe Gly Gly Asn Tyr Asp Arg Leu Glu Gln
           115                 120                 125
   Leu Ala Gly Asn Leu Arg Glu Asn Ile Glu Leu Gly Asn Gly Pro Leu
       130                 135                 140
   Glu Glu Ala Ile Ser Ala Leu Tyr Tyr Tyr Ser Thr Gly Gly Thr Gln
   145                 150                 155                 160
   Leu Pro Thr Leu Ala Arg Ser Phe Ile Ile Cys Ile Gln Met Ile Ser
                   165                 170                 175
   Glu Ala Ala Arg Phe Gln Tyr Ile Glu Gly Glu Met Arg Thr Arg Ile
               180                 185                 190
   Arg Tyr Asn Arg Arg Ser Ala Pro Asp Pro Ser Val Ile Thr Leu Glu
           195                 200                 205
   Asn Ser Trp Gly Arg Leu Ser Thr Ala Ile Gln Glu Ser Asn Gln Gly
       210                 215                 220
   Ala Phe Ala Ser Pro Ile Gln Leu Gln Arg Arg Asn Gly Ser Lys Phe
   225                 230                 235                 240
   Ser Val Tyr Asp Val Ser Ile Leu Ile Pro Ile Ile Ala Leu Met Val
                   245                 250                 255
   Tyr Arg Cys Ala Pro Pro Arg Gly Ile Ile Thr Ser Lys Thr Lys Ser
               260                 265                 270
   Leu Val Pro Arg Gly Ser Lys Ala Asp Val Cys Met Asp Pro Glu Pro
           275                 280                 285
   Ile Val Arg Ile Val Gly Arg Asn Gly Leu Cys Val Asp Val Arg Asp
       290                 295                 300
   Gly Arg Phe His Asn Gly Asn Ala Ile Gln Leu Trp Pro Cys Lys Ser
   305                 310                 315                 320
   Asn Thr Asp Ala Asn Gln Leu Trp Thr Leu Lys Arg Asp Asn Thr Ile
                   325                 330                 335
   Arg Ser Asn Gly Lys Cys Leu Thr Thr Tyr Gly Tyr Ser Pro Gly Val
               340                 345                 350
   Tyr Val Met Ile Tyr Asp Cys Asn Thr Ala Ala Thr Asp Ala Thr Arg
           355                 360                 365
   Trp Gln Ile Trp Asp Asn Gly Thr Ile Ile Asn Pro Arg Ser Ser Leu
   370 375 380
   Val Leu Ala Ala Thr Ser Gly Asn Ser Gly Thr Thr Leu Thr Val Gln
   385 390 395 400
   Thr Asn Ile Tyr Ala Val Ser Gln Gly Trp Leu Pro Thr Asn Asn Thr
   405 410 415
   Gln Pro Phe Val Thr Thr Ile Val Gly Leu Tyr Gly Leu Cys Leu Gln
   420 425 430
   Ala Asn Ser Gly Gln Val Trp Ile Glu Asp Cys Ser Ser Glu Lys Ala
   435 440 445
   Glu Gln Gln Trp Ala Leu Tyr Ala Asp Gly Ser Ile Arg Pro Gln Gln
   450 455 460
   Asn Arg Asp Asn Cys Leu Thr Ser Asp Ser Asn Ile Arg Glu Thr Val
   465 470 475 480
   Val Lys Ile Leu Ser Cys Gly Pro Ala Ser Ser Gly Gln Arg Trp Met
   485 490 495
   Phe Lys Asn Asp Gly Thr Ile Leu Asn Leu Tyr Ser Gly Leu Val Leu
   500 505 510
   Asp Val Arg Arg Ser Asp Pro Ser Leu Lys Gln Ile Ile Leu Tyr Pro
   515 520 525
   Leu His Gly Asp Pro Asn Gln Ile Trp Leu Pro Leu Phe Leu Glu His
   530 535 540
   His His His His His
   545

## Beschreibung

Ein Gegenstand der vorliegenden Erfindung betrifft ein Verfahren zur Herstellung von Proteinen in zweikettiger Form mittels rekombinanter Expression in E. coli-Wirtszellen. Ein anderer Aspekt der vorliegenden Erfindung betrifft Proteine bzw. Polypeptide in zweikettiger und biologisch aktiver Form, die sich mittels des genannten Verfahrens herstellen lassen.

Der wesentliche Vorteil gegenüber entsprechenden rekombinanten Proteinen/Polypeptiden, die nicht die erfindungsgemäßen Merkmale aufweisen, besteht darin, dass sie nicht mit einer spezifischen Protease zur gezielten Spaltung der Polypeptidkette behandelt werden müssen, so dass sich das Herstellungsverfahren wesentlich vereinfacht. Weitere Gegenstände der vorliegenden Erfindung sind Nukleinsäuren, die für die erfindungsgemäßen Polypeptide/Proteine kodieren, Vektoren, die solche Nukleinsäuren bzw. Nukleinsäure-Sequenzen enthalten, Wirtszellen, die wiederum die genannten Vektoren enthalten und schließlich pharmazeutische Zubereitungen, die die zweikettigen und biologisch aktiven Proteine/Polypeptide enthalten.

Clostridiale Neurotoxine sind starke Inhibitoren der kalziumabhängigen Neurotransmitter-Sekretion in neuronalen Zellen. Nach oraler Aufnahme von Botulinum-Toxinen (BoNT), z.B. über verdorbene Lebensmittel, kommt es zu einem als Botulismus bezeichnetem Krankheitsbild, das durch Lähmung verschiedener Muskeln gekennzeichnet ist. Eine Lähmung der Atemmuskulatur kann schließlich zum Tod des Betroffenen führen. Dabei ist die Signalweiterleitung vom Nerv zum Muskel an der motorischen Endplatte unterbrochen, da die Motorneuronen kein Acetylcholin mehr ausschütten können. Ihre hemmende Wirkung entfalten die Botulinum-Toxine durch die proteolytische Spaltung von an den Sekretionsprozessen beteiligten Proteinen, den sogenannten SNARE-Proteinen. Dabei besitzen die Neurotoxine verschiedener Serotypen unterschiedliche Spezifitäten hinsichtlich der SNARE-Proteine und der Spaltstellen in den jeweiligen Aminosäure-Sequenzen. BoNT(A) und BoNT(E) spalten das SNARE-Protein SNAP-25, während BoNT(C) sowohl SNAP-25 als auch Syntaxin-1 als Substrat erkennt. Die Toxine der Serotypen B, D, F und G sowie das Tetanus-Toxin (TeNT) schließlich spalten VAMP-2 (Synaptobrevin-2) (Schiavo et al., 1997).

Bei den clostridialen Neurotoxinen handelt es sich um die stärksten bekannten Gifte. So beträgt die intravenös verabreichte letale Dosis, bei der die Hälfte aller Mäuse der Dosisgnippe an Botulismus sterben, lediglich 5 pg. Dass die Toxine der meisten Serotypen auch oral toxisch wirken, ist auf Komplexproteine zurückzuführen, in die sie eingebettet sind und die sie somit in der Magen-Darm-Passage vor dem Abbau durch Verdauungsenzyme schützen. Ihnen wird auch eine Funktion bei der Resorption der Toxine durch das Dünndarm-Epithel zugeschrieben (Fujinaga, 1997).

Im Laufe der letzten Jahrzehnte wurden die Botulinum-Toxine der Serotypen A und B therapeutisch nutzbar gemacht. So können durch gezielte Injektion von nur geringen Dosen einzelne, chronisch verkrampfte Muskeln entspannt werden. Ein besonderer Vorteil ist die lange Wirkdauer von z.B. BoNT(A) und BoNT(B) über drei bis sechs Monate. Erste Indikationsgebiete sind u.a. Dystonien wie Torticollis, Blepharospasmus und Strabismus gewesen, weitere wie Hyperhydrose oder kosmetische Behandlungen zur Faltenglättung sind hinzugekommen. Der Markt für Botulinum-Toxin als Therapeutikum wächst rapide, nicht zuletzt durch die Erschließung weiterer Indikationsgebiete und der intensiveren Nutzung bei bereits bestehenden Anwendungen. Dabei gibt es Bestrebungen, die Eigenschaften der Neurotoxine hinsichtlich der Wirkdauer, Wirkstärke und des antigenen Potentials zu verbessern. Untersuchungen haben gezeigt, dass die Komplexproteine, die in den bislang kommerziell erhältlichen Präparationen (BOTOX von Allergan und Dysport von Ipsen-Beaufort als BoNT(A)-Präparationen sowie Myobloc/Neurobloc von Elan als BoNT(B)-Präparation) enthalten sind, keinerlei positive Auswirkungen auf Wirkdauer und -stärke haben, aber aufgrund der im Vergleich zu einer Präparation des reinen Neurotoxins mit gleicher Aktivität höheren Proteinmenge dazu beitragen können, im Patienten eine Immunreaktion auszulösen, die weitere Injektionen unwirksam werden lässt.

Da also die Komplexproteine in der Wirkstoff-Formulierung nicht benötigt werden und sogar eher von Nachteil sind und einige Modifikationen zur Verbesserung der Eigenschaften nur auf gentechnischem Wege zu erreichen sind, besteht ein hoher Bedarf, die Neurotoxine rekombinant, z.B. durch Expression in *Escherichia coli* herzustellen (so erzeugte Neurotoxine sind frei von den oben genannten Komplexproteinen). Neue Indikationsgebiete sollen zudem dadurch erschlossen werden, dass den Botulinum-Toxinen eine andere Zellspezifität verliehen wird. Auch hierfür ist der Weg über ein rekombinantes Toxin bzw. Toxinderivat zu bevorzugen.

Die Botulinum-Toxine sowie das Tetanus-Toxin weisen hohe Homologien in ihrer Aminosäure-Sequenz auf und ähneln sich insbesondere in ihrer Domänenstruktur. Sie bestehen aus einer Rezeptorbindungsdomäne (H_{C}), einer Translokationsdomäne (H_{N}) und einer katalytischen Untereinheit (L), die in der Nervenzelle die Spaltung des entsprechenden SNARE-Proteins bewirkt. H_{C} ist für die spezifische Bindung der Neurotoxine an die motorischen Endplatten verantwortlich, während die Translokationsdomäne dafür sorgt, dass L aus den Endosomen in das Zytoplasma der Neuronen gelangt. H_{N} (N-terminales Ende) und H_{C} (C-terminales Ende) bilden die Schwere Kette von 100 kDa, während L die Leichte Kette ist und die katalytische Untereinheit von 50 kDa ausmacht. Beide Polypeptidketten sind durch eine Disulfidbrücke miteinander verbunden. Zwischen den beteiligten Cystein-Resten erstreckt sich ein Linker- oder Loop-Bereich (synonym dafür auch Linker- oder Loop-Sequenz bzw. vereinfacht Linker oder Loop), dessen (deren) Länge zwischen den Botulinum-Toxinen der einzelnen Serotypen stark variiert. Spätestens bei der Freisetzung der Toxine aus den Clostridien im Zuge der Zelllyse wird der Loop durch eine bislang nicht charakterisierte clostridiale Endopeptidase gespalten, wobei das Verhältnis von gespaltenen und ungespaltenen Spezies zwischen den Serotypen variiert. Für die Aktivität der Neurotoxine ist die Spaltung des Loops zum Zwei-Ketten-Toxin essentiell (Schiavo et al., 1997). Beispielsweise wird beim Botulinum-Neurotoxin A ein Dekapeptid aus dem Loop ausgeschnitten, das heißt, in der Loop-Sequenz VRGIITSKTKSLDKGYNKALNDL, die N- wie C-terminal einen Cystein-Rest als unmittelbaren Nachbarn hat, wird nicht nur *eine* Peptidbindung gespalten, sondern es wird zweimal proteolytisch gespalten. Damit liegt das Molekulargewicht des biologisch aktiven Botulinum-Neurotoxins A naturgemäß unter dem des ursprünglich clostridiell translatierten Toxins.

Da die clostridiale Protease in anderen Wirtsorganismen wie *Escherichia coli* nicht vorhanden ist, werden rekombinante Botulinum-Toxine und deren Fragmente bzw. Derivate dort als einkettige Polypeptide exprimiert. Dies gilt entsprechend auch für beliebige andere Proteine, die ihre normale biologische/biochemische Aktivität als zweikettige Proteine ausüben: allgemein werden solche Proteine mittels der rekombinanten DNA-Technologie als einkettige Proteine erhalten, ihre biologische/biochemische Aktivität, die sie natürlicherweise als zweikettige Proteine ausüben, ist daher kaum oder gar nicht vorhanden.

Um ein aktives Protein, insbesondere ein aktives Botulinum-Toxin zu generieren, ist daher bislang der Einbau einer Erkennungssequenz für eine sequenzspezifische Protease wie z.B. Thrombin, Faktor Xa oder Genenase erforderlich, so dass nach der Reinigung eine Spaltung und damit Aktivierung durch Zugabe einer dieser Endoproteasen durchgeführt werden kann. Dies ist zum Beispiel in WO 2004/024909 beschrieben. Die Verwendung einer solchen Endoprotease hat im wesentlichen zwei Nachteile: Zum einen ist nicht immer auszuschließen, dass noch weitere Spaltstellen außer der einen, gentechnisch eingefügten, Spaltstelle in der Aminosäure-Sequenz vorhanden sind. Auch wenn an diesen Sekundärspaltstellen wesentlich ineffizienter geschnitten werden mag, kann nach der Proteasebehandlung ein Gemisch verschiedener Spaltvarianten des Toxins entstehen, das nur schwer aufzutrennen wäre. Zum anderen ist es bei pharmazeutischen Präparationen aus arzneimittelrechtlichen (regulatorischen) Gründen von erheblichem Nachteil, nachträglich ein Protein zuzufügen bzw. die Präparation mit einem weiteren Protein in Kontakt kommen zu lassen, da in der weiteren Aufarbeitung die vollständige Entfernung dieses Proteins und seiner ggf. vorhandenen Verunreinigungen nachgewiesen werden muss, was in der Regel mit einem erheblichen Aufwand verbunden ist.

Eine Aktivierung durch proteolytische Spaltung zu einem zweikettigen disulfidverbrückten Polypeptid ist auch bei anderen bakteriellen Toxinen wie z.B. dem Pseudomonas-Exotoxin oder Diphtheria-Toxin notwendig, damit die enzymatische Domäne die toxische Wirkung (z.B. durch ADP-Ribosylierung eines Elongationsfaktors und damit Hemmung der Proteinsynthese) ausüben kann. Diese Toxine finden Verwendung bei der Herstellung sogenannter Immunotoxine, die insbesondere in der Tumortherapie eingesetzt werden. Hierfür wird die Zellbindungsdomäne des Toxins gegen eine Protein-Domäne ausgetauscht, die eine hohe Bindungsaffinität zu einem tumorspezifischen Oberflächenprotein (Differenzierungsantigen oder tumorassoziiertes Antigen) aufweist. Während in den klassischen Immunotoxinen diese Protein-Domäne aus einem monoklonalen Antikörper oder einem Fragment desselben besteht, kann die Spezifität für bestimmte Tumorzellen auch durch Cytokine, Wachstumsfaktoren sowie mutierte und selektierte Proteine aus der Familie der Affiline, Ankyrin Repeat Proteine oder Anticaline verliehen werden, um nur einige Beispiele zu nennen. Bei der rekombinanten Expression derartiger Fusionsproteine erhält man einkettige Polypeptide. Während z.B. Ricin keine Prozessierungsstelle für Proteasen außer derjenigen aus *Ricinus communis* besitzt und eine solche erst eingefügt werden muss, können die Diphteria-Toxin- und Pseudomonas-Exotoxin-Fragmente als Bestandteile der Immunotoxine nach der Internalisierung im endosomalen Kompartiment durch eine Protease der Zielzelle gespalten werden. Dies geschieht im Loop-Bereich zwischen den Cystein-Resten, die eine Disulfidbrücke ausbilden. Allerdings werden nicht alle internalisierten Immunotoxin-Moleküle derartig prozessiert, sondern nur ein geringer Teil (Ogata et al., 1990).

Um rekombinante Proteine, insbesondere kleinere Polypeptide, in ausreichenden Mengen und in löslicher Form zu erhalten, ist es in vielen Fällen notwendig, diese als Fusions- bzw. Hybridprotein mit z.B. Glutathion-S-Transferase oder Maltose-Bindungsprotein in *E. coli* zu exprimieren. Ferner sind zahlreiche Expressionssysteme auf dem Markt, durch die das gewünschte Polypeptid mit einem N- oder C-terminalen Anhängsel zur Affinitätsreinigung wie dem His-Tag, StrepTag oder FLAG-Tag exprimiert wird. Vielfach befindet sich im Expressionsplasmid zwischen der multiplen Klonierungsstelle, wo die für das gewünschte Protein codierende DNS-Sequenz eingefügt wird, und der codierenden Sequenz für den Fusionspartner oder das Affinitätsanhängsel eine Protease-Erkennungssequenz. Diese soll es ermöglichen, dass nach Expression und Reinigung des Fusionsproteins das gewünschte Protein durch Zusatz einer entsprechenden sequenzspezifischen Endoprotease (z.B.Thrombin, Faktor Xa oder Genenase) von den zusätzlichen Peptidbereichen befreit werden kann. Würden die beiden Fusionspartner nicht durch eine Peptidbindung, sondern über eine Disulfidbrücke kovalent miteinander verbunden sein, könnte nach der Reinigung eine Trennung voneinander durch eine einfache Reduktion mit thiolhaltigen Substanzen wie ß-Mercaptoethanol, DTT oder reduziertes Glutathion erfolgen. Beispielsweise ließe sich das gewünschte Protein von einer Affinitätsmatrix wie z.B. Ni-NTA-Agarose oder StrepTactin-Sepharose mit den genannten Reduktionsmitteln eluieren, während die Affinitätsanhängsel an der Matrix gebunden blieben. Ein weiterer Reinigungsschritt zur Abtrennung des Affinitätsanhängsels oder einer zugefügten Endoprotease könnte somit unterbleiben.

Wünschenswert wäre daher ein Verfahren der rekombinanten Expression von Proteinen/Polypeptiden allgemein, insbesondere von Neurotoxinen sowie von Fragmenten und Derivaten dieser Neurotoxine, und von Fusions- bzw. Hybridproteinen, insbesondere von Immunotoxinen, die bereits nach der Lyse der Wirtszellen in ihrer (biologisch) aktiven Zwei-Ketten-Struktur vorliegen, wobei die beiden Ketten disulfidverbrückt sind. Ein solches Verfahren zur Herstellung derartiger Proteine und Polypeptide wird durch die hier beschriebene Erfindung bereitgestellt.

Überraschenderweise hat der Erfinder nun gefunden, dass das LH_{N}-Fragment von BoNT(A) wie auch das komplette Neurotoxin A, die rekombinant einkettig erhalten werden, ihre normale biologische/biochemische Aktivität aber in zweikettiger, disulfidverbrückter Form ausüben, rekombinant in zweikettiger Form erhalten werden, wenn das LH_{N}-Fragment bzw. das komplette Toxin, vorzugsweise auf dem Nukleinsäure-Level, wenigstens einer bestimmten Änderung unterworfen werden wie in Anspruch 1 dargestellt. Nachfolgende Untersuchungen des Erfinders haben ergeben, dass entsprechendes auch für beliebige andere Proteine/Polypeptide zutrifft, sofern sie nach herkömmlichen rekombinanten Verfahren einkettig erhalten werden, ihre biologische Aktivität aber in zweikettiger, disulfidverbrückter Form ausüben.

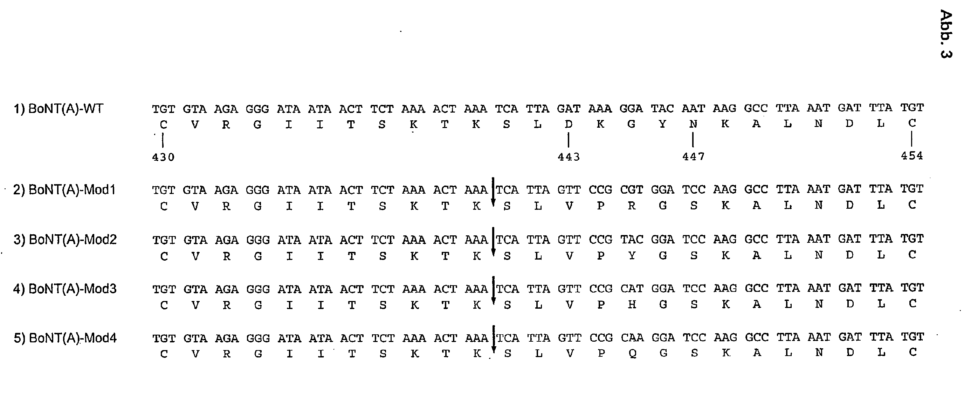

Bei der genannten "wenigstens einen Änderung" handelt es sich im Fall von BoNT(A) bzw. im Fall des LH_{N}-Fragments von BoNT(A) um die Einfügung (Insertion) einer hierin als PRS (für protease recognition site) bezeichneten Pentapeptid-Sequenz wie in Anspruch 1 dargestellt. Im allgemeinen Fall des Proteins/ Polypeptids kann eine Pentapeptid-Sequenz, die in dem zu verändernden Protein/Polypeptid vorhanden ist, (vorzugsweise auf dem Nukleinsäure-Level) alternativ so modifiziert werden (z.B. durch mindestens einen Austausch eines Aminosäure-Restes oder durch Insertion von nur einigen Aminosäure-Resten der PRS oder durch Deletion von Aminosäure-Resten), dass sie der durch Insertion in die vorhandene Sequenz eingefügten Pentapeptid-Sequenz PRS entspricht. Genauso kann auch eine Hexa-/Hepta-/Okta-(etc.)peptid-Sequenz inseriert werden, mit oder ohne dass eine oder zwei oder drei oder mehrere Aminosäure-Reste deletiert werden müssen. Erfindungsgemäß vorteilhaft ist einzig, dass das schließlich exprimierte Polypeptid die PRS-(Pentapeptid-)Sequenz in seinem Loop-Bereich aufweist, wobei der Loop-Bereich erfindungsgemäß definiert ist als die zwischen den beiden an der Disulfidbrücke beteiligten Cystein-Resten liegende Aminosäure-Sequenz. Liegt diese PRS-Sequenz im Loop-Bereich vor, führt dies dazu, dass bei Spaltung des einkettigen Polypeptids in Nachbarschaft zu der Polypeptid-Sequenz PRS (auf Aminosäure-Level) die auch natürlicherweise auf zwei verschiedenen Ketten vorliegenden Sequenzen auf zwei verschiedenen Ketten vorliegen. Im Fall des Botulinum-Neurotoxins A (BoNT(A)) wird diese PRS-Sequenz vorzugsweise unter Deletion des Pentapeptids Asp₄₄₃ - Asn₄₄₇ von BoNT(A) (siehe Fig. 3-1) in den Loop eingefügt. Bei anderen Proteinen/Polypeptiden (z.B. bei BoNT(B), BoNT(C1), BoNT(D), BoNT(E), bei Ricin, bei PE40 des Pseudomonas-Exotoxins oder bei dem Diphtheria-Toxin (DT)) ist es stattdessen bevorzugt, einen modifizierten Loop von BoNT(A) (siehe Fig. 3-2 bis 3-5) in die Loop-Sequenz einzuführen (wobei Aminosäure-Reste der natürlichen Loop-Sequenz deletiert werden können oder auch nicht). Die modifizierten Loop-Sequenzen in Fig. 3-2 bis 3-5 sind die jeweiligen Sequenzen ohne die beiden randständigen Cys-Reste, wobei die zentrale Aminosäure der PRS-Sequenz nicht nur R, Y, H oder Q, sondern jede der natürlich vorkommenden Aminosäuren sein kann. Bei den soeben genannten anderen Proteinen/Polypeptiden ist es besonders bevorzugt, nur einen Teil des modifizierten Loops von BoNT(A), insbesondere die Sequenz GIITSKTKSLVPXGSKALNDL (X = eine natürlich vorkommende Aminosäure), in die Loop-Sequenz einzuführen (wobei Aminosäure-Reste der natürlichen Loop-Sequenz deletiert werden können oder auch nicht). Die modifizierten Loop-Sequenzen in Fig. 3-2 bis 3-5 sind die jeweiligen Sequenzen ohne die beiden randständigen Cys-Reste.

Für das LH_{N}-Fragment von BoNT(A) bzw. für das komplette rekombinante Toxin heißt das also, dass die Sequenz-Änderung eine Veränderung im Loop-Bereich zwischen L und H_{N} ist, die für das Vorliegen einer PRS-Sequenz sorgt. Die PRS-Sequenz ist erfindungsgemäß, und nicht nur für BoNT(A), die Pentapeptid-Sequenz Val-Pro-Xaa-Gly-Ser. Xaa steht für jede beliebige, natürlich vorkommende Aminosäure. Egal ob Xaa Arg oder eine andere natürlich vorkommende Aminosäure ist, die Pentapeptid-Sequenz Val-Pro-Xaa-Gly-Ser wird in jedem Fall als PRS-Pentapeptid-Sequenz bezeichnet. Ist hingegen einer der vier anderen Aminosäure-Reste der PRS-Sequenz ausgetauscht, was im Rahmen der Erfindung durchaus sein kann, insbesondere durch entsprechend hydrophile/hydrophobe bzw. polare oder unpolare Reste, ist hier und nachfolgend die Rede von einer Variante der PRS-Pentapeptid-Sequenz. Varianten liegen z.B. vor, wenn Val durch Leu, Ile, Ala, Phe, Pro oder Gly ersetzt ist. Des weiteren liegen Varianten vor, wenn (auch oder nur) Prolin an der zweiten Position der PRS, gesehen vom N-Terminus aus, durch z.B Leu, Ile, Ala, Phe, Val oder Gly ersetzt ist. Auch das Glycin an der vierten Position der PRS schließlich kann z.B. durch Leu, Ile, Ala, Pro, Phe oder Val ersetzt sein, was zu wiederum anderen Varianten führt. Und wenn das Serin an der fünften Position der PRS z.B. durch Tyr, Trp oder Thr, ggf. auch durch Cys oder Met, ersetzt ist, liegt eine weitere Art von Varianten vor. Erfindungsgemäß werden als Varianten der Pentapeptid-Sequenz also solche Sequenzen bezeichnet, die an mindestens einer der Positionen 1, 2, 4 und 5 der PRS-Sequenz einen Aminosäure-Rest enthalten, der von Val-1, Pro-2, Gly-4 und/oder Ser-5 verschieden ist.

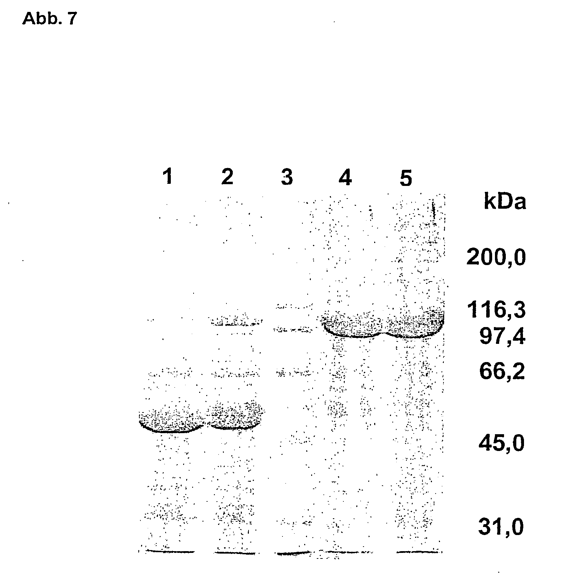

Enthält das LH_{N}-Fragment von BoNT(A) (oder das komplette Toxin) oder irgendein anderes Protein/Polypeptid, das rekombinant normalerweise als einkettiges Protein/Polypeptid erhalten wird, biologisch/biochemisch aktiv aber (nur) in zweikettiger Form ist, die Pentapeptid-Sequenz Val-Pro-Xaa-Gly-Ser (wobei Xaa für jede beliebige der 20 natürlich vorkommenden Aminosäuren steht, und auch die vier anderen Aminosäure-Reste im Sinne des vorangehenden Absatzes ausgetauscht sein können), wird es im Lysat der E. coli-Wirtszellen (z.B. *E. coli* K12, insbesondere *E. coli* K12-Wirtszellen der Stämme M15[pREP4], XL1-BLUE oder UT5600) in der zweikettigen Form vorliegen, wobei im Fall des BoNT(A) die Leichte Kette mit H_{N} bzw. mit der kompletten Schweren Kette durch eine Disulfidbrücke kovalent verknüpft bleibt (Fig. 7). Die Spaltung der Polypeptidkette erfolgt entweder unmittelbar nach der Zelllyse oder ist nach mehrstündiger Inkubation des Zelllysates weitgehend abgeschlossen. Eine Autoproteolyse durch die Aktivität der Protease-Domäne des Toxins bzw. Toxin-Fragments kann ausgeschlossen werden, da Protease-inaktive und im Loop-Bereich entsprechend modifizierte Mutanten nach Expression und Aufschluss der *E. coli*-Wirtszellen ebenfalls in der zweikettigen Struktur vorliegen. Offensichtlich ist für die Spaltung der PRS-Pentapeptid-Sequenz eine Protease des *E. coli*-Wirtsstammes verantwortlich.

Eine weitere erfindungsgemässe Änderung gemäß des Absatzes "Überraschenderweise hat der Erfinder ...." vier Absätze weiter oben (auf Seite 5) besteht darin, dass N-terminal der PRS-Sequenz im Abstand von 1 bis 20 Aminosäure-Resten (die Aminosäure Richtung N-Terminus, die unmittelbar benachbart dem Valin-Rest der Pentapeptid-PRS-Sequenz sitzt, im Fall der Fig. 3-2 bis 3-5 ein Leucin-Rest, hat einen Abstand von 1 Aminosäure-Rest von der PRS-Sequenz), insbesondere im Abstand von 3 bis 15 Aminosäure-Resten, speziell im Abstand von 3 bis 10 Aminosäure-Resten, besonders bevorzugt im Abstand von 3 bis 8 Aminosäure-Resten, und ganz besonders bevorzugt im Abstand von 3 Aminosäure-Resten, ein basischer Aminosäure-Rest, bevorzugt ein Lysin- oder Arginin-Rest vorhanden ist, an dessen C-Terminus die Protease der *E. coli*-Wirtszelle die Loop-Sequenz spaltet. Nach der Spaltung wird also ein Polypeptid erhalten, das z.B. zwei Aminosäure-Reste (wenn der gerade definierte Abstand 3 Aminosäure-Reste beträgt) N-terminal von dem Valin-Rest der PRS-Sequenz aufweist. Im vorliegenden Fall bedeutet "Änderung" nicht notwendigerweise eine Änderung im wahren Sinne, also z.B. eine Insertion oder Substitution eines Aminosäure-Restes, so dass anschließend N-terminal der PRS-Sequenz im oben vorgegebenen Abstand von 1 bis 20 Aminosäure-Resten ein basischer-Aminosäure-Rest (z.B. ein Lysin-Rest) sitzt. Entscheidend ist allein, dass ein basischer Aminosäure-Rest (wie ein Lysin- oder Arginin-Rest) N-terminal der PRS-Sequenz im vorgenannten Abstand vorliegt.

Eine weitere, gleichfalls nicht notwendige, aber bevorzugte Änderung gemäß des Absatzes "Überraschenderweise hat der Erfinder ...." fünf Absätze weiter oben besteht darin, dass die Loop-Sequenz, in der die Protease der *E. coli*-Wirtszellen spaltet, eine Länge von wenigstens neun Aminosäure-Resten hat. Bevorzugte Längen der Loop-Sequenz sind mindestens zwölf, mindestens 15, mindestens 18, mindestens 20 und mindestens 23 Aminosäure-Reste. Besonders bevorzugte Längen der Loop-Sequenz sind 1.5 bis 22, insbesondere 18 bis 22 Aminosäure-Reste.

Das erfindungsgemäße Verfahren ist ganz allgemein also ein Verfahren zur Herstellung von Proteinen/Polypeptiden in zweikettiger Form, wobei die beiden Ketten disulfidverbrückt sind, mittels rekombinanter Expression in *E. coli*-Wirtszellen, wobei (i) das Protein/Polypeptid seine biologische Aktivität als zweikettiges, disulfidverbrücktes Protein/Polypeptid ausübt, (ii) der C-terminale Aminosäure-Rest der ersten Kette ein Arg- oder Lys-Rest ist, (iii) die zweite Kette des Proteins/Polypeptids N-terminal von einem Cystein-Rest als N-Terminus 1 bis 20 Aminosäure-Reste und eine als PRS bezeichnete Pentapeptid-Sequenz VPXGS aufweist, wobei X jede beliebige natürlich vorkommende Aminosäure bedeutet, wobei V Val, Leu, Ile, Ala, Phe, Pro oder Gly bedeutet, wobei P Pro, Leu, Ile, Ala, Phe, Val oder Gly bedeutet, wobei G Gly, Leu, Ile, Ala, Pro, Phe oder Val bedeutet und wobei S Ser, Tyr, Trp oder Thr bedeutet; und (iv) das Verfahren die folgenden Schritte umfasst: (a) Veränderung des Proteins/Polypeptids, auf dem Nukleinsäure-Level, so dass das Protein/Polypeptid in seiner veränderten Form in seinem Loop-Bereich die genannte Pentapeptid-Sequenz (VPXGS) aufweist; (b) Einbringen des auf dem Nukleinsäure-Level veränderten Konstrukts in *E. coli*-Zellen; (c) Kultivierung und anschließende Lyse der Wirtszellen; und (d) Isolierung des zweikettigen Proteins/Polypeptids.

Die erste Kette des Proteins/Polypeptids ist erfindungsgemäß bevorzugterweise die Kette, die von dem N-terminalen Ende der entsprechenden DNA kodiert wird, während die zweite Kette des Proteins/Polypeptids dementsprechend die Kette ist, die von dem C-terminalen Ende der entsprechenden DNA kodiert wird. Da also die Expression von 5'-DNA-3' zu N-Polypeptid-C führt, heißt das für den vorgenannten bevorzugten Fall der Erfindung, dass die Expression wie folgt dargestellt werden kann: 5'-DNA-3' exprimiert zu N-erstePolypeptidkette-C-Loop-N-zweitePolypeptidkette-C. Der Loop wird gemäß der vorliegenden Erfindung bereits *in situ* gespalten, so dass schliesslich das erfindungsgemäßePolypeptid/Protein N-erstePolypeptidkette-C-N-zweitePolypeptidkette-C in Zweiketten-Struktur erhalten wird.

Mit dem Ausdruck "die zweite Kette des Proteins/Polypeptids weist N-terminal von einem Cystein-Rest als N-Terminus 1 bis 20 Aminosäure-Reste und eine als PRS bezeichnete Pentapeptid-Sequenz VPXGS auf" ist gemeint, dass der N-Terminus nicht von dem z.B. Valin-Rest der Pentapeptid-Sequenz VPXGS gebildet wird, sondern von einem anderen (beliebigen) Aminosäure-Rest. Zwischen diesem und dem Valin-Rest der PRS können dann noch weitere 1 bis 19 Aminosäure-Reste sitzen, der N-terminale Aminosäure-Rest kann aber mit dem z.B. Valin-Rest auch direkt mittels Peptidbindung verknüpft, also direkter Nachbar des Valin-Restes der PRS sein.

Die erfindungsgemäßen Proteine/Polypeptide, die in ihrer (biologisch) aktiven Zwei-Ketten-. Struktur isoliert werden können sind Proteine, deren C-terminales Ende der ersten Kette ein basischer Aminosäure-Rest, insbesondere ein Arg- oder Lys-Rest, ist und deren zweite Kette N-terminal 1 bis 20 Aminosäure-Reste und die als PRS bezeichnete Pentapeptid-Sequenz VPXGS aufweist, wobei X, V, P, G und S wie zuvor definiert sind.

Gemäß der vorliegenden Erfindung erübrigt sich z.B. im Falle von Immunotoxinen, die auf rekombinantem Ricin basieren, eine Behandlung durch eine sequenzspezifische Protease wie Thrombin oder Faktor Xa zur Aktivierung. Bei z.B. Diphtheria-Toxin- bzw. Pseudomonas-Exotoxin-basierten Immunotoxinen war eine deutliche Effizienzsteigerung zu erwarten, die tatsächlich auch erzielt wird, da die Prozessierung durch eine Protease der Zielzelle als geschwindigkeitbestimmender Schritt für die Translokation der enzymatischen Domäne der Toxine in das Zytoplasma nicht mehr notwendig ist. Derartige, bereits als zweikettige, disulfidverbrückte Polypeptide vorliegende Immunotoxine können in geringeren Dosen eingesetzt werden und dennoch dieselbe zelltoxische Wirkung erzielen. Dies senkt einerseits die Therapiekosten, andererseits verringert es die Gefahr der Bildung von Antikörpern, die das Immunotoxin bei weiteren Applikationen wirkungslos werden ließen. Ein Verfahren zur Herstellung von zweikettigen, disulfidverbrückten und damit aktivierten Immunotoxinen wird durch die vorliegende Erfindung bereitgestellt.

Mit dem erfindungsgemäß bereitgestellten Verfahrenlassen sich auch Fusions- bzw. Hybridproteine herstellen, also z.B. Proteine mit einem Peptidanhängsel für die Affinitätsreinigung, in einer zweikettigen Form, deren beide Polypeptidketten durch eine Disulfidbrücke kovalent verbunden und nach einer affinitätschromatographischen oder anderen Reinigungsmethode durch einfache Reduktion mit thiolhaltigen Substanzen wie ß-Mercaptoethanol, DTT oder reduziertes Glutathion voneinander zu trennen sind.

Die rekombinante Expression von clostridialen Neurotoxinen und deren Fragmenten (z.B. ein LH_{N}-Fragment oder ein Derivat eines clostridialen Neurotoxins, z.B. mit veränderter Zellspezifität) in Expressionsstämmen von *E. coli* wie M15[pREP4] oder BL21(DE3) führt zu einkettigen Polypeptiden. Durch Behandlung dieser Polypeptide mit Trypsin erfolgt eine Spaltung im Bereich der Loop-Sequenz im Übergangsbereich von der Protease- zur Translokationsdomäne. Da es sich bei Trypsin nicht um eine sequenzspezifische Protease handelt, ist eine - meist unerwünschte - Spaltung in weiteren Bereichen der Polypeptide wahrscheinlich. So wird BoNT(A) durch Trypsin zusätzlich zwischen H_{N} und H_{C} gespalten, wobei ein zweikettiges LH_{N}-Fragment und ein H_{C}-Fragment entstehen. Um eine in den meisten Fällen gewünschte selektive Spaltung im Loop-Bereich zu gewährleisten, ist das Vorliegen, ggf. nach ihrer Einführung, einer Erkennungssequenz für spezifische Endoproteasen erforderlich.

Die Spaltung von rekombinant hergestellten Fusions-/Hybridproteinen mittels sequenzspezifischer Endoproteasen wie Thrombin, Faktor Xa, Genenase etc. gehört zum allgemein bekannten Methodenspektrum. So ist es möglich, einen Fusionspartner, der dem rekombinanten Protein/Polypeptid zu einer verbesserten Löslichkeit und/oder Expression verhilft oder als Peptidanhängsel für die Affinitätsreinigung dient, nach der Reinigung abzutrennen. Dazu wird die Proteinlösung mit der geeigneten Endoprotease in löslicher Form oder immobilisiert an eine Matrix inkubiert.

Diese Technik lässt sich auch bei der Expression von den oben genannten rekombinanten Proteinen/Polypeptiden, die ihre normale biologische/biochemische Aktivität als zweikettige Proteine/Polypeptide ausüben, mittels der rekombinanten DNA-Technologie aber als inaktive einkettige Proteine/Polypeptide erhalten werden (z.B. bei der Expression von clostridialen Neurotoxinen, Fragmenten von clostridialen Neurotoxinen wie LH_{N}-Fragmenten oder von Derivaten clostridialer Neurotoxine, z.B. mit veränderter Zellspezifität) nutzbar machen: In das Polypeptid, vorzugsweise auf der Ebene der Nukleinsäuren, wird z.B. im Loop-Bereich zwischen L und H_{N} eine Erkennungssequenz für eine Endoprotease kloniert, und außerdem wird am N- oder C-Terminus eine weitere Erkennungssequenz für die gleiche oder eine weitere Endoprotease, flankiert von einem Peptidanhängsel für die Affinitätsreinigung, in das Polypeptid kloniert. Das einkettig exprimierte Protein/Polypeptid wird dann durch Behandlung mit der oder den entsprechenden Endoproteasen gleichzeitig oder nacheinander durch Spaltung im Loop-Bereich zwischen L und H_{N} aktiviert und vom Peptidanhängsel befreit.

Abgesehen von den Kosten für den Einsatz solcher Endoproteasen und die damit verbundenen zusätzlichen Arbeitsschritte ist ihre Verwendung bei pharmazeutischen Präparationen (z.B. bei Verwendung von rekombinanten Botulinum-Toxinen oder deren Derivaten) aus arzneimittelrechtlichen (regulatorischen) Gründen höchst problematisch. Zum einen muss die Reinheit der eingesetzten Endoprotease experimentell nachgewiesen sein, zum anderen ist ihre vollständige Abreicherung und insbesondere die Virusfreiheit der Präparation im weiteren Verlauf des Reinigungsprotokolls genauestens zu dokumentieren, was in der Regel einen enormen analytischen Aufwand bedeutet. Da zukünftig auch Botulinum-Toxine z.B. mit verbesserten Eigenschaften oder veränderten Zellspezifitäten auf rekombinantem Wege produziert werden sollen, besteht ein hoher Bedarf für ein Expressionsverfahren, das die Bereitstellung der oben genannten rekombinanten Proteine/Polypeptide, die ihre normale biologische/biochemische Aktivität als zweikettige Proteine/Polypeptide ausüben, mittels der rekombinanten DNA-Technologie aber als inaktive einkettige Proteine/Polypeptide erhalten werden, insbesondere die Bereitstellung dier Botulinum-Toxine oder deren Derivate, als zweikettige, disulfidverbrückte und somit biologisch aktive Polypeptide/Proteine ermöglicht, ohne dass Endoproteasen eingesetzt werden müssen.

Die in den folgenden Abschnitten näher ausgeführte Erfindung stellt also im weitesten Sinn Verfahren bereit, mit denen Proteine wie clostridiale Neurotoxine sowie deren Fragmente und Derivate rekombinant in *E. coli*-Wirtszellen exprimiert und in ihrer zweikettigen, disulfidverbrückten und somit biologisch aktiven Form isoliert werden können, ohne dass es zu ihrer Aktivierung einer zugesetzten Endoprotease bedarf.

In einer ersten bevorzugten Ausführungsform der Erfindung ist die Aminosäure-Sequenz des Loop-Bereichs von BoNT(A) zwischen den Cystein-Resten 430 und 454 (siehe Fig. 3-1 bis 3-5) dahingehend verändert, dass das exprimierte Toxin, oder dessen Fragmente/Derivate, im Lysat der *E. coli*-Wirtszellen bereits als zweikettige Polypeptide vorliegen. Die beiden Ketten sind dabei unter Beteiligung der Cystein-Reste 430 und 454 über eine Disulfidbrücke kovalent miteinander verbunden. In einer besonders bevorzugten Ausführungsform der Erfindung kann, wie in Fig. 3 erläutert, das Pentapeptid Asp₄₄₃ - Asn₄₄₇ (DKGYN) durch Val-Pro-Arg-Gly-Ser (VPRGS) ersetzt werden. In weiteren bevorzugten Ausführungsformen der Erfindung kann das Pentapeptid Asp₄₄₃ - Asn₄₄₇ (DKGYN) auch durch Val-Pro-Tyr-Gly-Ser (VPYGS), Val-Pro-His-Gly-Ser (VPHGS) oder Val-Pro-Gln-Gly-Ser (VPQGS) ersetzt werden. Es gilt auch hier, dass nicht nur der zentrale Aminosäure-Rest eine beliebige, natürlich vorkommende Aminosäure sein kann, sondern auch, dass die vier anderen Aminosäure-Reste ebenfalls ausgetauscht sein können, wie weiter oben ausführlich erläutert (bei Austausch von mindestens einem dieser Reste liegt eine Variante der PRS-Sequenz im Sinne der Erfindung vor). Weiterhin gilt für diese wie alle weiteren bevorzugten Ausführungsformen, die nachfolgend beschrieben werden, dass es zusätzlich bevorzugt ist, wenn die Loop-Sequenz N-terminal zur PRS im Abstand von 1 bis 20 Aminosäuren einen basischen Aminosäure-Rest, speziell einen Lysin- oder Arginin-Rest, aufweist.

Es ist dem Fachmann ohne weiteres ersichtlich, dass weitere Austausche einzelner oder mehrerer Aminosäure-Reste oder die Insertion bzw. Deletion weiterer Aminosäure-Reste im Bereich des oben charakterisierten Loops von BoNT(A) ebenfalls zu dem Ergebnis führen, dass das exprimierte erfindungsgemäße Toxin bzw. die davon abgeleiteten Fragmente/Derivate im Lysat als zweikettige Polypeptide vorliegen. Diese möglichen Varianten sind ebenfalls Gegenstand der Erfindung.

Es ist dem Fachmann aber genauso ohne weiteres ersichtlich, dass das im Wildtyp des BoNT(A) vorliegende Pentapeptid Asp₄₄₃ - Asn₄₄₇ (DKGYN) durch ein Hexapeptid, durch ein Heptapeptid, durch ein Oktapeptid, etc. ersetzt werden kann, solange im exprimierten und einkettig translatierten Polypeptid/Protein die PRS-Pentapeptid-Sequenz oder eine ihrer denkbaren Varianten im Loop-Bereich vorliegt. Wie schon weiter oben festgestellt ist es bevorzugt, wenn N-terminal vom Pentapeptid ein basischer Aminosäure-Rest (bevorzugt Lysin) vorhanden ist.

Der Fachmann wird zudem erkennen, dass es sich bei der bevorzugten Ausführungsform des Pentapeptids (Val-Pro-Arg-Gly-Ser) der PRS um einen Teil einer möglichen Erkennungssequenz für die Protease Thrombin handelt, die in der Blutgerinnungskaskade eine wichtige Rolle spielt und eine hohe Sequenzspezifität aufweist. Es wird ausdrücklich darauf hingewiesen, dass 1. weder bei dem Botulinum-Neurotoxin Typ A noch bei anderen Polypeptiden eine Spaltung durch Thrombin erforderlich ist, um die gewünschte zweikettige, disulfidverbrückte Form zu erhalten, und dass 2. die Thrombin-Erkennungssequenz als solche, das heißt in ihrer unveränderten Form, für die Spaltung durch die Protease-Aktivität des *E. coli*-Lysats zwar förderlich, aber keineswegs notwendig ist. Ausführungsformen der in die entsprechenden Polypeptide (besser: in ihre Loops) eingefügten bzw. dort erzeugten PRS-Pentapeptid-Sequenzen, die den Arginin-Rest, an dessen C-Terminus Thrombin spalten kann, nicht enthalten (sondern stattdessen eine andere der natürlich vorkommenden Aminosäuren), führen, wie oben bereits erwähnt, ebenfalls zur Spaltung im Loop. Die Spaltung erfolgt dabei bevorzugterweise an einem Lysin-Rest des Loops N-terminal vom Pentapeptid, wie weiter oben bereits erläutert (siehe auch Beispiel 2; Fig. 3).

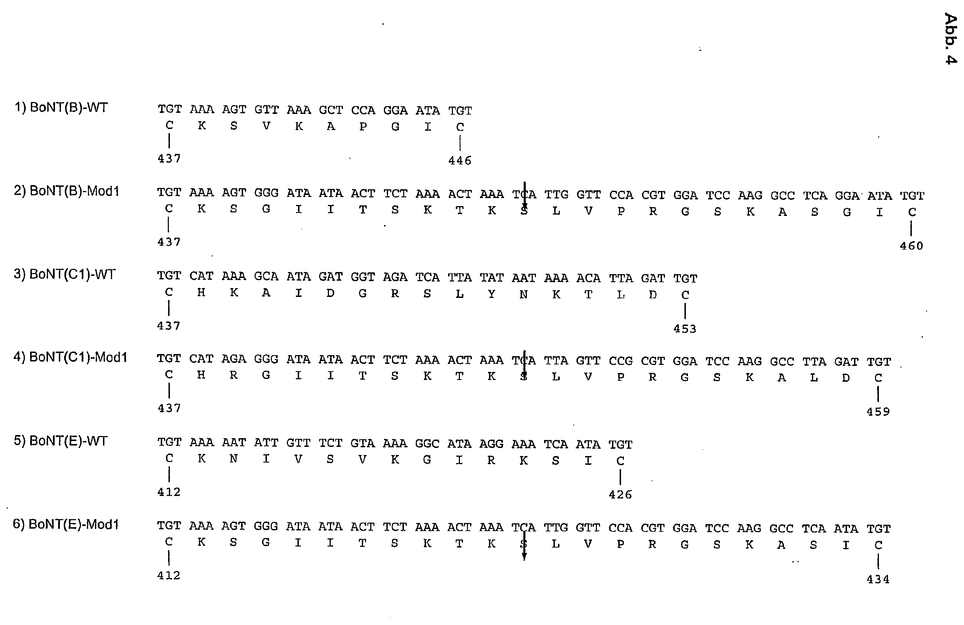

Da auch andere Serotypen der Botulinum-Toxine wie BoNT(B) und BoNT(C1) als langwirkende und BoNT(E) als kurzwirkendes Neurotoxin, und auch ganz andere Polypeptide/Proteine, die rekombinant einkettig erzeugt werden, aber nur zweikettig biologische Aktivität entfalten, therapeutisch nutzbar sind, wäre es wünschenswert, dass diese Neurotoxine sowie Fragmente oder Derivate davon (und auch die anderen Polypeptide/Proteine) ebenfalls als zweikettige, disulfidverbrückte Polypeptide/Proteine aus *E. coli*-Lysaten gewonnen werden können. Insbesondere im Falle von BoNT(B) brächte eine vollständige Spaltung des rekombinanten Toxins im *E. coli*-Lysat zum zweikettigen Polypeptid/Protein einen deutlichen Vorteil zum in *Clostridium botulinum* sekretierten, nativen Neurotoxin, das in der Regel zu mindestens 40 % als einkettiges und damit inaktives Polypeptid vorliegt und von der aktiven, zweikettigen Form nicht abgetrennt werden kann. Auffällig ist, dass die Loop-Bereiche der Neurotoxine der Serotypen B, C1 und E zwischen den an der Disulfidbrücke beteiligten Cystein-Resten gegenüber dem Loop von BoNT(A) deutlich kürzer sind (Fig. 3 und 4). Während es bei BoNT(A) 23 Aminosäure-Reste (Val₄₃₁ - Leu₄₅₃) sind, findet man bei BoNT(B) lediglich 8 (Lys₄₃₈ - Ile₄₄₅), bei BoNT(C1) 15 (His₄₃₈ - Asp₄₅₂) und bei BoNT(E) 13 Aminosäure-Reste (Lys₄₁₃ - Ile₄₂₅) in dieser Region. Trotzdem zeigte sich mit der Ausnahme von BoNT(B), dass diese vergleichsweise verkürzten Regionen ausreichend lang sind, um die Spaltung der Kette und die Ausbildung von Disulfidbrücken zu erlauben, wenn sie die erfindungsgemäße PRS-Sequenz aufwiesen. Obwohl auch das BoNT(B) bei einem Austausch eines Pentapeptids im Loop durch eine PRS-Pentapetid-Sequenz (damit Gesamtlänge der Loop-Sequenz nur acht Aminosäure-Reste) im Sinne der Erfindung in zwei Ketten (leicht und schwer) gespalten wurde, wurden doch bessere Ergebnisse erzielt, das heißt, ist es erfindungsgemäß bevorzugt, einen Loop von wenigstens 9, mindestens 15, mindestens 20 oder sogar mindestens 22 Aminosäure-Resten zu haben. Eine der letztgenannten Ausführungsformen, bei der der Loop 22 Aminosäure-Reste aufweist, ist beispielhaft zu entnehmen den Sequenzen in Fig. 4-1 und 4-2 bzw. einem Vergleich zwischen diesen beiden.

Es wurde daher auch experimentell bestätigt, dass ein Austausch der Loop-Bereiche in den Subtypen B, C1, etc., oder wesentlicher Teile davon, gegen den Loop-Bereich von BoNT(A), oder wesentliche Teile davon, bevorzugt sein würde, was die Spaltung der Neurotoxine zu disulfidverbrückten, zweikettigen Polypeptiden/Proteinen betrifft, insbesondere wenn durch diese Maßnahme der Loop auf mindestens 9, besser 15 Reste verlängert und/oder N-terminal der PRS ein basischer Aminosäure-Rest (z.B. und bevorzugt ein Lys-Rest) eingeführt werden konnte (sofern zuvor noch kein N-terminaler basischer bzw. Lys-Rest vorlag). Besonders bevorzugt waren Änderungen, wie sie in Fig. 4 dargestellt sind (wobei die PRS-Sequenzen in Fig. 4 VPRGS sind, gleichfalls, und genauso bevorzugt sind aber auch die Sequenzen VPYGS, VPHGS, VPQGS, VPKGS, VPIGS und VPAGS).

In weiteren Ausführungsformen der Erfindung sind die Aminosäure-Sequenzen und die hierfür codierenden Genabschnitte der Loop-Bereiche in den Botulinum-Toxinen der Serotypen B, C1, D, E, F und G sowie von Tetanus-Toxin zwischen den an der Disulfidbrücke zwischen L und H_{N} beteiligten Cystein-Resten dahingehend verändert, dass die exprimierten Toxine oder die von ihnen abgeleiteten Fragmente/Derivate im Lysat der *E. coli*-Wirtszellen bereits als zweikettige Polypeptide vorliegen, in denen die beiden Ketten über eine Disulfidbrücke kovalent verbunden sind (entsprechendes gilt auch für beliebige andere Polypeptide/Proteine, die rekombinant einkettig erzeugt werden, aber nur zweikettig biologische Aktivität entfalten). In bevorzugten Ausfuhrungsformen der Erfindung können die kompletten Loop-Bereiche (oder Teile dieser) der Neurotoxine oder der von ihnen abgeleiteten Toxin-Fragmente/Derivate gegen den kompletten Loop-Bereich von BoNT(A), wie er in Fig. 3 charakterisiert wird, oder Teilen des Loop-Bereichs von BoNT(A) ausgetauscht werden, wobei das Pentapeptid Asp₄₄₃ - Asn₄₄₇ vorzugsweise z.B. durch Val-Pro-Arg-Gly-Ser (VPRGS) ersetzt ist. In weiteren bevorzugten Ausführungsformen der Erfindung kann das Pentapeptid Asp₄₄₃ - Asn₄₄₇ auch durch Val-Pro-Tyr-Gly-Ser, Val-Pro-His-Gly-Ser oder Val-Pro-Gln-Gly-Ser ersetzt werden. In besonders bevorzugten Ausführungsformen der Erfindung können die Loop-Bereiche oder Teile der Loop-Bereiche der genannten Neurotoxine und der von ihnen abgeleiteten Fragmente/Derivate durch das Oligopeptid Arg/Ser-Gly-Ile-Ile-Thr-Ser-Lys-Thr-Lys-Ser-Leu-Val-Pro-Arg-Gly-Ser-Lys-Ala (18mer: R/SGIITSKT KSLVPRGSKA) ersetzt werden. Weitere Austausche, Insertionen oder Deletionen einzelner oder mehrerer Aminosäure-Reste im Bereich der oben charakterisierten Loop-Sequenz, wie sie z.B. in Fig. 4 ersichtlich sind, und die ebenfalls dazu führen, dass das exprimierte Neurotoxin bzw. dessen Fragment/Derivat nach der Expression in *E. coli* (z.B. in *E. coli*-K12-Wirtszellen oder deren Derivaten) als disulfidverbrücktes, zweikettiges Polypeptid/Protein vorliegt, sind ausdrücklich Bestandteil dieser Erfindung (entsprechendes gilt auch für beliebige andere Polypeptide/Proteine, die rekombinant einkettig erzeugt werden, aber nur zweikettig biologische Aktivität entfalten).

Wie schon wiederholt erläutert, lassen sich mit dem erfindungsgemäßen Verfahren gemäß einer weiteren Ausführungsform der Erfindung auch Fusions- bzw. Hybridproteine herstellen, die beispielsweise die folgenden Komponenten A, B und C aufweisen:
- eine Effektor-Domäne, die durch ihre enzymatische Aktivität z.B. imstande ist, die Sekretion in Zielzellen zu hemmen oder diese abzutöten (A);
- eine Loop-Sequenz, die erfindungsgemäß, wie oben beschrieben, modifiziert ist und die oben definierte PRS-Pentapeptid-Sequenz VPXGS aufweist (z.B. eine der in Fig. 3 dargestellten, modifizierten Loop-Sequenzen von BoNT(A) oder Varianten davon) und der N- und/oder C-terminal ein Cystein-Rest angefügt sein kann (B), sowie
- eine Zellbindungsdomäne, die dem Fusions-/Hybridprotein eine Zellspezifität verleiht (C).

Die Komponente B (Loop-Sequenz) kann aber in beiden unmittelbar zuvor genannten Ausführungsformenbevorzugterweise ebenso (i) eine der in Fig. 4 dargestellten modifizierten Loop-Sequenzen, (ii) jede insofern von diesen abgeleitete Sequenz, als der zentrale Rest der PRS der Rest einer jeden natürlich vorkommenden Aminosäure sein kann, oder (iii) eine Variante (Definition von Variante siehe oben) von (i) oder (ii) sein. In Fig.4 wurden die jeweiligen Loop-Sequenzen von BoNT(B), BoNT(C1) oder BoNT(E) bis auf ein oder zwei N-terminale und die beiden C-terminalen Aminosäure-Reste deletiert und die deletierten Aminosäure-Reste durch das 17-mer GIITSKTKSLVPRGSKA (Fig- 4-2 und 4-6) oder das 18-mer RGIITSKTKSLVPRGSK A (Fig. 4-4) aus der modifizierten Loop-Sequenz von BoNT(A) ersetzt.

Zusätzlich zu den oben genannten Komponenten A, B und C können die Fusions-/Hybridproteine eine Translokationsdomäne aufweisen (die sich im Fall der Botulinum-Neurotoxine zwischen der Loop-Sequenz und der Zellbindungsdomäne befindet). Diese zusätzliche Domäne ist bei der Einschleusung der Effektor-Domäne in das Zytoplasma der Zielzelle behilflich. Die Expression derartiger Fusionsproteine in *E. coli* (z.B. *E. coli* K12 oder Abkömmlingen davon) führt zu zweikettigen Polypeptiden/Proteinen, in denen eine Domäne auf der einen Kette und die beiden anderen Domänen auf der zweiten Kette sitzen (im Fall der Botulinum-Toxine ist die Effektor-Domäne auf der leichten Kette über eine Disulfidbrücke mit den beiden anderen Domänen auf der schweren Kette kovalent verbunden.

Bei diesen erfmdungsgemäßen Fusions- oder Hybridproteinen kann es sich um sogenannte Immunotoxine handeln, die insbesondere in der Tumortherapie Anwendung finden. Dabei wird einer Toxin-Domäne durch Anfügen einer Zellbindungsdomäne eine Spezifität für einen bestimmten Zelltyp, in der Regel eine Tumorzelle, verliehen. Als Toxin-Domänen kommen vor allem die enzymatischen Domänen von Diphtheria-Toxin, Pseudomonas-Exotoxin und Ricin zum Einsatz. Diese Toxine gehören zu den zweikettigen AB-Toxinen, in denen die A-Kette, die die enzymatische Aktivität enthält, durch eine Disulfidbrücke kovalent mit der B-Kette verbunden ist, welche die Translokations- und Zellbindungsaktivität vereint. Es sind aber auch andere Toxine oder Toxin-Fragmente in Immunotoxinen denkbar, sofern sie die gewünschte Wirkung (z.B. das Abtöten von Tumorzellen) in den Zielzellen entfalten. Während die erste Generation von Immunotoxinen durch chemische Kopplung einer Toxin-Domäne wie z.B. die A-Kette von Ricin mit einem monoklonalen Antikörper hergestellt wurde, werden die Immunotoxine der zweiten Generation rekombinant als Fab-Toxine, einzelkettige Fv-Toxine (scFv-Toxine) oder Disulfid-stabilisierte Fv-Toxine (dsFv-Toxine), aber auch als Fusionsproteine mit Wachstumsfaktoren oder Zytokinen vorwiegend in *E. coli* exprimiert (Reiter, 2001). In kommenden Generationen von Immunotoxinen kann die Zellspezifität auch von modifizierten und nach hochaffiner Bindung an z.B. ein tumorspezifisches Oberflächen-protein selektierten Polypeptiden z.B. aus den Proteinfamilien der Affiline, Ankyrin Repeat Proteine oder Anticaline verliehen werden.

In allen denkbaren Varianten der Immunotoxine muss gewährleistet sein, dass die enzymatische Toxin-Domäne in das Zytoplasma der Zielzelle gelangt, um dort die toxische Wirkung entfalten zu können. Da die Immunotoxine in *E.* c*oli* als einkettige Polypeptide exprimiert werden, sind eine proteolytische Spaltung sowie eine Reduktion einer Disulfidbrücke notwendig, um die enzymatische Toxin-Domäne von der Translokationseinheit und der Zellbindungsdomäne kettenmäßig abzutrennen. Im Falle des rekombinanten Diphtheria-Toxin-Fragments und des rekombinanten Pseudomonas-Exotoxin-Fragments erfolgt eine Spaltung nach Internalisierung im endosomalen Kompartiment der Zielzelle durch eine zelluläre Protease wie Furin (Williams et al., 1990). Ricin hingegen besitzt keine derartige Prozessierungsstelle und bedarf daher einer künstlich eingefügten Protease-Erkennungssequenz, um bereits als ein zweikettiges, disulfidverbrücktes Immunotoxin appliziert werden zu können. Aber auch bei den Immunotoxinen, die auf Diphtheria-Toxin und Pseudomonas-Exotoxin beruhen, wird nur ein geringer Teil der internalisierten Fusionsproteine gespalten, so dass auch nur ein ebenso geringer Teil der enzymatischen Domänen in das Zytoplasma gelangen kann (Ogata et al., 1990). Die nachfolgend vorgestellten bevorzugten Ausführungsformen der Erfindung beschreiben Verfahren und Konstrukte, wobei mit den Verfahren Varianten von Immunotoxinen, wie sie in den vorherigen Abschnitten beschrieben wurden, rekombinant in *E. coli*-Wirtszellen exprimiert und in ihrer zweikettigen, disulfidverbrückten und somit biologisch (enzymatisch) aktiven Form isoliert werden können, ohne dass es zu ihrer Aktivierung einer zellulären oder einer *in vitro* zugefügten Endoprotease bedarf. Diese Immunotoxine sind in der Lage, die enzymatischen Toxin-Domänen in einer translokationskompetenten Form zur Zielzelle zu bringen, so dass eine Spaltung durch eine zelluläre Protease nicht notwendig ist und wesentlich geringere Dosen des Immunotoxins eingesetzt werden müssen, um die gleichen gewünschten zelltoxischen Effekte zu erreichen.

Eine weitere bevorzugte Ausführungsform der Erfindung umfasst demgemäß weiter ein Fusions- bzw. Hybridprotein, das die folgenden Komponenten A, B und C aufweist:
- eine Toxin-Domäne oder deren Fragment/Derivat (A),
- eine Loop-Sequenz, die erfindungsgemäß, wie oben beschrieben, modifiziert ist und die oben definierte PRS-Pentapeptid-Sequenz VPXGS aufweist (z.B. eine der in Fig. 3 dargestellten, modifizierten Loop-Sequenzen von BoNT(A) oder Varianten davon) und der N- und/oder C-terminal ein Cystein-Rest angefügt sein kann (B), sowie
- eine Zellbindungsdomäne, die einem Vertreter der Proteinfamilien der monoklonalen Antikörper, deren Fragmenten, der Affiline, der Ankyrin Repeat Proteine, der Anticaline, der Wachstumsfaktoren (z.B. TGF-alpha, FGF, VEGF oder IGF-1) oder der Zytokine (z.B. IL2, IL4 oder IL6) entnommen sein kann (C).

Die Komponente B (Loop-Sequenz) kann gemäß dieser letzten bevorzugten Ausführungsform ebenso (i) eine der in Fig. 4 dargestellten modifizierten Loop-Sequenzen, (ii) jede insofern von diesen abgeleitete Sequenz, als der zentrale Rest der PRS der Rest einer jeden natürlich vorkommenden Aminosäure sein kann, oder (iii) eine Variante (Definition von Variante siehe oben) von (i) oder (ii) sein.

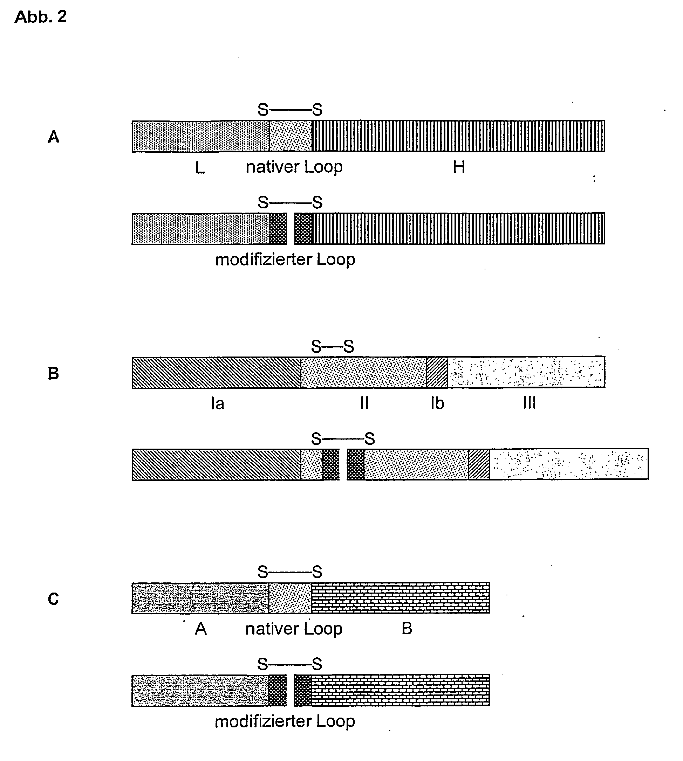

Die Toxin-Domäne kann die A-Kette von Ricin, ein Fragment des Pseudomonas-Exotoxins wie PE40 oder PE38 (Domänen II und III mit bzw. ohne Domäne Ib umfassend; Fig. 2) oder ein Fragment des Diphteria-Toxins sein. Die genannten Effektor- bzw. Toxin- und Zellbindungsdomänen sind als Beispiele zu verstehen. Es sind alle Proteine oder Proteinfragmente von der Erfindung erfasst, die einerseits dem Fusions-/Hybridprotein eine spezifische Bindungsaktivität an ein Oberflächenantigen einer Zielzelle, z.B. einer Tumorzelle, verleihen und andererseits in einer Zielzelle nach Internalisierung eine bestimmte Wirkung, z.B. das Absterben der Zelle, ausüben, wobei die Expression solcher erfindungsgemäßen Fusions-/Hybridproteine in *E. coli* zu zweikettigen Polypeptiden/Proteinen führt, in denen die Toxin-Domäne oder Derivate hiervon durch eine Disulfidbrücke mit der Zellbindungsdomäne kovalent verbunden sind.

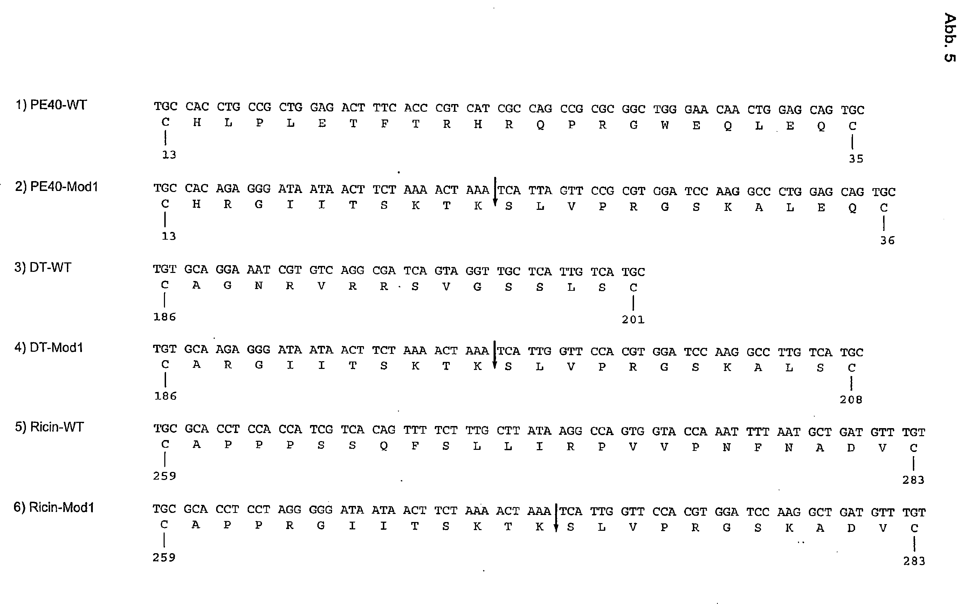

Zur Verbesserung der Effizienz und Spezifität von auf Pseudomonas-Exotoxin basierenden Immunotoxinen wurden in der Vergangenheit verschiedene Ansätze gewählt. So wurde z.B. die Rezeptorbindungsdomäne (Domäne Ia mit den Aminosäure-Resten 1 - 152) gegen Fragmente eines monoklonalen Antikörpers ausgetauscht und gleichzeitig der Loop-Bereich (Fig. 2 und 5) in der Translokationsdomäne (Domäne II) zwischen den Cystein-Resten 13 und 35 (Nummerierung bezogen auf Domäne II) dahingehend verändert, dass dieser nicht mehr für die Spaltung der ubiquitären zellulären Protease Furin empfindlich war, sondern stattdessen für spezielle Proteasen, die nur von bestimmten Tumorzellen in größerem Ausmaß exprimiert und teilweise sekretiert werden (US-Patent 6,426,075). Diese veränderte Protease-Empfindlichkeit sollte dem Immunotoxin zusätzlich zur ausgetauschten Rezeptorbindungsdomäne eine erhöhte Zellspezifität verleihen. Es ist allerdings nicht davon auszugehen, dass es durch andere zelluläre Proteasen zu einer vermehrten Spaltung im Loop und damit verbesserten Translokationseffizienz der enzymatischen Domäne III kommt.

In einem weiteren Ansatz für ein Immunotoxin wurden die Rezeptorbindungsdomäne und der N-terminale Bereich der Translokationsdomäne bis zum Arginin-Rest 27 im Loop-Bereich entfernt. Die notwendige Zellspezifität wurde einem solchen Immunotoxin z.B. durch Einfügen einer V_{H}-Domäne eines monoklonalen Antikörpers, an die über eine Disulfidbrücke die V_{L}-Domäne gebunden war, an die Stelle der Ib-Domäne zwischen den Domänen II und III oder durch Anfügen an den C-Terminus der Domäne III verliehen (US-Patent 5,980,895). In solchen Konstrukten ist eine Aktivierung durch eine Protease nicht mehr notwendig, was einerseits eine deutlich erhöhte Translokationseffizienz bewirken sollte. Allerdings ist zu befürchten, dass andererseits die Translokation durch die sich N- oder C-terminal der enzymatischen Domäne III befindlichen Rezeptorbindungsdomänen wie die V_{H}-Domäne eines monoklonalen Antikörpers oder TGF-alpha behindert wird. Da diese Rezeptorbindungsdomänen nicht von der enzymatischen Domäne getrennt werden, ist auch mit negativen Auswirkungen auf die enzymatische Aktivität und damit die Toxizität in den Zielzellen zu rechnen. Ein relatives Höchstmaß an zytotoxischer Aktivität wird mit einem Pseudomonas-Exotoxin-basierten Immunotoxin erhalten, wenn zum einen der Loop zwischen den Cystein-Resten 13 und 35 bereits bei der Applikation in der gespaltenen, disulfidverbrückten Form vorliegt und eine Aktivierung durch eine zelluläre Protease daher nicht notwendig ist, zum anderen die Rezeptorbindungsdomäne an Stelle der Domäne I des Exotoxins an den N-Terminus der Translokations-Domäne fusioniert ist, so dass sie nach der Reduktion im Zytoplasma von den Toxin-Domänen abgetrennt wird und daher die enzymatische Aktivität der Domäne III nicht beeinträchtigen kann.

Eine besonders bevorzugte Ausführungsform der Erfindung umfasst daher ein Fusions-/Hybridprotein umfassend eine Zellbindungsdomäne, die einem Vertreter der Protein-Familien der monoklonalen Antikörper, deren Fragmenten, der Affiline, der Ankyrin Repeat Proteine, der Anticaline, der Wachstumsfaktoren (z.B. TGF-alpha, FGF, VEGF oder IGF-1) oder der Zytokine (z.B. IL2, IL4 oder IL6) entnommen sein kann, an die C-terminal ein modifiziertes PE38-Fragment, das am extremen C-Terminus das Retentionssignal für das endoplasmatische Retikulum, Lys-Asp-Glu-Leu, oder Varianten davon, tragen kann, fusioniert ist. Die Modifika-tion des PE38-Fragments besteht darin, dass die komplette Loop-Sequenz (oder auch nur ein Teil von dieser) zwischen den Cystein-Resten 13 und 35gegen die PRS-Pentapeptid-Sequenz VPXGS, vorzugsweise gegen die in Fig. 3 dargestellte, modifizierte Loop-Sequenz von BoNT(A) oder Varianten davon, ausgetauscht wurde, insbesondere gegen die Peptidsequenz Arg-Gly-Ile-Ile-Thr-Ser-Lys-Thr-Lys-Ser-Leu-Val-Pro-Arg-Gly-Ser-Lys-Ala (Fig. 5) (bzgl. der Definition der Varianten, s.o.). Vorzugsweise wird auch in dieser Ausführungsform darum Sorge getragen, dass N-terminal zur PRS im Abstand von 1 bis 20 Aminosäure-Resten ein basischer Aminosäure-Rest sitzt, wie in der in Fig. 5 dargestellten Sequenz. Ein entsprechend modifiziertes PE-38-Fragment sowie Fusions-/Hybridproteine, die dieses modifizierte Fragment enthalten, liegen im Lysat der *E. coli*-Wirtszellen (z.B. M15[pREP4]) in der zweikettigen, disulfidverbrückten Form vor

Anders als beim Pseudomonas-Exotoxin befindet sich die enzymatische Domäne des Diphtheria-Toxins, die A-Kette, am N-Terminus. Auf der C-terminalen B-Kette befinden sich die Translokations- und die Rezeptorbindungsomäne. Beide Ketten sind durch eine Loop-Sequenz verbunden, in der am Arginin-Rest 193 bei der Sekretion aus Zellen von *Corynebacterium diphtheriae* durch eine Protease eine proteolytische Spaltung erfolgt (Collier, 2001). Die beiden Ketten bleiben nach der Spaltung durch eine Disulfidbrücke zwischen den Cystein-Resten 186 und 201 kovalent miteinander verknüpft. Insoweit ähnelt das Diphtheria-Toxin in seiner Domänenstruktur den Botulinum-Toxinen und dem Tetanus-Toxin.

Zur Herstellung von rekombinanten Immunotoxinen wurde die Rezeptorbindungsdomäne oder ein Teil davon z.B. gegen VEGF oder IL2 ausgetauscht (Arora et al., 1999; Williams et al., 1990), um dem Fusionsprotein eine neue Zellspezifität zu verleihen. Damit die A-Kette in das Zytoplasma der Zielzellen gelangen kann, muss zum einen die Polypeptidkette des in *E. coli* einkettig exprimierten Immunotoxins im Bereich des Loops zwischen A- und B-Kette gespalten werden, zum anderen die Disulfidbrücke reduziert werden. Während letzteres im Zuge des Translokationsprozesses stattfindet, ist die proteolytische Spaltung durch eine zelluläre Protease unvollständig, so dass nur ein geringer Anteil der A-Ketten in das Zytoplasma freigesetzt werden kann (Williams et al., 1990). Läge das Immunotoxin bereits bei der Applikation in der zweikettigen, disulfidverbrückten Form vor, wäre eine deutliche Effizienzsteigerung zu erwarten, da sämtliche A-Ketten in einer translokationskompetenten Form bereitgestellt würden.

Eine weitere besonders bevorzugte Ausführungsform der Erfindung umfasst daher ein Fusions- bzw. Hybridprotein umfassend eine Zellbindungsdomäne, die einem Vertreter der ProteinFamilien der monoklonalen Antikörper, deren Fragmenten, der Affiline, der Ankyrin Repeat Proteine, der Anticaline, der Wachstumsfaktoren (z.B. TGF-alpha, FGF, VEGF oder IGF-1) oder der Zytokine (z.B. IL2, IL4 oder IL6) entnommen sein kann, an die N-terminal ein modifiziertes Diphtheria-Toxin-Fragment fusioniert ist. Dieses Toxin-Fragment kann die A-Kette sowie mindestens die Translokationsdomäne der B-Kette (Gly₁ - Phe₃₈₉ oder Gly₁ - Asn₄₈₆) umfassen. Die Modifikation des Diphtheria-Toxin-Fragments besteht darin, dass die komplette Loop-Sequenz (oder auch nur ein Teil von dieser) zwischen den Cystein-Resten 186 und 201 gegen die in Fig. 3 dargestellte, modifizierte Loop-Sequenz von BoNT(A) oder Varianten davon ausgetauscht wurde, insbesondere gegen die Peptidsequenz Arg-Gly-Ile-Ile-Thr-Ser-Lys-Thr-Lys-Ser-Leu-Val-Pro-Arg-Gly-Ser-Lys-Ala (Fig. 5) (bzgl. der Definition der Varianten, s.o.). Ein entsprechend modifiziertes Diphtheria-Toxin-Fragment sowie Fusionsproteine, die dieses modifizierte Fragment enthalten, liegen im Lysat der *E. coli*-Wirtszellen wie z.B. M15[pREP4] in der zweikettigen, disulfidverbrückten Form vor.

Ricin-basierte Immunotoxine der ersten Generation wurden durch Verknüpfung der A-Kette des Ricins mit einem monoklonalen Antikörper hergestellt. Dies geschah bislang durch Derivatisierung des Antikörpers mit einem chemischen Linker-Molekül, das mit der Thiol-Funktion des am C-Terminus der A-Kette befindlichen Cystein-Restes eine Disulfidbrücke ausbildete. Derartige Konjugate waren wegen der ungerichteten Derivatisierung des Antikörpers heterogen. Die Wirksamkeit gegen Tumore erwies sich nicht zuletzt wegen der Größe des Konjugats und des Fehlens der auf der B-Kette lokalisierten Translokationsdomäne als nicht ausreichend. Ist auch die B-Kette in der nativen Form Bestandteil des Immunotoxins, ist die Toxizität zwar deutlich heraufgesetzt, es kommt aber aufgrund der Lektin-artigen Zellbindungseigenschaften der B-Kette zur unspezifischen Aufnahme auch in ändere als die gewünschten Zielzellen. Diesem Zielkonflikt wurde mit einer Strategie begegnet, bei der die B-Kette dahingehend modifiziert wurde, dass zwar die Translokationsaktivität erhalten blieb, die Bindungsaffinität für Glykostrukturen an den Zelloberflächen aber deutlich herabgesetzt war (Patentanmeldung WO 89/04839). Rekombinant exprimierte Immunotoxine, die eine solche modifizierte B-Kette enthalten, sind aber einkettig, so dass aufgrund der fehlenden Erkennungssequenz für eine zelluläre Protease im Linker-Peptid zwischen A- und B-Kette eine Freisetzung und Translokation der A-Kette bei der Aufnahme des Immunotoxins in die Zielzelle nicht oder nur sehr ineffizient möglich ist. In US-Patent 6,593,132 werden Modifikationen dieses nativen Linker-Peptids dokumentiert, die Erkennungssequenzen für unterschiedliche, zellspezifische Proteasen darstellen. Ricin-Varianten mit derartigen Modifikationen sollen eine entsprechende Zellspezifität besitzen, sofern die jeweilige Protease, die das modifizierte Linker-Peptid proteolytisch spalten kann, nur in den gewünschten Zielzellen im Vergleich zu anderen Zelltypen in signifikant erhöhten Mengen exprimiert ist. Allerdings ist davon auszugehen, dass die Spaltung nur an einem Bruchteil der internalisierten Toxin-Moleküle erfolgt und somit auch nur eine entsprechend geringe Menge A-Ketten in das Zytoplasma transloziert werden kann. Wünschenswert wären Ricin-basierte, zweikettige Immunotoxine, in denen die A-Kette über eine Disulfidbrücke mit einer modifizierten B-Kette verbunden ist, bei der die Translokationsaktivität erhalten bleibt, die unspezifischen, Lektin-artigen Zellbindungseigenschaften aber unterdrückt sind, und die an ihrem C-Terminus mit einer spezifischen Zellbindungsdomäne fusioniert sind. Derartige Immunotoxine würden Zellspezifität und hohe Toxizität vereinen.

Noch eine weitere besonders bevorzugte Ausführungsform der Erfindung umfasst daher ein Fusionsprotein, das die folgenden Komponenten A, B und C aufweist:
- die A-Kette des Ricin (A),
- eine Loop-Sequenz, die erfindungsgemäß, wie oben beschrieben, modifiziert ist und die oben definierte PRS-Pentapeptid-Sequenz VPXGS aufweist (z.B. eine der in Fig. 3 dargestellten, modifizierten Loop-Sequenzen von BoNT(A) oder Varianten davon) und der N- und/oder C-terminal ein Cystein-Rest angefügt sein kann (B), sowie
- eine Zellbindungsdomäne, die einem Vertreter der Proteinfamilien der monoklonalen Antikörper, deren Fragmenten, der Affiline, der Ankyrin Repeat Proteine, der Anticaline, der Wachstumsfaktoren (z.B. TGF-alpha, FGF, VEGF oder IGF-1) oder der Zytokine (z.B. IL2, IL4 oder IL6) entnommen sein kann (C).

Die Komponente B kann gemäß dieser letzten bevorzugten Ausführungsform ebenso (i) eine der in Fig. 4 dargestellten modifizierten Loop-Sequenzen, (ii) jede insofern von diesen abgeleitete Sequenz, als der zentrale Rest der PRS der Rest einer jeden natürlich vorkommenden Aminosäure sein kann, oder (iii) eine Variante (Definition von Variante siehe oben) von (i) oder (ii) sein.

Insbesondere kann die Loop-Sequenz die Peptidsequenz Ala-Pro-Pro-Arg-Gly-Ile-Ile-Thr-Ser-Lys-Thr-Lys-Ser-Leu-Val-Pro-Arg-Gly-Ser-Lys-Ala-Asp-Val aufweisen (Fig. 5-6), also ein modifizierter Loop der A-Kette des Ricin sein. An die Loop-Sequenz wird vorzugsweise zusätzlich C-terminal ein Cystein-Rest angebracht. In der darin enthaltenen PRS-Sequenz Val-Pro-Arg-Gly-Ser kann Arg jedoch auch jede beliebige andere natürliche Aminosäure Xaa sein. Zu beiden Seiten kann die Loop-Sequenz durch weitere Aminosäure-Reste (z.B Glycin- und Serin-Reste) erweitert sein. Weiterhin kann die A-Kette des Ricins mit der kompletten B-Kette oder Teilen oder Varianten davon über eine Loop-Sequenz verbunden sein, das die Aminosäure-Reste zwischen den Cystein-Resten 259 und 283 der Wildtyp-Sequenz des Pro-Ricins ganz oder teilweise ersetzt und mindestens den Bereich des in Fig. 3 beschriebenen, modifizierten BoNT(A)-Loops oder Varianten davon umfasst. Dabei wird eine Disulfidbrücke von den Cystein-Resten 259 und 283 (bezogen auf die Wildtyp-Sequenz des Pro-Ricins) ausgebildet. An den C-Terminus der B-Kette ist eine Zellbindungsdomäne fusioniert, die den oben genannten Polypeptid-Familien entnommen sein kann. Entsprechende Fusions-/Hybridproteine liegen im Lysat der *E. coli*-Wirtszellen, z.B. von Zellen des Stammes M15[pREP4], in der zweikettigen, disulfidverbrückten Form vor.

Eine weitere Ausführungsform der Erfindung betrifft rekombinante Fusionsproteine, die die folgenden Komponenten A, B und C aufweisen:
- ein Protein oder ein Oligopeptid, das dem Fusionsprotein eine bessere Löslichkeit verleiht, eine höhere Expressionsrate bewirkt und/oder eine Affinitätsreinigung ermöglicht (z.B. Glutathion-S-Transferase (GST), Maltose-Bindungsprotein (MBP), His-Tag, StrepTag, FLAG-Tag) (A),
- eine Loop-Sequenz, die erfindungsgemäß, wie oben beschrieben, modifiziert ist und die oben definierte PRS-Pentapeptid-Sequenz VPXGS aufweist (z.B. eine der in Fig. 3 dargestellten, modifizierten Loop-Sequenzen von BoNT(A) oder Varianten davon) und der N- und/oder C-terminal ein Cystein-Rest angefügt sein kann, sowie
- ein beliebiges Polypeptid (C).

Die Komponente B (Loop-Sequenz) kann gemäß dieser letzten bevorzugten Ausführungsform ebenso (i) eine der in Fig. 4 dargestellten modifizierten Loop-Sequenzen, (ii) jede insofern von diesen abgeleitete Sequenz, als der zentrale Rest der PRS der Rest einer jeden natürlich vorkommenden Aminosäure sein kann, oder (iii) eine Variante (Definition von Variante siehe oben) von (i) oder (ii) sein.

Insbesondere kann der Loop die Peptidsequenz Val-Arg-Gly-Ile-Ile-Thr-Ser-Lys-Thr-Lys-Ser-Leu-Val-Pro-Arg-Gly-Ser-Lys-Ala-Leu-Asn-Asp-Leu aufweisen, wobei Arg im Zentrum der PRS wiederum als Xaa zu verstehen ist. Zu beiden Seiten kann er durch weitere Aminosäure-Reste (z.B Glycin- und Serin-Reste) erweitert sein. Die Expression solcher Fusionsproteine in *E. coli* führt zu zweikettigen Polypeptiden/Proteinen, deren beide Ketten durch eine Disulfidbrücke kovalent verbunden sind und nach erfolgter Reinigung ohne Zusatz einer Protease nach einfacher Reduktion durch thiolhaltige Substanzen (z.B. ß-Mercaptoethanol, DTT oder reduziertes Glutathion) voneinander getrennt werden können. Ein solches Expressionssystem ist besonders geeignet für rekombinante Proteine, denen an einem der beiden Termini ein Cystein-Rest angefügt werden soll, um nach der Reinigung und Abtrennung des Fusionspartners mit der reaktiven Thiolgruppe einen Ansatzpunkt z.B. für Kopplungsreaktionen mit Thiol-reaktiven Linkermolekülen oder Modifikationen mit z.B. Polyethylenglykol zu haben.

Desweiteren werden bereitgestellt alle Nukleinsäuren, die für die in den vorhergehenden Abschnitten beschriebenen erfindungsgemäßen Polypeptide codieren unter Berücksichtigung der verschiedenen Möglichkeiten der Codon-Nutzung. Ferner werden kommerziell erhältliche oder individuell konstruierte Klonierungs- und Expressionsplasmide, die die codierenden DNS-Sequenzen für die jeweiligen erfindungsgemäßen Polypeptide enthalten, sowie geeignete Klonierungs- und Expressionsstämme von *E. coli,* die mit den entsprechenden Expressionsplasmiden transformiert sind und die jeweiligen erfindungsgemäßen Polypeptide in ihrer aktiven, zweikettigen und disulfidverbrückten Form exprimieren können, bereitgestellt. Ein Beispiel für ein solches Expressionssystem ist ein Expressionsplasmid der pQE-Serie in Verbindung mit dem *E. coli*-Wirtsstamm M15[pREP4].

Für den einschlägigen Fachmann, der sich insbesondere mit der Entwicklung von pharmazeutisch zu verwendenden Polypeptiden/Proteinen beschäftigt, sind die Vorteile klar ersichtlich, die damit verbunden sind, dass zur Aktivierung dieser Polypeptide/Proteine keine Endoproteasen zugesetzt werden müssen. Der größte Teil der in den vorherigen Abschnitten beschriebenen erfindungsgemäßen Polypeptide/Proteine sind insbesondere für den pharmazeutischen Einsatz bestimmt. Bestandteil der Erfindung sind somit auch pharmazeutische Präparationen, die eines der erfindungsgemäßen Polypeptide/Proteine oder ein Gemisch der erfindungsgemäßen Polypeptide/Proteine als Wirkstoffkomponente sowie nützliche Zusätze enthalten, die der Präparation eine ausreichende Stabilität verleihen und deren Zusammensetzung der gewünschten Darreichungsform angepasst ist.

Die beigefügten Figuren und Sequenzen aus dem Sequenzprotokoll werden wie folgt beschrieben:

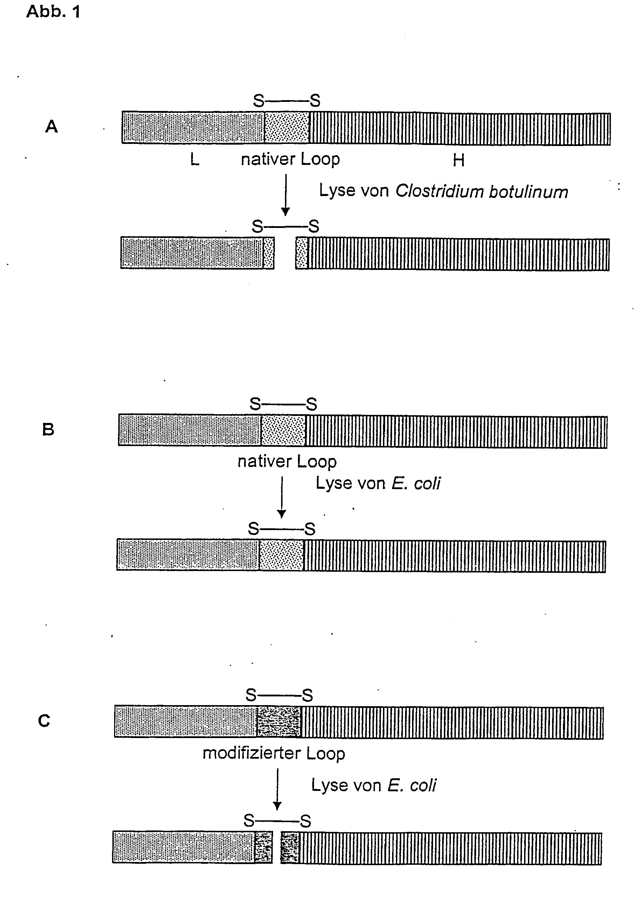

Fig. 1 zeigt eine schematische Darstellung der Freisetzung von Botulinum-Neurotoxin Typ A mit Wildtyp-Loop oder erfindungsgemäß modifiziertem Loop aus *Clostridium botulinum* bzw. *Escherichia coli* K12. A: Bei der Lyse von *Clostridium botulinum*-Zellen wird das Neurotoxin im Loop-Bereich zwischen Leichter (L) und Schwerer Kette (H) durch eine clostridiale Endoprotease gespalten. Beide Ketten sind durch eine Disulfidbrücke miteinander verbunden. B: Nach Expression eines rekombinanten Neurotoxins mit Wildtyp-Loop in *E. coli* und Lyse der Zellen liegt dieses in der einkettigen Form vor. C: Bei der Freisetzung eines rekombinanten Neurotoxins mit erfindungsgemäß modifiziertem Loop aus *E. coli*-Zellen erfolgt eine Spaltung im Loop-Bereich durch eine Endoproteasen.
Fig. 2 zeigt eine schematische Darstellung verschiedener rekombinanter Toxine mit Wildtyp-Loop-Bereichen sowie erfindungsgemäß modifizierten Loop-Bereichen im Vergleich nach ihrer Freisetzung aus *E. coli*-Zellen. A: Botulinum-Neurotoxin; B: Pseudomonas-Exotoxin; C: Diphtheria-Toxin.
Fig. 3 zeigt einen Vergleich des Wildtyp-Loops mit einer Auswahl von erfindungsgemäß modifizierten Loop-Sequenzen von BoNT(A). Dargestellt sind die Nukleotidsequenzen und die abgeleiteten Aminosäure-Sequenzen, die die begrenzenden Cystein-Reste der Leichten und Schweren Kette mit einschließen. Der Pfeil markiert die Spaltstelle für die Endoprotease im *E. coli-Lysat.*
Fig. 4 zeigt einen Vergleich der Wildtyp-Loops mit jeweils einer beispielhaften, erfindungsgemäß modifizierten Loop-Sequenz der Botulinum-Neurotoxine der Serotypen B, C1 und E. Dargestellt sind die Nukleotidsequenzen und die abgeleiteten Aminosäure-Sequenzen, die die begrenzenden Cystein-Reste der Leichten und Schweren Kette mit einschließen. Der Pfeil markiert die Spaltstelle für die Endoprotease im *E. coli*-Lysat.
Fig. 5 zeigt einen Vergleich der Wildtyp-Loops mit jeweils einer beispielhaften, erfindungsgemäß modifizierten Loop-Sequenz von Fragment PE40 des Pseudomonas-Exotoxins, Diphtheria-Toxin (DT) und Ricin. Dargestellt sind die Nukleotidsequenzen und die abgeleiteten Aminosäure-Sequenzen, die die begrenzenden Cystein-Reste mit einschließen. Der Pfeil markiert die Spaltstelle für die Endoprotease im *E. coli*-Lysat.

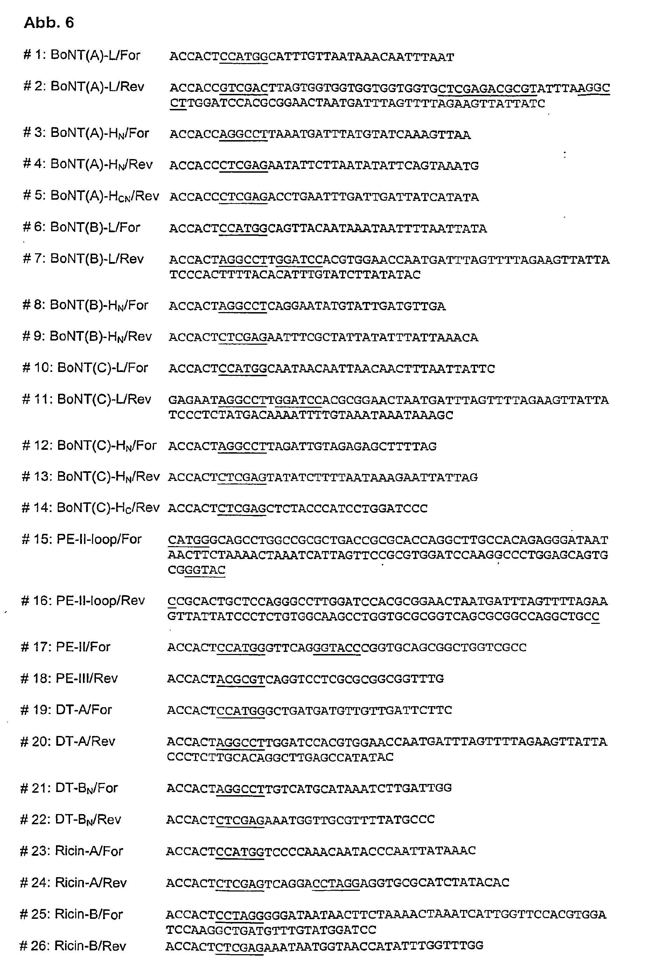

Fig. 6 zeigt eine Zusammenstellung der Oligonukleotide, die bei den Klonierungen der rekombinanten Toxine und Toxin-Fragmente eingesetzt wurden. Erkennungssequenzen für Restriktionsendonukleasen sind unterstrichen.
Fig. 7 zeigt eine Analyse des rekombinanten LH_{N}-Fragmentes von BoNT(A) mit erfindungsgemäß modifizierter Loop-Sequenz im SDS-Polyacrylamidgel. Die Expression des LH_{N}-Fragmentes erfolgte in M15[pREP4]-Zellen, die mit dem Plasmid pQE-BoNT(A)-L_{mod1}H_{N} transformiert waren. Spuren 2 und 5: über Ni-NTA-Agarose gereinigtes LH_{N}-Fragment; Spuren 1 und 4: LH_{N}-Fragment nach Inkubation mit Thrombin; Spur 3: Molekulargewichtsmarker. Probenauftrag unter reduzierenden Bedingungen (Spuren 1 und 2) bzw. nicht-reduzierenden Bedingungen (Spuren 4 und 5).

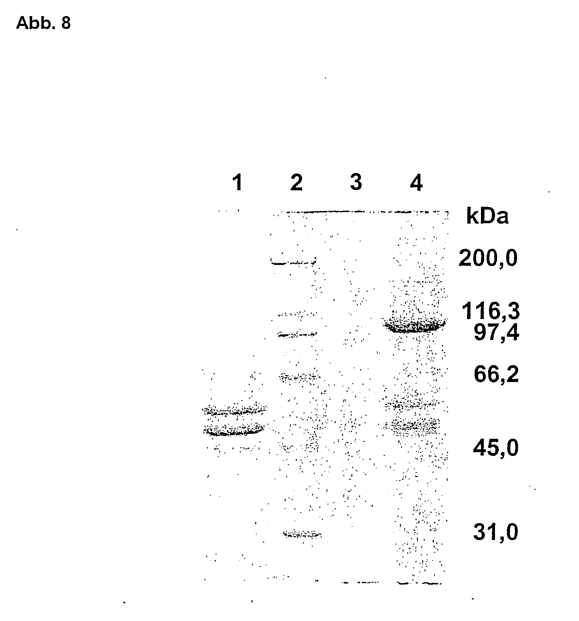

Fig. 8 zeigt eine Analyse des rekombinanten LH_{N}-Fragmentes von BoNT(B) mit erfindungsgemäß modifizierter Loop-Sequenz im SDS-Polyacrylamidgel. Die Expression des LH_{N}-Fragmentes erfolgte in M15[pREP4]-Zellen, die mit dem Plasmid pQE-BoNT(B)-L_{mod1}H_{N} transformiert waren. Spuren 1 und 4: über Ni-NTA-Agarose gereinigtes LH_{N}-Fragment; Spur 2: Molekulargewichtsmarker; Spur 3: kein Auftrag. Probenauftrag unter reduzierenden Bedingungen (Spur 1) bzw. nicht-reduzierenden Bedingungen (Spur 4).

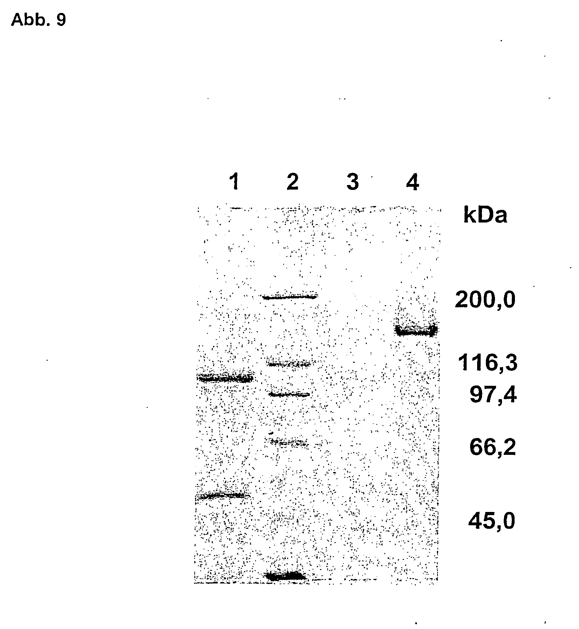

Fig. 9 zeigt eine Analyse von rekombinantem BoNT(C1) mit erfindungsgemäß modifizierter Loop-Sequenz im SDS-Polyacrylamidgel. Die Expression des Toxins erfolgte in M15[pREP4]-Zellen, die mit dem Plasmid pQE-BoNT(C1)-L_{mod1}H_{N}H_{C} transformiert waren. Spuren 1 und 4: über Ni-NTA-Agarose gereinigtes Toxin; Spur 2: Molekulargewichtsmarker; Spur 3: kein Auftrag. Probenauftrag unter reduzierenden Bedingungen (Spur 1) bzw. nicht-reduzierenden Bedingungen (Spur 4).
SEQ ID NO:1 ist ein Beispiel für eine Nukleinsäure (DNA), die für ein rekombinantes Botulinum-Neurotoxin Typ A mit erfindungsgemäß modifizierter Loop-Sequenz und C-terminalem Hexahistidin-Anhängsel (rBoNT(A)-mod1) kodiert.
SEQ ID NO:2 ist ein Beispiel für ein rekombinantes Botulinum-Neurotoxin Typ A mit erfindungsgemäß modifizierter Loop-Sequenz und C-terminalem Hexahistidin-Anhängsel (rBoNT(A)-mod1).
SEQ ID NO:3 ist ein Beispiel für eine Nukleinsäure (DNA), die für ein rekombinantes LH_{N}-Fragment des Botulinum-Neurotoxins Typ A mit erfindungsgemäß modifizierter Loop-Sequenz und C-terminalem Hexahistidin-Anhängsel (rBoNT(A)-L_{mod1}H_{N}) kodiert. Die Sequenz entspricht der SEQ ID NO:1, wobei die Nukleotide 2620 - 3888 deletiert sind.
SEQ ID NO:4 ist ein Beispiel für ein rekombinantes. LH_{N}-Fragment des Botulinum-Neurotoxins Typ A mit erfindungsgemäß modifizierter Loop-Sequenz und C-terminalem Hexahistidin-Anhängsel (rBoNT(A)-L_{mod1}HN). Die Sequenz entspricht der SEQ ID NO:2, wobei die Aminosäurereste 874 - 1296 deletiert sind.
SEQ ID NO:5 ist ein Beispiel für eine Nukleinsäure (DNA), die für ein rekombinantes LH_{N}H_{CN}-Fragment des Botulinum-Neurotoxins Typ A mit erfindungsgemäß modifizierter Loop-Sequenz und C-terminalem Hexahistidin-Anhängsel (rBoNT(A)-L_{mod1}H_{N}H_{CN}) kodiert. Die Sequenz entspricht der SEQ ID NO:1, wobei die Nukleotide 3286 - 3888 deletiert sind.
SEQ ID NO:6 ᵢst ein Beispiel für ein rekombinantes LH_{N}H_{CN}-Fragment des Botulinum-Neurotoxins Typ A mit erfindungsgemäß modifizierter Loop-Sequenz und C-terminalem Hexahistidin-Anhängsel (rBoNT(A)-L_{mod1}H_{N}H_{CN}). Die Sequenz entspricht der SEQ ID NO:2, wobei die Aminosäurereste 109 - 1296 deletiert sind.
SEO ID NO:7 ist ein Beispiel für eine Nukleinsäure (DNA), die für ein rekombinantes Botulinum-Neurotoxin Typ B mit erfindungsgemäß modifizierter Loop-Sequenz und C-terminalem Hexahistidin-Anhängsel (rBoNT(B)-mod1) kodiert.
SEO ID NO:8 ist ein Beispiel für ein rekombinantes Botulinum-Neurotoxin Typ B mit erfindungsgemäß modifizierter Loop-Sequenz und C-terminalem Hexahistidin-Anhängsel (rBoNT(B)-mod1).
SEQ ID NO:9 ist ein Beispiel für eine Nukleinsäure (DNA), die für ein rekombinantes LH_{N}-Fragment des Botulinum-Neurotoxins Typ B mit erfindungsgemäß modifizierter Loop-Sequenz und C-terminalem Hexahistidin-Anhängsel (rBoNT(B)-L_{mod1}H_{N}) kodiert. Die Sequenz entspricht der SEQ ID NO:7, wobei die Nukleotide 2623 - 3915 deletiert sind.
SEO ID NO:10 ist ein Beispiel für ein rekombinantes LH_{N}-Fragment des Botulinum-Neurotoxins Typ B mit erfindungsgemäß modifizierter Loop-Sequenz und C-terminalem Hexahistidin-Anhängsel (rBoNT(B)-L_{mod1}H_{N}). Die Sequenz entspricht der SEQ ID NO:8, wobei die Aminosäurereste 875 - 1305 deletiert sind.
SEO ID NO:11 ist ein Beispiel für eine Nukleinsäure (DNA), die für ein rekombinantes Botulinum-Neurotoxin Typ C1 mit erfindungsgemäß modifizierter Loop-Sequenz und C-terminalem Hexahistidin-Anhängsel (rBoNT(C1)-mod1) kodiert.
SEO ID NO:12 ist ein Beispiel ein rekombinantes Botulinum-Neurotoxin Typ C1 mit erfindungsgemäß modifizierter Loop-Sequenz und C-terminalem Hexahistidin-Anhängsel (rBoNT(C1)-mod1).
SEO ID NO: 13 ist ein Beispiel für eine Nukleinsäure (DNA), die für ein rekombinantes LH_{N}-Fragment des Botulinum-Neurotoxins Typ C1 mit erfindungsgemäß modifizierter Loop-Sequenz und C-terminalem Hexahistidin-Anhängsel (rBoNT(C1)-L_{mod1}H_{N}.) kodiert. Die Sequenz entspricht der SEQ ID NO:11, wobei die Nukleotide 2599 - 3858 deletiert sind.
SEQ ID NO:14 ist ein Beispiel für ein rekombinantes LH_{N}-Fragment des Botulinum-Neurotoxins Typ C1 mit erfindungsgemäß modifizierter Loop-Sequenz und C-terminalem Hexahistidin-Anhängsel (rBoNT(C1)-L_{mod1})H_{N}.). Die Sequenz entspricht der SEQ ID NO:12, wobei die Aminosäurereste 867 - 1286 deletiert sind.
SEQ ID NO:15 ist ein Beispiel für eine Nukleinsäure (DNA), die für ein rekombinantes Botulinum-Neurotoxin Typ E mit erfindungsgemäß modifizierter Loop-Sequenz und C-terminalem Hexahistidin-Anhängsel (rBoNT(E)-modl) kodiert.
SEQ ID NO:16 ist ein Beispiel für ein rekombinantes Botulinum-Neurotoxin Typ E mit erfindungsgemäß modifizierter Loop-Sequenz und C-terminalem Hexahistidin-Anhängsel (rBoNT(E)-mod1).
SEQ ID NO:17 ist ein Beispiel für eine Nukleinsäure (DNA), die für ein rekombinantes, die Domänen II, Ib und III umfassendes 40 kDa-Fragment des Pseudomonas-Exotoxins mit erfindungsgemäß modifizierter Loop-Sequenz und C-terminalem Hexahistidin-Anhängsel (PE40-mod1) kodiert.
SEQ ID NO:18 ist ein Beispiel für ein rekombinantes, die Domänen II, Ib und III umfassendes 40 kDa-Fragment des Pseudomonas-Exotoxins mit erfindungsgemäß modifizierter Loop-Sequenz und C-terminalem Hexahistidin-Anhängsel (PE40-mod1).
SEQ ID NO: 19 ist ein Beispiel für eine Nukleinsäure (DNA), die für ein rekombinantes, die A-Kette und ein N-terminales Fragment der B-Kette umfassendes Fragment des Diphtheria-Toxins mit erfindungsgemäß modifizierter Loop-Sequenz und C-terminalem Hexahistidin-Anhängsel (DT389-mod1) kodiert.
SEO ID NO:20 ist ein Beispiel für ein rekombinantes, die A-Kette und ein N-terminales Fragment der B-Kette umfassendes Fragment des Diphtheria-Toxins mit erfindungsgemäß modifizierter Loop-Sequenz und C-terminalem Hexahistidin-Anhängsel (DT389-mod1).
SEQ ID NO:21 ist ein Beispiel für eine Nukleinsäure (DNA), die für ein rekombinantes Ricin-Toxin mit erfindungsgemäß modifizierter Loop-Sequenz und C-terminalem Hexahistidin-Anhängsel (rRicin-mod1) kodiert.
SEO ID NO:22 ist ein Beispiel für ein rekombinantes Ricin-Toxin mit erfindungsgemäß modifizierter Loop-Sequenz und C-terminalem Hexahistidin-Anhängsel (rRicin-mod1).

### Beispiele

### Beispiel 1: Klonierung und Expression des LH_{N}-Fragments von Botulinum-Neurotoxin Typ A mit modifiziertem Loop

Zur Klonierung der DNS-Sequenzen der Leichten Kette sowie der Translokationsdomäne wurde aus einer Kultur von *Clostridium botulinum* Typ A (Stamm ATCC 3502) chromosomale DNS isoliert. Durch PCR-Amplifikation mit den Primem # 1 und # 2 (Fig. 6) wurde ein für die Leichte Kette von BoNT(A) mit modifizierter Loop-Sequenz und C-terminalem His-Tag codierendes Genfragment erhalten. Das PCR-Amplifikat wurde in das Expressionsplasmid pQE-60 über die Restriktionsschnittstellen für *Nco* I und *Sal* I kloniert, wodurch das Plasmid pQE-BoNT(A)-L_{mod1} resultierte. Durch PCR-Amplifikation mit den Primern # 3 und # 4 (Fig. 6) wurde das für die Translokationsdomäne von BoNT(A) codierende Genfragment generiert. Über die Restriktionsschnittstellen für *Stu* I und *Xho* I wurde es zwischen die Loop-Sequenz und die Sequenz für den His-Tag in pQE-BoNT(A)-L_{mod1} kloniert (Plasmid pQE-BoNT(A)-L_{mod1}H_{N}; Sequenz # 2; Fig. 3, Nr. 2). Der *E. coli*-Expressionsstamm M15[pREP4] (Qiafen) wurde mit dem Plasmid pQE-BoNT(A)-L_{mod1}H_{N} transformiert. Die Expression des modifizierten LH_{N}-Fragments erfolgte durch eine abgestufte Induktion mit 500 µM Endkonzentration IPTG bei 25°C über Nacht. Die Zellen wurden in einem 50 mM Phosphatpuffer bei pH 8.0 mit 300 mM NaCl durch Lysozym- und Ultraschallbehandlung aufgeschlossen. Das zentrifugierte Lysat wurde über eine Ni-NTA-Agarose-Säule chromatographiert. Eine Analyse im SDS-Polyacrylamidgel ergab, dass durch Coomassie unter reduzierenden Bedingungen zwei Banden bei ca. 50 kDa sowie eine Bande bei 100 kDa angefärbt wurden, während unter nicht-reduzierenden Bedingungen nur die Bande bei ca. 100 kDa zu beobachten war (Fig. 7). Damit ist eindeutig gezeigt, dass das LH_{N}-Fragment zu > 75 % als zweikettiges Polypeptid aus den Bakterien freigesetzt wurde, in dem die beiden Ketten durch eine Disulfidbrücke kovalent miteinander verknüpft waren. Die nachträgliche Behandlung mit Thrombin führte zum einen zur Spaltung der einkettigen Form, zum anderen zu einer Verkürzung der Translokationsdomäne im zweikettigen Polypeptid (Fig. 7). Eine zweistündige Inkubation des *E. coli*-Lysats vor der Reinigung des LH_{N}-Fragments führte bei vollständiger Spaltung zum zweikettigen Polypeptid.

Ein entsprechend exprimiertes und gereinigtes LH_{N}-Fragment mit der nativen Loop-Sequenz (Fig. 3, Nr. 1) zeigte im SDS-Polyacrylamidgel sowohl unter nicht-reduzierenden als auch unter reduzierenden Bedingungen eine Bande bei 100 kDa. Das einkettige Polypeptid ließ sich erst durch Spaltung mit Trypsin in das zweikettige, disulfidverbrückte LH_{N}-Fragment umwandeln.

### Beispiel 2: Klonierung und Expression des LH_{N}H_{CN}Fragments von Botulinum-Neurotoxin Typ A mit modifiziertem Loop und Charakterisierung der Spaltstelle

Das H_{N}H_{CN}-Fragment (Translokationsdomäne mit N-terminaler Hälfte der Rezeptorbindungsdomäne von BoNT(A)) wurde durch PCR-Amplifikation mit den Primem # 3 und # 5 (Fig. 6) generiert und über die Restriktionsschnittstellen für *Stu* I und *Xho* I in das Plasmid pQE-BoNT(A)-L_{mod1} kloniert (Plasmid pQE-BoNT(A)-L_{mod1}H_{N}H_{CN}; Sequenz # 3). Expression und Reinigung erfolgten nach dem in Beispiel 1 beschriebenen Schema. Eine Analyse im SDS-Polyacrylamidgel ergab neben einer schwächeren Bande, die dem einkettigen Polypeptid entsprach, und weiteren nicht definierten Banden eine Bande bei 50 kDa sowie eine bei 75 kDa, die der Leichten Kette bzw. dem H_{N}H_{CN}-Fragment entsprachen: Die N-terminale Sequenzierung der ersten vier Aminosäure-Reste des H_{N}H_{CN}-Fragments ergab die Sequenz Ser-Leu-Val-Pro. Die Spaltung durch die Proteaseaktivität im *E. coli*-Lysat erfolgte somit hinter Lys₄₄₀ und damit N-terminal vom in den Loop eingefügten Pentapeptid Val-Pro-Arg-Gly-Ser.

### Beispiel 3: Klonierung und Expression des LH_{N}-Fragments von Botulinum-Neurotoxin Typ B mit modifiziertem Loop

Zur Klonierung der DNS-Sequenzen der Leichten Kette sowie der Translokationsdomäne wurde aus einer Kultur von *Clostridium botulinum* Typ B (Stamm Okra) chromosomale DNS isoliert. Durch PCR-Amplifikation mit den Primem # 6 und # 7 (Fig. 6) wurde ein für die Leichte Kette von BoNT(B) mit modifizierter Loop-Sequenz von BoNT(A) codierendes Genfragment erzeugt. Mit den Primem # 8 und # 9 (Fig. 6) wurde das für die Translokationsdomäne von BoNT(B) codierende Genfragment generiert. Die Klonierung in das Expressionsplasmid pQE-60 erfolgte zunächst durch Austausch des BoNT(A)-L-Genfragments in pQE-BoNT(A)-L_{mod1} gegen das BoNT(B)-L_{mod1}-Amplifikat über die Restrikionsschnittstellen für *Nco* I und *Stu* I. Anschließend wurde dahinter das BoNT(B)-H_{N}-Amplifikat über die Restriktionsschnittstellen für *Stu* I und *Xho* I kloniert, wodurch das Plasmid pQE-BoNT(B)-L_{mod1}H_{N} resultierte (Sequenz # 5). Die Expression im Wirtsstamm M15[pREP4] und die Reinigung des LH_{N}-Fragments erfolgten analog zu Beispiel 1. Eine Analyse im SDS-Polyacrylamidgel ergab, dass durch Coomassie unter reduzierenden Bedingungen zwei Banden bei ca. 50 kDa und 55 kDa angefärbt wurden, während unter nicht-reduzierenden Bedingungen eine Bande bei ca. 105 kDa zu beobachten war (Fig. 8). Damit ist eindeutig gezeigt, dass das LH_{N}-Fragment weitgehend als zweikettiges Polypeptid aus den Bakterien freigesetzt wurde, in dem die beiden Ketten zu > 80 % durch eine Disulfidbrücke kovalent miteinander verknüpft waren.

### Beispiel 4: Klonierung und Expression des LH_{N}-Fragments von Botulinum-Neurotoxin Typ C1 mit modifiziertem Loop und Charakterisierung der Spaltstelle

Zur Klonierung der DNS-Sequenzen der Leichten Kette sowie der Translokationsdomäne wurde aus einer Kultur von *Clostridium botulinum* Typ C1 (Stamm C205) chromosomale DNS präpariert. Durch PCR-Amplifikation mit den Primem # 10 und # 11 (Fig. 6) wurde ein für die Leichte Kette von BoNT(C1) mit modifizierter Loop-Sequenz von BoNT(A) codierendes Genfragment erzeugt. Mit den Primem # 12 und # 13 (Fig. 6) wurde das für die Translolcationsdomäne von BoNT(C1) codierende Genfragment generiert. Die Klonierung in das Expressionsplasmid pQE-60 erfolgte zunächst durch Austausch des BoNT(A)-L-Genfragments in pQE-BoNT(A)-L_{mod1} gegen das BoNT(C1)-L_{mod1}-Amplifikat über die Restriktionsschnittstellen für *Nco* I und *Stu* I. Anschließend wurde dahinter das BoNT(C1)-H_{N}-Amplifikat über die Restriktionsschnittstellen für *Stu* I und *Xho* I kloniert, wodurch das Plasmid pQE-BoNT(C1)-L_{mod1}H_{N} resultierte (Sequenz # 7). Die Expression im Wirtsstamm M15[pREP4] und die Reinigung des LH_{N}-Fragments erfolgten analog zu Beispiel 1. Eine Analyse im SDS-Polyacrylamidgel ergab, dass unter reduzierenden Bedingungen durch Coomassie zwei Banden bei ca. 50 kDa und 55 kDa angefärbt wurden, während unter nicht-reduzierenden Bedingungen eine Bande bei ca. 105 kDa zu beobachten war. Damit ist eindeutig gezeigt, dass das LH_{N}-Fragment zu > 90 % als zweikettiges Polypeptid aus den Bakterien freigesetzt wurde, in dem die beiden Ketten durch eine Disulfidbrücke kovalent miteinander verknüpft waren. Die N-terminale Sequenzierung der ersten vier Aminosäure-Reste des H_{N}-Fragments ergab die Sequenz Ser-Leu-Val-Pro. Die Spaltung durch die Proteaseaktivität im *E. coli*-Lysat erfolgte somit hinter Lys₄₄₇ und damit N-terminal vom in den BoNT(A)-Loop eingefügten Pentapeptid Val-Pro-Arg-Gly-Ser. Durch gerichtete Mutagenese wurde der Arginin-Rest des eingefügten Pentapeptids gegen Histidin, Tyrosin bzw. Glutamin ausgetauscht. Die mutagenisierten, auf gleiche Weise exprimierten LH_{N}-Fragmente lagen nach zweistündiger Inkubation des *E. coli*-Zelllysats zu > 90 % in der zweikettigen, disulfidverbrückten Form vor, wobei die Effizienz der Spaltung geringfügig geringer war als bei dem LH_{N}-Fragment, das den mit dem Pentapeptid Val-Pro-Arg-Gly-Ser modifizierten BoNT(A)-Loop enthält.

### Beispiel 5: Klonierung und Expression eines rekombinanten Botulinum-Neurotoxins Typ C1 mit modifiziertem Loop

Unter Verwendung von chromosomaler DNS aus dem Stamm *Clostridium botulinum* C205 wurde mit den Primem # 12 und # 14 (Fig. 6) das für die Schwere Kette codierende Genfragment mittels PCR amplifiziert. Über die Restriktionsschnittstellen für *Stu* I und *Xho* I wurde es zwischen den für die Leichte Kette codierenden Sequenzabschnitt und die Sequenz für den His-Tag in das Plasmid BoNT(C1)-L_{mod1}H_{N} kloniert (Plasmid pQE-BoNT(C1)-L_{mod1}H_{N}H_{C}; Sequenz # 6). Der *E. coli*-Expressionsstamm M15[pREP4] (Qiagen) wurde mit dem entsprechenden Expressionsplasmid transformiert. Die Expression im Wirtsstamm M15[pREP4] und die Reinigung erfolgten analog zu Beispiel 1. Eine Analyse im SDS-Polyacrylamidgel ergab, dass durch Coomassie unter reduzierenden Bedingungen zwei Banden bei ca. 50 kDa und 105 kDa angefärbt wurden, während unter nicht-reduzierenden Bedingungen eine Bande bei ca. 155 kDa zu beobachten war (Fig. 9). Damit ist eindeutig gezeigt, dass das rekombinante Neurotoxin zu > 90 % als zweikettiges Polypeptid aus den Bakterien freigesetzt wurde, in dem die beiden Ketten durch eine Disulfidbrücke kovalent miteinander verknüpft waren. Ein Aktivitätstest im Hemidiaphragma-Assay ergab eine vergleichbar hohe Toxizität wie die des nativen, aus *Clostridium botulinum* isolierten Neurotoxins Typ C1. Die Modifikation des Loop-Bereichs zwischen der Leichten Kette und der Translokationsdomäne hatte somit keinerlei Auswirkungen auf die Toxizität.

### Beispiel 6: Klonierung und Expression eines rekombinanten Fragmentes des Pseudomonas-Exotoxins (PE40) mit modifiziertem Loop

Unter Verwendung von chromosomaler DNS aus dem Stamm *Pseudomonas aeruginosa* 103 wurde mittels PCR mit den Primem # 17 und # 18 (Fig. 6) ein Genfragment amplifiziert, das für den Bereich der Domäne II, der C-terminal vom Loop zwischen den Cystein-Resten 13 und 36 liegt, sowie für die Domäne III codiert. Das Amplifikat wurde über *Nco* I und *Mlu* I im Austausch gegen das Genfragment BoNT(A)-L_{mod1} in das Plasmid pQE-BoNT(A)-L_{mod1} kloniert (Plasmid pQE-PEII_{3·}III). Der Sequenzabschnitt für den Bereich der Domäne II N-terminal vom Loop wurde durch Hybridisierung der Oligonukleotide # 15 und # 16 (Fig. 6) und klonierung über die Restriktionsschnittstellen für *Nco* I und *Kpn* I in das Plasmid pQE-PEII₃·III eingebracht (Plasmid pQE-PEII_{mod}III; Sequenz # 9). Der *E. coli*-Expressionsstamm M15[pREP4] (Qiagen) wurde mit dem entsprechenden Expressionsplasmid transformiert. Die Expression im Wirtsstamm M15[pREP4] und die Reinigung erfolgten analog zu Beispiel 1. Eine Analyse im SDS-Gel unter reduzierenden Bedingungen ergab eine schwächere Bande bei 40 kDa sowie eine stärkere bei 37 kDa. Unter nicht-reduzierenden Bedingungen war hingegen nur eine Bande bei 40 kDa zu beobachten. Wurde das Zelllysat vor der affinitätschromatographischen Reinigung für mindestens zwei Stunden bei Raumtemperatur inkubiert, war die 40 kDa-Bande unter reduzierenden Bedingungen nicht mehr nachzuweisen. Durch den Austausch des Loop-Bereichs zwischen den Cystein-Resten 13 und 36 in Domäne II des PE40-Fragments gegen einen modifizierten BoNT(A)-Loop erfolgte somit eine Spaltung der Polypeptidkette, wobei die genannten Cystein-Reste eine Disulfidbrücke ausbildeten. Das N-terminale, ca. 3 kDa große Fragment war nach Reduktion im 12 %igen SDS-Gel nicht nachzuweisen.

### Beispiel 7: Klonierung und Expression eines rekombinanten Fragmentes des Diphtheria-Toxins (DT389) mit modifiziertem Loop

Unter Verwendung von chromosomaler DNS aus dem Stamm *Corynebacterium diphtheriae* NCTC 13129 wurde mit den Primem # 19 und # 20 (Fig. 6) das für die A-Kette des Diphtheria-Toxins codierende Genfragment mittels PCR amplifiziert. Über die Restriktionsschnittstellen für *Nco* I und *Stu* I wurde das Amplifikat in das Plasmid pQE-BoNT(A)-L_{mod1} (siehe Beispiel 1) kloniert (Plasmid pQE-DT-A_{mod1}). Auf gleiche Weise wurde das für ein N-terminales Fragment der B-Kette codierende Genfragment mit den Primem # 21 und # 22 (Fig. 6) amplifiziert und über die Restriktionsschnittstellen für *Stu* I und *Xho* I in pQE-DT-A_{mod1} kloniert (Plasmid pQE-DT389-mod1; Sequenz # 10). Der *E. coli*-Expressionsstamm M15[pREP4] (Qiagen) wurde mit dem entsprechenden Expressionsplasmid transformiert. Die Expression im Wirtsstamm M15 [pREP4] und die Reinigung erfolgten analog zu Beispiel 1. Eine Analyse im SDS-Polyacrylamidgel ergab, dass durch Coomassie unter reduzierenden Bedingungen zwei Banden bei ca. 22 kDa angefärbt wurden, während unter nicht-reduzierenden Bedingungen eine Bande bei ca. 43 kDa zu beobachten war. Damit ist eindeutig gezeigt, dass das rekombinante Diphtheria-Toxin-Fragment zu > 90 % als zweikettiges Polypeptid aus den Bakterien freigesetzt wurde, in dem die beiden Ketten durch eine Disulfidbrücke kovalent miteinander verknüpft waren.

### Beispiel 8: Klonierung und Expression von rekombinantem Ricin mit modifiziertem Loop

Unter Verwendung von mRNA aus Samen von *Ricinus communis* wurde mit den Primer # 23 und # 24 (Fig. 6) das für die A-Kette von Ricin codierende Genfragment mittels RT-PCR amplifiziert. Über die Restriktionsschnittstellen für *Nco* I und *Xho* I wurde es in das Plasmid pQE-BoNT(A)-L_{mod1} (siehe Beispiel 1) kloniert (Plasmid pQE-Ricin-A). Auf gleiche Weise wurde das für die B-Kette codierende Genfragment mit den Primern # 25 und # 26 (Fig. 6) amplifiziert und über die Restriktionsschnittstellen für *Kpn* I und *Xho* I in pQE-Ricin-A kloniert (Plasmid pQE-Ricin-mod1; Sequenz # 11). Der *E. coli*-Expressionsstamm M15[pREP4] (Qiagen) wurde mit dem entsprechenden Expressionsplasmid transformiert. Die Expression im Wirtsstamm M15[pREP4] und die Reinigung des löslichen Anteils des exprimierten Ricins erfolgten analog zu Beispiel 1. Eine Analyse im SDS-Polyacrylamidgel ergab, dass durch Coomassie unter reduzierenden Bedingungen zwei Banden bei ca. 19 kDa und 42 kDa angefärbt wurden, während unter nicht-reduzierenden Bedingungen eine Bande bei ca. 62 kDa zu beobachten war. Damit ist eindeutig gezeigt, dass der lösliche Anteil des rekombinanten Ricins zu > 90 % als zweikettiges Polypeptid aus den Bakterien freigesetzt wurde, in dem die beiden Ketten durch eine Disulfidbrücke kovalent miteinander verknüpft waren.

### Wissenschaftliche Literatur

Arora et al. (1999), Cancer Res. 59: 183-8
Collier (2001), Toxicon 39(11): 1793-803
Fujinaga (1997), Microbiology 143: 3841-47
Ogata et al. (1990), J. Biol. Chem. 265(33): 20678-85
Reiter (2001), Adv. Cancer Res. 81: 93-124
Schiavo und Montecucco (1997), The Clostridia: Molecular Biology and Pathogenesis, Academic Press, San Diego: 295 - 322
Williams et al., (1990), J. Biol. Chem. 265(33): 20673-77

### Patentliteratur

Borgford, US-Patent 6,593,132
Brown und Jones, WO 89/04839
Fitzgerald et al., US-Patent 6,426,075
Pastan et al., US-Patent 5,980,895
Health Protection Agency, WO2004/024909

## Patentansprüche

1. Verfahren zur Herstellung von Polypeptiden bzw. Proteinen in zweikettiger Form, wobei die beiden Ketten disulfidverbrückt sind, mittels rekombinanter Expression in E. coli-Wirtszellen, wobei
(i) das Polypeptid bzw. Protein seine biologische Aktivität als zweikettiges disulfidverbrücktes Polypeptid bzw. Protein ausübt,
(ii) der C-terminale Aminosäure-Rest der ersten Kette ein basischer Aminosäure-Rest ist;
(iii) die zweite Kette des Proteins/Polypeptids N-terminal in der Richtung N-C 1 bis 20 Aminosäure-Reste und eine als PRS bezeichnete Pentapeptid-Sequenz VPXGS auf weist, wobei X jede beliebige natürlich vorkommende Aminosäure bedeutet, wobei V Val, Leu, Ile, Ala, Phe, Pro oder Gly bedeutet, wobei P Pro, Leu, Ile, Ala, Phe, Val oder Gly bedeutet, wobei G Gly, Leu, Ile, Ala, Pro, Phe oder Val bedeutet und wobei S Ser, Tyr, Trp oder Thr bedeutet; und
(iv) das Verfahren die folgenden Schritte umfasst:
(a)Veränderung des Polypeptids bzw. Proteins, auf dem Nukleinsäure-Level, so dass das Polypeptid bzw. Protein in seiner veränderten Form in seinem Loop-Bereich die Sequenz VPXGS und in N-terminaler Richtung von dieser PRS-Sequenz im Abstand von 1 bis 20 Aminosäure-Resten einen basischen Aminosäure-Rest aufweist, wobei X, V, P, G und S wie oben definiert sind, und wobei der Loop-Bereich definiert ist als die zwischen den beiden an der Disulfidbrücke beteiligten Cystein-Resten liegende Aminosäure-Sequenz;
(b)Einbringen des auf dem Nukleinsäure-Level veränderten Konstrukts in *E*. *coli-*Zellen;
(c) Kultivierung und anschließende Lyse der Wirtszellen; und
(d)Isolierung des zweikettigen disulfidverbrückten Polypeptids bzw. Proteins.

2. Das Verfahren nach Anspruch 1, wobei der basische Aminosäure-Rest in (ii) ein Arg- oder Lys-Rest ist.

3. Das Verfahren nach Anspruch 1 oder 2, wobei die erste Kette des Polypeptids/Proteins die leichtere Kette des Polypeptids/Proteins und die zweite Kette die schwerere Kette des Polypeptids/Proteins ist, insbesondere wobei das Protein ein Hybridprotein ist.

4. Das Verfahren nach einem der Ansprüche 1 bis 3, wobei das Polypeptid bzw. Protein ein Botulinum-Neurotoxin, insbesonderedas Botulinum-Neurotoxin des Serotyps A (BoNT(A)), und insbesondere das LH_{N}-Fragment von BoNT(A) ist.

5. Das Verfahren nach Anspruch 4, wobei die PRS-Sequenz VPXGS unter Deletion der Aminosäuren 443-447 zwischen die Aminosäuren Leu₄₄₂ und Lys₄₄₈ von BoNT(A) inseriert wird, insbesondere wobei die PRS-Sequenz VPRGS, VPYGS, VPHGS oder VPQGS inseriert wird.

6. Das Verfahren nach einem der Ansprüche 1 bis 4, wobei die PRS-Sequenz VPXGS unter Deletion von mindestens einer Aminosäure in das Oktapeptid Lys₄₃₈ - Ile₄₄₅ von BoNT(B), in das 15-mer His₄₃₈ - Asp₄₅₂ von BoNT(C1) oder in das 13-mer Lys₄₁₃ - Ile₄₂₅ von BoNT(E) inseriert wird, insbesondere wobei die PRS-Sequenz VPXGS in Form des 17-mers GIITSKTKSLVPRGSKA oder des 18-mers RGIITSKTKSLVPRGSKA inseriert wird.

7. Das Verfahren nach Anspruch 3, wobei das Hybridprotein die folgenden Komponenten A, B und C aufweist:
- eine Effektor-Domäne, die durch ihre enzymatische Aktivität imstande ist, die Sekretion in Zielzellen zu hemmen oder diese abzutöten oder eine Toxin-Domäne (Komponente A);
- eine Loop-Sequenz, die die Sequenz VPXGS aufweist (Komponente B), sowie
- eine Zellbindungsdomäne, die dem Fusions-/Hybridprotein eine Zellspezifität verleiht (Komponente C); und die optionale Komponente D:
- eine Translokationsdomäne.

8. Das Verfahren nach Anspruch 7, wobei die Toxin-Domäne (A) die Domäne des Diphtheria-Toxins, des Pseudomonas-Exotoxin oder von Ricin ist, insbesondere wobei die Toxin-Domäne (A) das Fragment PE40 (Domäne III, Domäne II und Domäne Ib) bzw. das Fragment PE38 (Domäne III und Domäne II) des Pseudomonas-Exotoxins oder die A-Kette von Ricin ist.

9. Das Verfahren nach Anspruch 7 oder 8, wobei die Zellbindungsdomäne (C) ein monoklonaler Antikörper, ein Affilin, ein Ankyrin Repeat Protein, ein Anticalin, ein Wachstumsfaktor wie TGF-alpha, FGF, VEGF oder IGF-1 oder ein Zytokin wie IL2, IL4 oder IL6 ist.

10. Das Verfahren nach Anspruch 3, wobei das Hybridprotein die folgenden Komponenten A, B und C aufweist:
- ein Protein oder ein Oligopeptid, das dem Fusionsprotein eine bessere Löslichkeit verleiht, eine höhere Expressionsrate bewirkt und/oder eine Affinitätsreinigung (Komponente A) ermöglicht,
- eine Loop-Sequenz, die die Sequenz VPXGS aufweist (Komponente B), sowie
- ein beliebiges Polypeptid (Komponente C).

11. Das Verfahren nach Anspruch 10, wobei die Komponente A die Glutathion-S-Transferase (GST), das Maltose-Bindungsprotein (MBP), ein His-Tag, ein StrepTag oder ein FLAG-Tag ist.

12. Das Verfahren nach einem der vorhergehenden Ansprüche, wobei die *E. coli*-Zellen *E*. *coli* K12-Zellen, insbesondere *E*. *coli* K12-Zellen der Stämme M15[pREP4], XL1-BLUE oder UT5600, sind.

13. Polypeptid oder Protein, wobei das Polypeptid/Protein als zweikettiges disulfidverbrücktes Polypeptid/Protein vorliegt und biologisch aktiv ist, **dadurch gekennzeichnet, dass** das C-terminale Ende der ersten Kette des Polypeptids/Proteins ein basischer Aminosäure-Rest ist und die zweite Kette des Polypeptids/Proteins N-terminal in der Richtung N-C 1 bis 20 Aminosäure-Reste und eine als PRS bezeichnete Pentapeptid-Sequenz VPXGS aufweist, wobei X jede beliebige natürlich vorkommende Aminosäure bedeutet, wobei V Val, Leu, Ile, Ala, Phe, Pro oder Gly bedeutet, wobei P Pro, Leu, Ile, Ala, Phe, Val oder Gly bedeutet, wobei G Gly, Leu, Ile, Ala, Pro, Phe oder Val bedeutet und wobei S Ser, Tyr, Trp oder Thr bedeutet.

14. Das Polypeptid oder Protein nach Anspruch 13, wobei der basische Aminosäure-Rest ein Arg- oder Lys-Rest ist.

15. Das Polypeptid oder Protein nach Anspruch 13 oder 14, wobei die erste Kette des Polypeptids/Proteins die leichtere Kette des Polypeptids/Proteins und die zweite Kette die schwerere Kette des Polypeptids/Proteins ist, insbesondere wobei das C-terminale Ende der ersten Kette ein Lys-Rest ist.

16. Das Polypeptid oder Protein nach einem der Ansprüche 13 bis 15, wobei die zweite Kette N-terminal die Pentapeptid-Sequenz VPXGS, die Hexapeptid-Sequenz XVPXGS oder die Heptapeptid-Sequenz XXVPXGS aufweist.

17. Das Polypeptid oder Protein nach einem der Ansprüche 13, 14 und 16, wobei das Polypeptid/Protein ein Botulinum-Neurotoxin, ein Derivat oder ein Fragment des Botulinum-Neurotoxins, insbesondere das LH_{N} Fragment, ist bzw. die biologische Aktivität eines Botulinum-Neurotoxins aufweist, insbesondere wobei das Polypeptid/Protein das Botulinum-Neurotoxin des Serotyps A (BoNT(A)) ist bzw. die biologische Aktivität des BoNT(A) aufweist, insbesondere, wobei das Polypeptid bzw. Protein das LH_{N}-Fragment von BoNT(A) ist bzw. die biologische Aktivität des LH_{N}-Fragments von BoNT(A) aufweist.

18. Das Polypeptid oder Protein nach einem der Ansprüche 13 bis 17, wobei die zweite Kette N-terminal die Heptapeptid-Sequenz SLVPXGS aufweist, insbesondere wobei X R, Y, H oder Q ist.

19. Das Polypeptid oder Protein nach einem der Ansprüche 13 bis 16 oder 18, wobei das Protein ein Hybridprotein ist, insbesondere wobei das Hybridprotein die folgenden Komponenten A, B und C aufweist:
- eine Effektor-Domäne, die durch ihre enzymatische Aktivität imstande ist, die Sekretion in Zielzellen zu hemmen oder diese abzutöten oder eine Toxin-Domäne (Komponente A);
- eine Loop-Sequenz, die die Sequenz VPXGS aufweist (Komponente B), sowie eine Zellbindungsdomäne, die dem Fusions-/Hybridprotein eine Zellspezifität verleiht (Komponente C); und die optionale Komponente D:
eine Translokationsdomäne

20. Das Polypeptid oder Protein nach Anspruch 19, wobei die Toxin-Domäne (A) die Domäne des Diphtheria-Toxins, des Pseudomonas-Exotoxin oder von Ricin ist, insbesondere wobei die Toxin-Domäne (A) das Fragment PE40 (Domäne III, Domäne II und Domäne Ib) bzw. das Fragment PE38 (Domäne III und Domäne II) des Pseudomonas-Exotoxins oder die A-Kette von Ricin ist.

21. Das Polypeptid oder Protein nach Anspruch 19 oder 20, wobei die Zellbindungsdomäne (C) ein monoklonaler Antikörper, ein Affilin, ein Ankyrin Repeat Protein, ein Anticalin, ein Wachstumsfaktor wie TGF-alpha, FGF, VEGF oder IGF-1 oder ein Zytokin wie IL2, IL4 oder IL6 ist.

22. Das Polypeptid oder Protein nach Anspruch 19, wobei das Hybridprotein die folgenden Komponenten A, B und C aufweist:
- ein Protein oder ein Oligopeptid, das dem Fusionsprotein eine bessere Löslichkeit verleiht, eine höhere Expressionsrate bewirkt und/oder eine Affinitätsreinigung (Komponente A) ermöglicht,
- eine Loop-Sequenz, die die Sequenz VPXGS aufweist (Komponente B), sowie
- ein beliebiges Polypeptid (Komponente C), insbesondere wobei die Komponente A die Glutathion-S-Transferase (GST), das Maltose-Bindungsprotein (MBP), ein His-Tag, ein StrepTag oder ein FLAG-Tag ist.

23. Pharmazeutische Zubereitung, umfassend das Polypeptid oder Protein nach einem der Ansprüche 13 bis 22.

## Claims

1. Method for producing polypeptides or proteins in the dichain form, wherein the two chains are disulfide-bridged, by means of recombinant expression in *E*. *coli* host cells, wherein
(i) the polypeptide or protein exerts its biologic activity as a dichain disulfide-bridged polypeptide or protein,
(ii) the C-terminal amino acid residue of the first chain is a basic amino acid residue,
(iii) the second chain of the protein/polypeptide has N-terminally in the N→C direction 1 to 20 amino acid residues and a pentapeptide sequence VPXGS referred to as PRS, wherein X is any naturally occurring amino acid, wherein V is Val, Leu, Ile, Ala, Phe, Pro or Gly, wherein P is Pro, Leu, Ile, Ala, Phe, Val or Gly, wherein G is Gly, Leu, Ile, Ala, Pro, Phe or Val, and wherein S is Ser, Tyr, Trp, or Thr; and
(iv) the method comprises the following steps:
(a) modification of the polypeptide or protein, at the nucleic acid level, so that the polypeptide or protein in its modified form in its loop area has the sequence VPXGS, wherein X, V, P, G, and S are as defined above, and exhibits in N-terminal direction of said PRS sequence in a distance of 1 to 20 amino acid residues a basic amino acid residue, and wherein the loop area is defined as the amino acid sequence lying between the two Cys residues involved in formation of the disulfide bridge;
(b) inserting the construct modified at the nucleic acid level into *E*. *coli* cells;
(c) cultivating and subsequently lysing the host cells; and
(d) isolating the dichain disulfide-bridged peptide or protein.

2. The method according to claim 1, wherein the basic amino acid residue in (ii) is an Arg residue or a Lys residue.

3. The method according to claim 1 or 2, wherein the first chain of the polypeptide/protein is the lighter chain of the polypeptide/protein and the second chain is the heavier chain of the polypeptide/protein, in particular wherein the protein is a hybrid protein.

4. The method according to any of claims 1 to 3, wherein the polypeptide or protein is a botulinum neurotoxin, in particular the botulinum neurotoxin of serotype A (BoNT(A)) and particularly the LH_{N} fragment of BoNT(A).

5. The method according to claim 4, wherein the PRS sequence VPXGS is inserted between the amino acids Leu₄₄₂ and Lys₄₄₈ of BoNT(A) with deletion of the amino acids 443-447, in particular wherein the PRS sequence VPRGS, VPYGS, VPHGS, or VPQGS is inserted.

6. The method according to any of claims 1 to 4, wherein the PRS sequence VPXGS is inserted into the octapeptide Lys₄₃₈ - Ile₄₄₅ of BoNT(B), into the 15mer His₄₃₈ - Asp₄₅₂ of BoNT(C1) or into the 13mer Lys₄₁₃ - Ile₄₂₅ of BoNT(E) with deletion of at least one amino acid, in particular wherein the PRS sequence VPXGS is inserted in the form of the 17mer GIITSKTKSLVPRGSKA or the 18mer RGIITSKTKSLVPRGSKA.

7. The method according to claim 3, wherein the hybrid protein has the following components A, B, and C:
- an effector domain that, by its enzymatic activity, is able to inhibit secretion in target cells or kill them, or a toxin domain (component A);
- a loop sequence that comprises the sequence VPXGS (component B); as well as
- a cell binding domain that imparts a cell specificity to the fusion protein or hybrid protein (component C); and the optional component D:
- a translocation domain.

8. The method according to claim 7, wherein the toxin domain (A) is the domain of the diphtheria toxin, of the pseudomonas exotoxin, or of ricin, in particular wherein the toxin domain (A) is the fragment PE40 (domain III, domain II, and domain Ib) or the fragment PE38 (domain III and domain II) of the pseudomonas exotoxin or the A-chain of ricin.

9. The method according to claim 7 or 8, wherein the cell binding domain (C) is a monoclonal antibody, an affilin, an ankyrin repeat protein, an anticalin, a growth factor such as TGF-alpha, FGF, VEGF, or IGF-1, or a cytokine such as IL2, IL4, or IL6.

10. The method according to claim 3, wherein the hybrid protein has the following components A, B, and C:
- a protein or an oligo peptide that imparts to the fusion protein a better solubility, effects a higher expression rate and/or enables affinity purification (component A);
- a loop sequence comprising the sequence VPXGS (component B), as well as
- any type of polypeptide (component C).

11. The method according to claim 10, wherein the component A is glutathione-S-transferase (GST), the maltose binding protein (MBP), a His tag, a StrepTag, or a FLAG tag.

12. The method according to one of the preceding claims, wherein the *E coli* cells are *E coli* K12 cells, in particular *E coli* K12 cells of the strains M15[pREP4], XL1-BLUE, or UT5600.

13. A polypeptide or protein, wherein the polypeptide/protein is present as a dichain disulfide-bridged polypeptide/protein and is biologically active, **characterized in that** the C-terminal end of the first chain of the polypeptide/protein is a basic amino acid residue and the second chain of the polypeptide/protein comprises N-terminally in the N→C direction 1 to 20 amino acid residues and a pentapeptide sequence VPXGS referred to as PRS, wherein X is any naturally occurring amino acid, wherein V is Val, Leu, Ile, Ala, Phe, Pro or Gly, wherein P is Pro, Leu, Ile, Ala, Phe, Val or Gly, wherein G is Gly, Leu, Ile, Ala, Pro, Phe or Val, and wherein S is Ser, Tyr, Trp, or Thr.

14. The polypeptide or protein according to claim 13 according to claim 13, wherein the basic amino acid residue is an Arg residue or a Lys residue.

15. The polypeptide or protein according to claim 13 or 14, wherein the first chain of the polypeptide/protein is the lighter chain of the polypeptide/protein and the second chain is the heavier chain of the polypeptide/protein, in particular wherein the C-terminal end of the first chain is a Lys residue.

16. The polypeptide or protein according to any of claims 13 to 15, wherein the second chain has N-terminally the pentapeptide sequence VPXGS, the hexapeptide sequence XVPXGS, or the heptapeptide sequence XXVPXGS.

17. The polypeptide or protein according to any of claims 13, 14, and 16, wherein the polypeptide/protein comprises a botulinum neurotoxin, a derivative or a fragment of botulinum neurotoxin, in particular the LH_{N} fragment, or has the biologic activity of a botulinum neurotoxin, in particular wherein the polypeptide/protein comprises the botulinum neurotoxin of the serotype A (BoNT(A)) or has the biologic activity of BoNT(A), in particular wherein the polypeptide or protein is the LH_{N} fragment of BoNT(A) or has the biologic activity of the LH_{N} fragment of BoNT(A).

18. The polypeptide or protein according to any of claims 13 to 17, wherein the second chain has N-terminally the heptapeptide sequence SLVPXGS, in particular wherein X is R, Y, H, or Q.

19. The polypeptide or protein according to one of the claims 13 to 16 or 18, wherein the protein is a hybrid protein, in particular wherein the hybrid protein has the following components A, B, and C:
- an effector domain that, by its enzymatic activity, is able to inhibit secretion in target cells or kill them, or a toxin domain (component A);
- a loop sequence that comprises the sequence VPXGS (component B); as well as
- a cell binding domain that imparts a cell specificity to the fusion protein or hybrid protein (component C); and the optional component D:
- a translocation domain.

20. The polypeptide or protein according to claim 19, wherein the toxin domain (A) is the domain of the diphtheria toxin, of the pseudomonas exotoxin, or of ricin, in particular wherein the toxin domain (A) is the fragment PE40 (domain III, domain II, and domain Ib) or the fragment PE38 (domain III and domain II) of the pseudomonas exotoxin or the A-chain of ricin.

21. The polypeptide or protein according to claim 19 or 20, wherein the cell binding domain (C) is a monoclonal antibody, an affilin, an ankyrin repeat protein, an anticalin, a growth factor such as TGF-alpha, FGF, VEGF, or IGF-1, or a cytokine such as IL2, IL4, or IL6.

22. The polypeptide or protein according to claim 19, wherein the hybrid protein has the following components A, B, and C:
- a protein or an oligopeptide that imparts to the fusion protein a better solubility, effects a higher expression rate and/or enables affinity purification (component A);
- a loop sequence comprising the sequence VPXGS (component B), as well as
- any type of polypeptide (component C),
in particular wherein the component A is glutathione-S-transferase (GST), the maltose binding protein (MBP), a His tag, a StrepTag or a FLAG tag.

23. A pharmaceutical preparation comprising the polypeptide or protein according to any of claims 13 to 22.

## Revendications

1. Procédé de préparation de polypeptides ou de protéines sous forme double chaîne, où les deux chaînes sont reliées par pont disulfure, au moyen d'une expression recombinante dans des cellules hôtes *E coli,* dans lequel :
(i) le polypeptide ou la protéine exerce son activité biologique en tant que polypeptide ou protéine double chaîne à pont disulfure,
(ii) le résidu d'acide aminé C-terminal de la première chaîne est un résidu d'acide aminé basique ;
(iii) la deuxième chaîne de la protéine/ du polypeptide présente en N-terminal dans la direction N-C 1 à 20 résidus d'acides aminés et une séquence de pentapeptide VPXGS appelée PRS, où X représente un acide aminé quelconque présent à l'état naturel, où V représente Val, Leu, Ile, Ala, Phe, Pro ou Gly, où P représente Pro, Leu, Ile, Ala, Phe, Val ou Gly, où G représente Gly, Leu, Ile, Ala, Pro, Phe ou Val et où S représente Ser, Tyr, Trp ou Thr ; et
(iv) ledit procédé comprend les étapes suivantes :
(a) la modification du polypeptide ou de la protéine, au niveau de l'acide nucléique, de sorte que le polypeptide ou la protéine présente sous sa forme modifiée dans sa région formant une boucle la séquence VPXGS et, dans la direction N-terminale de cette séquence PRS, à une distance de 1 à 20 résidus d'acides aminés, un résidu d'acide aminé basique, où X, V, P, G et S sont tels que définis ci-dessus, et où la région formant une boucle est définie comme la séquence d'acides aminés située entre les deux résidus de cystéine faisant partie du pont disulfure ;
(b) l'introduction du construit modifié au niveau de l'acide nucléique dans les cellules *E. coli* ;
(c) la culture et la lyse consécutive des cellules hôtes ; et
(d) l'isolation du polypeptide ou de la protéine double chaîne à pont disulfure.

2. Procédé selon la revendication 1, dans lequel le résidu d'acide aminé basique au point (ii) est un résidu Arg ou Lys.

3. Procédé selon la revendication 1 ou 2, dans lequel la première chaîne du polypeptide/de la protéine est la chaîne légère du polypeptide/de la protéine et la deuxième chaîne est la chaîne lourde du polypeptide/de la protéine, en particulier dans lequel la protéine est une protéine hybride.

4. Procédé selon l'une des revendications 1 à 3, dans lequel le polypeptide ou la protéine est une neurotoxine botulique, en particulier la neurotoxine botulique de sérotype A (BoNT(A)), et en particulier le fragment LH_{N} de la BoNT(A).

5. Procédé selon la revendication 4, dans lequel la séquence PRS VPXGS est insérée entre les acides aminés Leu₄₄₂ et Lys₄₄₈ de BoNT(A) par délétion des acides aminés 443-447, en particulier dans lequel la séquence PRS VPRGS, VPYGS, VPHGS ou VPQGS est insérée.

6. Procédé selon l'une des revendications 1 à 4, dans lequel la séquence PRS VPXGS est insérée dans l'octapeptide Lys₄₃₈ - Ile₄₄₅ de BoNT(B), dans le 15-mère His₄₃₈ - Asp₄₅₂ de BoNT(C1) ou dans le 13-mère Lys₄₁₃ - Ile₄₂₅ de BoNT(E) par délétion d'au moins un acide aminé, en particulier dans lequel la séquence PRS VPXGS est insérée sous la forme du 17-mère GIITSKTKSLVPRGSKA ou du 18-mère RGIITSKTKSLVPRGSKA.

7. Procédé selon la revendication 3, dans lequel la protéine hybride présente les composantes A, B et C suivantes :
- un domaine effecteur, qui est en mesure, grâce à son activité enzymatique, d'inhiber la sécrétion dans des cellules cibles ou de tuer ces cellules, ou un domaine de toxine (composante A) ;
- une séquence de boucle, qui comprend la séquence VPXGS (composante B), et
- un domaine de liaison cellulaire, qui confère une spécificité cellulaire à la protéine de fusion/hybride (composante C) ; et la composante facultative D:
- un domaine de translocation.

8. Procédé selon la revendication 7, dans lequel le domaine de toxine (A) est le domaine de la toxine diphtérique, de l'exotoxine de *Pseudomonas* ou du ricin, en particulier dans lequel le domaine de toxine (A) est le fragment PE40 (domaine III, domaine II et domaine Ib) ou le fragment PE38 (domaine III et domaine II) de l'exotoxine de *Pseudomonas* ou la chaîne A du ricin.

9. Procédé selon la revendication 7 ou 8, dans lequel le domaine de liaison cellulaire (C) est un anticorps monoclonal, une affiline, une protéine à domaine ankyrine répété, une anticaline, un facteur de croissance comme TGF-alpha, FGF, VEGF ou IGF-1 ou une cytokine comme IL-2, Il ou IL-6.

10. Procédé selon la revendication 3, dans lequel la protéine hybride comprend les composantes A, B et C suivantes :
- une protéine ou un oligopeptide, qui confère à la protéine de fusion une meilleure solubilité, suscite un taux d'expression plus élevé et/ou permet une purification par affinité (composante A),
- une séquence de boucle, qui comprend la séquence VPXGS (composante B), et
- un polypeptide quelconque (composante C).

11. Procédé selon la revendication 10, dans lequel la composante A est la glutathion-S-transférase (GST), la protéine de liaison du maltose (MBP), une étiquette His, une étiquette Strep ou une étiquette FLAG.

12. Procédé selon l'une des revendications précédentes, dans lequel les cellules *E. coli* sont des cellules K12 de *E*. *coli,* en particulier des cellules K12 de *E*. *coli* des souches M15[pREP4], XL1-BLUE ou UT5600.

13. Polypeptide ou protéine, où ledit polypeptide/ladite protéine se présente sous la forme d'un polypeptide/d'une protéine double chaîne à pont disulfure et est biologiquement actif ou active, caractérisé(e) en ce que l'extrémité C-terminale de la première chaîne du polypeptide/de la protéine est un résidu d'acide aminé basique et la deuxième chaîne du polypeptide/de la protéine comprend en N-terminal dans la direction N-C 1 à 20 résidus d'acides aminés et une séquence de pentapeptide VPXGS appelée PRS, où X représente un acide aminé quelconque présent à l'état naturel, où V représente Val, Leu, Ile, Ala, Phe, Pro ou Gly, où P représente Pro, Leu, Ile, Ala, Phe, Val ou Gly, où G représente Gly, Leu, Ile, Ala, Pro, Phe ou Val et où S représente Ser, Tyr, Trp ou Thr.

14. Polypeptide ou protéine selon la revendication 13, où le résidu d'acide aminé basique est un résidu Arg ou Lys.

15. Polypeptide ou protéine selon la revendication 13 ou 14, où la première chaîne du polypeptide/de la protéine est la chaîne légère du polypeptide/de la protéine et la deuxième chaîne est la chaîne lourde du polypeptide/de la protéine, en particulier où l'extrémité C-terminale de la première chaîne est un résidu Lys.

16. Polypeptide ou protéine selon l'une des revendications 13 à 15, où la deuxième chaîne comprend en N-terminal la séquence de pentapeptide VPXGS, la séquence d'hexapeptide XVPXGS ou la séquence d'heptapeptide XXVPXGS.

17. Polypeptide ou protéine selon l'une des revendications 13, 14 et 16, où le polypeptide/la protéine est une neurotoxine botulique, un dérivé ou un fragment de la neurotoxine botulique, en particulier le fragment LH_{N}, ou présente l'activité biologique d'une neurotoxine botulique, en particulier où le polypeptide/la protéine est la neurotoxine botulique de sérotype A (BoNT(A)) ou présente l'activité biologique de la BoNT(A) ou le polypeptide/la protéine est le fragment LH_{N} de la BoNT(A) ou présente l'activité biologique du fragment LH_{N} de la BoNT(A).

18. Polypeptide ou protéine selon l'une des revendications 13 à 17, où la deuxième chaîne comprend en N-terminal la séquence d'heptapeptide SLVPXGS, en particulier où X est R, Y, H ou Q.

19. Polypeptide ou protéine selon l'une des revendications 13 à 16 ou 18, où la protéine est une protéine hybride, en particulier où la protéine hybride présente les composantes A, B et C suivantes :
- un domaine effecteur, qui est en mesure, grâce à son activité enzymatique, d'inhiber la sécrétion dans des cellules cibles ou de tuer ces cellules, ou un domaine de toxine (composante A) ;
- une séquence de boucle, qui comprend la séquence VPXGS (composante B), et
- un domaine de liaison cellulaire, qui confère une spécificité cellulaire à la protéine de fusion/hybride (composante C) ; et la composante facultative D:
- un domaine de translocation.

20. Polypeptide ou protéine selon la revendication 19, où le domaine de toxine (A) est le domaine de la toxine diphtérique, de l'exotoxine de *Pseudomonas* ou du ricin, en particulier où le domaine de toxine (A) est le fragment PE40 (domaine III, domaine II et domaine Ib) ou le fragment PE38 (domaine III et domaine II) de l'exotoxine de *Pseudomonas* ou la chaîne A du ricin.

21. Polypeptide ou protéine selon la revendication 19 ou 20, où le domaine de liaison cellulaire (C) est un anticorps monoclonal, une affiline, une protéine à domaine ankyrine répété, une anticaline, un facteur de croissance comme TGF-alpha, FGF, VEGF ou IGF-1 ou une cytokine comme IL-2, IL-4 ou IL-6.

22. Polypeptide ou protéine selon la revendication 19, où la protéine hybride comprend les composantes A, B et C suivantes :
- une protéine ou un oligopeptide, qui confère à la protéine de fusion une meilleure solubilité, suscite un taux d'expression plus élevé et/ou permet une purification par affinité (composante A),
- une séquence de boucle, qui comprend la séquence VPXGS (composante B), et
- un polypeptide quelconque (composante C), en particulier où la composante A est la glutathion-S-transférase (GST), la protéine de liaison du maltose (MBP), une étiquette His, une étiquette Strep ou une étiquette FLAG.

23. Composition pharmaceutique comprenant le polypeptide ou la protéine selon l'une des revendications 13 à 22.
